# EUROPÄISCHE PATENTSCHRIFT

(11) **EP 0 508 126 B1**
(45) Veröffentlichungstag und Bekanntmachung des Hinweises auf die Patenterteilung: **02.05.1997**
(21) Anmeldenummer: 92104044.0
(22) Anmeldetag: 10.03.1992
(51) Int. Cl.: C07D 231/32, A01N 43/56, C07D 487/04, C07D 487/08, C07C 59/88, C07C 57/34

(54) **3-Hydroxy-4-aryl-5-oxo-pyrazolin Derivate**
3-Hydroxy-4-aryl-5-oxo-pyrazolin derivatives
Dérivés de hydroxy-3-aryl-4-oxo-5-pyrazoline

(30) Priorität: 21.03.1991 DE 4109208
(43) Veröffentlichungstag der Anmeldung: 14.10.1992
(73) Patentinhaber: BAYER AG, 51368 Leverkusen (DE)
(72) Erfinder: Krüger, Bernd-Wieland, Dr., W-5060 Bergisch Gladbach 2 (DE); Fischer, Reiner, Dr., W-4019 Monheim 2 (DE); Bertram, Heinz-Jürgen, Dr., W-3450 Holzminden (DE); Bretschneider, Thomas, Dr., W-5200 Siegburg (DE); Böhm, Stefan, Dr., W-5090 Leverkusen 1 (DE); Krebs, Andreas, Dr., W-5068 Odenthal-Holz (DE); Schenke, Thomas, Dr., W-5060 Bergisch Gladbach 2 (DE); Santel, Hans-Joachim, Dr., W-5090 Leverkusen 1 (DE); Lürssen, Klaus, Dr., W-5060 Bergisch Gladbach 2 (DE); Schmidt, Robert R., Dr., W-5060 Bergisch Gladbach 2 (DE); Erdelen, Christoph, Dr., W-5653 Leichlingen 3 (DE); Wachendorff-Neumann, Ulrike, Dr., W-4019 Monheim (DE); Stendel, Wilhelm, Dr., W-5600 Wuppertal 1 (DE)

(56) Entgegenhaltungen:
- WO-A-92/16510
- US-A- 3 149 115
- THE JOURNAL OF ORGANIC CHEMISTRY, Band 53, Nr. 13, 24. juni 1988, Seiten 2889- 2298, Easton, US; K.T. POTTS et al.: "Cross-conjugated and pseudo-cross- conjugated mesomeric betaines. 1. Synthesis and characterization"
- SYNTHESIS, Nr 2, 1. Februar 1990, Seiten 167-170, Stuttgart, DE; W. RIED et al. : "Synthese neuer 1,3-Oxazinone aus Cyanamiden und Chlorocarbonylketenen"
- JOURNAL OF THE CHEMICAL SOCIETY. CHEMICAL COMMUNICATION, Nr. 11, 1987, Seiten 840-842, Letchworth, GB; K.T. POTTS et al.: "Carbon-carbon bond formation by intramolecular 1,4-dipolar cycloaddition: heterocyclic betaines generated in situ from amides and N-substituted amides"
- MONATSHEFTE FÜR CHEMIE, Band 119, Nr. 6/7, Juni/Juli 1988, Seiten 727-737, Wien, AT; T. KAPPE et al.: "Nichtsteroide Entzündungshemmer, 4 [1] Umlagerungen von Heterocyclen, 11 [2] Ketenoide Umlagerung und Oxidation von 2-Pyronen zu 1, 4-Naphthochinon-5-alkansäuren"
- CHEMICAL ABSTRACTS, Band 73. Nr. 3, 20. Juli 1970, Seite 290, Spalte 2, Zusammenfassung Nr. 13946v, Columbus, Ohio, US; V. VLASOV et al.: "Aromatic fluoro derivatives. XL. Effect of a pentafluorophenyl ring on the strength of CH-acids", & ZH. ORG. KHIM. 1970, 6(4), 758-67
- JOURNAL OF HETEROCYCLIC CHEMISTRY, Band 25, Nr. 5, September-Oktober 1988, Seiten 1301-1305, Provo, US; G. ZVILICHOVSKY: "Crystal structure, dissociation and Zwitterion formation in 2,6-Diaryl-1(3)-oxo-3(1)-hydroxy-5(7)-imino-7(5)- amino-1H,5H-(3H,7H)-pyrazolo[1,2-alpha]pyrazoles"
- JOURNAL OF HETEROCYCLIC CHEMISTRY, Band 25, Nr. 5, September-Oktober 1988, Seiten 1307-1310, Provo, US; G. ZVILICHOVSKY et al.: "Acidity and alkylation of 4-phenyl-3,5-dihydroxypyrazole and its derivatives. C versus O and N alkylation"

## Beschreibung

Die Erfindung betrifft neue polycyclische 3-Hydroxy-4-aryl-5-oxo-pyrazolin-Derivate, mehrere Verfahren zu ihrer Herstellung und ihre Verwendung als Insektizide, Akarizide und Herbizide.

Aus der Literatur sind bestimmte 3H-Pyrazol-3-on-Derivate, wie beispielsweise 1,2-Diethyl-1,2-dihydro-5-hydroxy-4-phenyl-3H-pyrazol-3-on oder {[5-Oxo-1,2-diphenyl-4-(p-sulfophenyl)-3-pyrazolin-3-yl]-oxy}-dinatriumsalz oder p-(3-Hydroxy-5-oxo-1,2-diphenyl-3-pyrazolin-4-yl)-benzolsulfonsäure bekannt (vgl. J.Heterocycl. Chem., 25(5), 1301-1305, 1988 oder J. Heterocycl. Chem., 25 (5), 1307-1310, 1988 oder Zh. Obshch. Khim., 34(7), 2397-2402, 1964). Eine biologische Wirkung dieser Verbindungen wird aber nicht beschrieben.

Weiterhin ist bekannt, daß das Trinatriumsalz der 4, 4', 4''-(5-Hydroxy-3-oxo-1H-pyrazol-1,2,4(3H)-triyl)-tris-benzolsulfonsäure pharmakologische Eigenschaften besitzt (vgl. Farmakol. Toksikol. (Moscow), 39(2), 180-186, 1976). Seine Verwendung im Pflanzenschutz ist aber nicht bekannt.

Es wurden nun neue 3-Hydroxy-4-aryl-5-oxo-pyrazolin-Derivato der FormeI (I) gefunden, 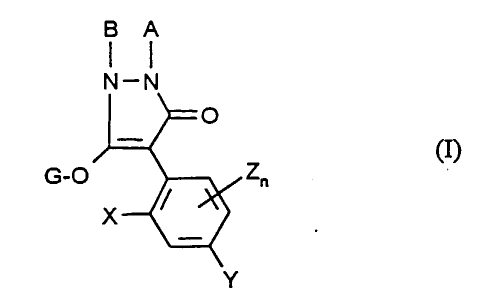 in welcher
- A und B: gleich oder verschieden sind und unabhängig voneinander jeweils für C₁-C₄-Alkyl_{,} C₂-C₄-Alkenyl, C₂-C₄-Alkinyl, C₁-C₄-Alkoxy-C₁-C₄-alkyl, C₁-C₄-Alkylthio-C₁-C₄-alkyl, Cycloalkyl mit 3 bis 7 Kohlenstoffatomen oder für gegebenenfalls einfach bis dreifach, gleich oder verschieden substituiertes Phenyl stehen, wobei als Phenylsubstituenten die für R³, R⁴ und R⁵ aufgeführten Phenylsubstituenten in Frage kommen oder
- A und B: zusammen mit den beiden Stickstoffatomen des Pyrazolinringes für eine gegebenenfalls einfach bis dreifach, gleich oder verschieden substituierte Gruppierung der nachfolgend aufgeführten Formeln 1', 2', 3' oder 4' stehen 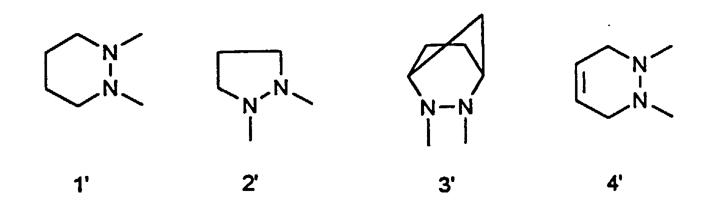 wobei als Substituenten Halogen, gegebenenfalls durch Fluor oder Chlor substituiertes C₁-C₈-Alkyl, C₂-C₈-Alkenyl und C₁-C₄-Alkoxy-C₁-C₄-alkyl in Frage kommen,
- X: für Methyl, Ethyl, Propyl, i-Propyl, Fluor, Chlor, Brom, Methoxy und Ethoxy steht,
- Y: für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,
- Z: für Methyl, Ethyl, i-Propyl, Butyl, i-Butyl, tert.-Butyl, Fluor, Chlor, Brom, Methoxy und Ethoxy steht,
- n: für eine Zahl 0, 1, 2 oder 3 steht,
- G: für Wasserstoff (a) oder für die Gruppen 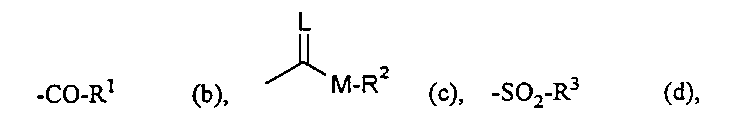 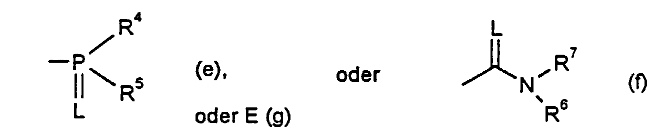 steht, in welchen
E für ein Metallionäquivalent oder ein Ammoniumion steht,
L und M für Sauerstoff und/oder Schwefel stehen,
R¹ für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Alkylthio-C₂-C₆-alkyl, C₁-C₄-Polyalkoxy-C₂-C₄-alkyl oder Cycloalkyl mit 3 bis 6 Ringatomen, das durch 1 bis 2 Sauerstoff- und/oder Schwefelatome unterbrochen sein kann, steht,
für gegebenenfalls durch Fluor, Chlor, Brom, Methyl, Ethyl, Propyl, i-Propyl, Methoxy, Ethoxy, Trifluormethyl, Trifluormethoxy, Nitro substituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy substituiertes Phenyl-C₁-C₃-alkyl steht,
für gegebenenfalls durch Fluor, Chlor, Brom, Methyl, Ethyl substituiertes Pyridyl, Pyrimidyl, Thiazolyl und Pyrazolyl steht,
für gegebenenfalls durch Fluor, Chlor, Methyl, Ethyl substituiertes Phenoxy-C₁-C₄-alkyl steht,
für gegebenenfalls durch Fluor, Chlor, Amino, Methyl, Ethyl substituiertes Pyridyloxy-C₁-C₄-alkyl, Pyrimidyloxy-C₁-C₄-alkyl und Thiazolyloxy-C₁-C₄-alkyl steht,
R² für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Polyalkoxy-C₂-C₆-alkyl steht,
oder für gegebenenfalls durch Fluor, Chlor, Nitro, Methyl, Ethyl, Propyl, i-Propyl, Methoxy, Ethoxy, Trifluormethyl substituiertes Phenyl oder Benzyl steht,
R³, R⁴ und R⁵ unabhängig voneinander für gegebenenfalls durch
Fluor oder Chlor substituiertes C₁-C₄-Alkyl, C₁-C₄-Alkoxy, C₁-C₄-Alkylamino, Di-(C₁-C₄)-alkylamino, C₁-C₄-Alkylthio, für gegebenenfalls durch Fluor, Chlor, Brom, Nitro, Cyano, C₁-C₂-Alkoxy, C₁-C₄-Fluoralkoxy, C₁-C₂-Chloralkoxy, C₁-C₂-Alkylthio, C₁-C₂-Fluoralkylthio, C₁-C₂-Chloralkylthio, C₁-C₃-Alkyl, C₁-C₃-Halogenalkyl substituiertes Phenyl, Phenoxy oder Phenylthio stehen,
R⁶ und R⁷ unabhängig voneinander für gegebenenfalls durch Fluor,
Chlor, Brom substituiertes C₁-C₁₀-Alkyl, C₁-C₁₀-Alkoxy, C₁-C₁₀-Alkoxy-C₁-C₁₀-alkyl,
für gegebenenfalls durch Fluor, Chlor, Brom, C₁-C₂₀-Halogenalkyl, C₁-C₂₀-Alkyl oder C₁-C₄-Alkoxy substituiertes Phenyl,
für gegebenenfalls durch Fluor, Chlor, Brom, C₁-C₄-Alkyl, C₁-C₄-Halogenalkyl oder C₁-C₄-Alkoxy substituiertes Benzyl stehen,
mit der Maßgabe, daß
- A und B: unabhängig voneinander nicht für C₁-C₄-Alkyl, C₃-C₄-Alkenyl oder C₃-C₄-Alkinyl stehen oder
- A und B: zusammen nicht für einen gegebenenfalls bis zu dreifach durch C₁-C₄-Alkyl substituierten Rest aus der Reihe 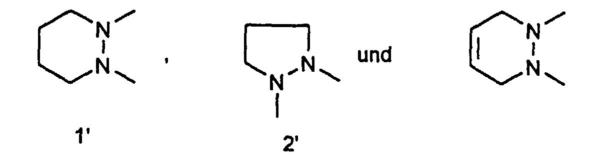 stehen, wenn G für Wasserstoff steht.

Unter Einbeziehung der verschiedenen Bedeutungen (a), (b), (c), (d), (e), (f) und (g) der Gruppe G der allgemeinen Formel (I) ergeben sich folgende hauptsächlichen Strukturen (Ia) bis (Ig): 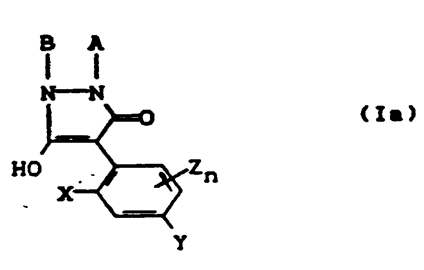 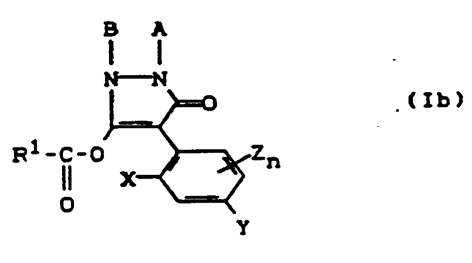 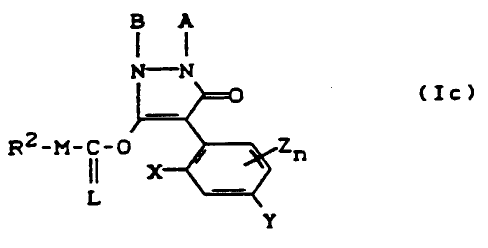 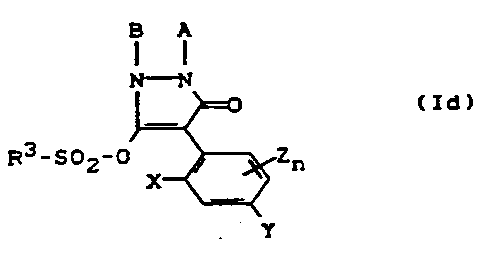 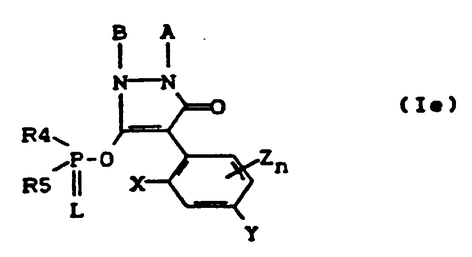 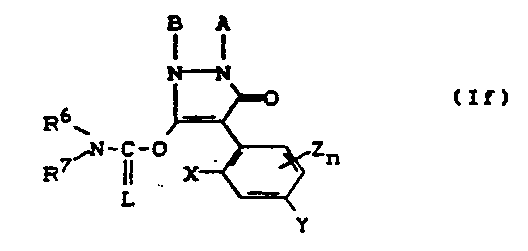 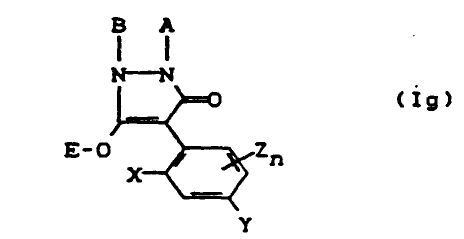 worin
A, B, E, L, M, X, Y, Zₙ, R¹, R², R³, R⁴, R⁵, R⁶ und R⁷ die oben angegebenen Bedeutungen besitzen.

Aufgrund eines oder mehrerer Chiralitätszentren, fallen die Verbindungen der Formel (Ia) - (Ig) im allgemeinen als Stereoisomerengemisch an. Sie können sowohl in Form ihrer Diastereomerengemische als auch als reine Diastereomere oder Enantiomere verwendet werden.

Weiterhin wurde gefunden, daß man die neuen 3-Hydroxy-4-aryl-5-oxo-pyrazolin-Derivate der Formel (I) nach einem der im folgenden beschriebenen Verfahren erhält.
(A) Man erhält 3-Hydroxy-4-aryl-5-oxo-pyrazoline der Formel (Ia) 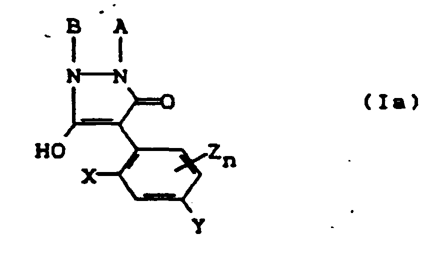 in welcher
   - A, B, X, Y, Z und n: die oben angegebene Bedeutung haben,
   wenn man
   (α) Halogencarbonylketene der Formel (II) 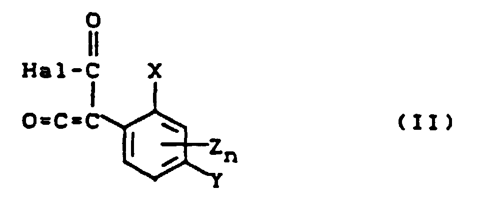 in welcher
      - X, Y, Z und n: die oben angegebene Bedeutung haben
      und
      - Hal: für Halogen, insbesondere Chlor oder Brom steht,
      oder
   (β) Malonsäurederivate der Formel (III) 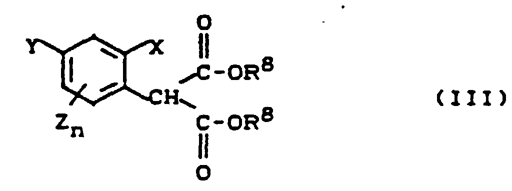 in welcher
      - X, Y, Z und n: die oben angegebene Bedeutung haben und
      - R⁸: für Alkyl steht,
      mit Hydrazinen der Formel (IV)

      A-NH-NH-B (IV)

      in welcher
      - A und B: die oben angegebene Bedeutung haben,
      gegebenenfalls in Gegenwart eines Verdünnungsmittels und gegebenenfalls in Gegenwart einer Base umsetzt;
   oder
(B) man erhält Verbindungen der Formel (Ib) 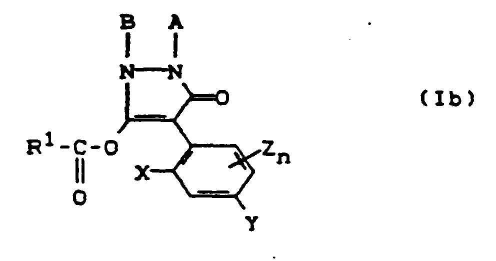 in welcher
   - A, B, X, Y, Z, R¹ und n: die oben angegebene Bedeutung haben,
   wenn man Verbindungen der Formel (Ia), 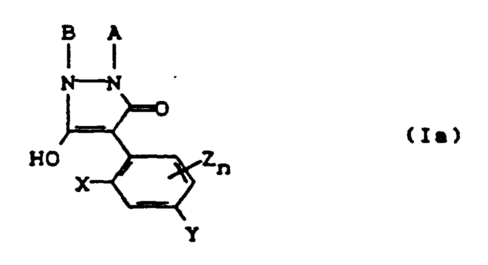 in welcher
   - A, B, X, Y, Z und n: die oben angegebene Bedeutung haben,

   α) mit Säurehalogeniden der allgemeinen Formel (V) 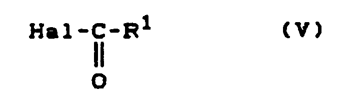 in welcher
      - R¹: die oben angegebene Bedeutung hat und
      - Hal: für Halogen, insbesondere Chlor und Brom steht,
      gegebenenfalls in Gegenwart eines Verdünnungsmittels und gegebenenfalls in Gegenwart eines Säurebindemittels umsetzt
      oder
   β) mit Carbonsäureanhydriden der allgemeinen Formel (VI)

      R¹-CO-O-CO-R¹ (VI)

      in welcher
      - R¹: die oben angegebene Bedeutung hat,
      gegebenenfalls in Gegenwart eines Verdünnungsmittels und gegebenenfalls in Gegenwart eines Säurebindemittels,
   umsetzt;
   oder
(C) man erhält Verbindungen der Formel (Ic-1) 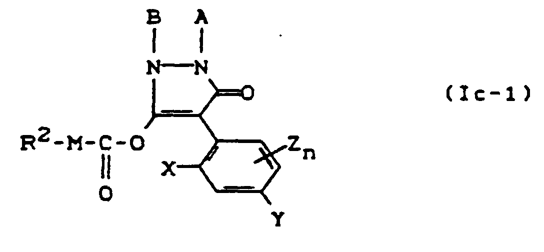 in welcher
   - A, B, X, Y, Z, R² und n: die oben angegebene Bedeutung haben,
   und
   - M: für Sauerstoff oder Schwefel steht,
   wenn man Verbindungen der Formel (Ia) 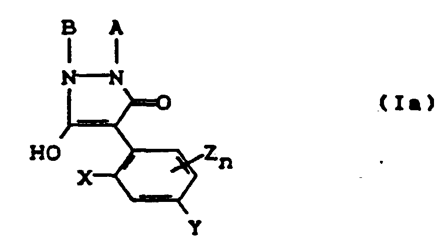 in welcher
   - A, B, X, Y, Z und n: die oben angegebene Bedeutung haben,
   mit Chlorameisensäureester oder Chlorameisensäurethiolester der allgemeinen Formel (VII)

   R²-M-CO-Cl (VII)

   in welcher
   - R² und M: die oben angegebene Bedeutung haben,
   gegebenenfalls in Gegenwart eines Verdünnungsmittels und gegebenenfalls in Gegenwart eines Säurebindemittels umsetzt;
   oder
(D) man erhält Verbindungen der Formel (Ic-2) 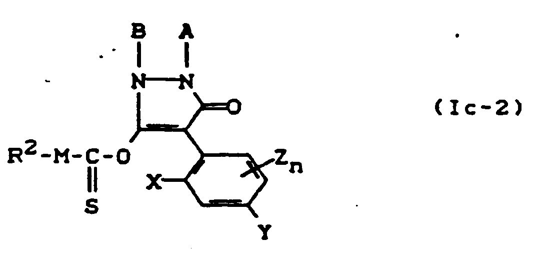 in welcher
   - A, B, R², X, Y, Z und n: die oben angegebene Bedeutung haben,
   und
   - M: für Sauerstoff oder Schwefel steht,
   wenn man Verbindungen der Formel (Ia) 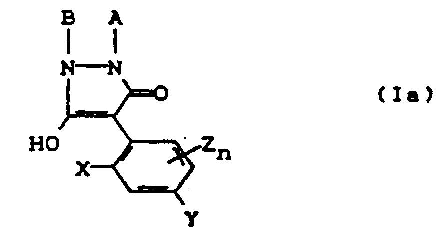 in welcher
   - A, B, X, Y, Z und n: die oben angegebene Bedeutung haben,

   α) mit Chlormonothioameisensäureestern oder Chlordithioameisensaureestern der allgemeinen Formel (VIII) 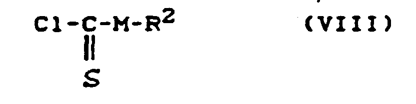 in welcher
      - M und R²: die oben angegebene Bedeutung haben,
      gegebenenfalls in Gegenwart eines Verdünnungsmittels und gegebenenfalls in Gegenwart eines
      Säurebindemittels umsetzt,
      oder
   β) mit Schwefelkohlenstoff und anschließend mit Alkylhalogeniden der allgemeinen Formel (IX)

      R²-Hal (IX)

      in welcher
      - R²: die oben angegebene Bedeutung hat
      und
      - Hal: für Chlor, Brom, Iod steht,
   umsetzt;
   oder
(E) man erhält Verbindungen der Formel (Id) 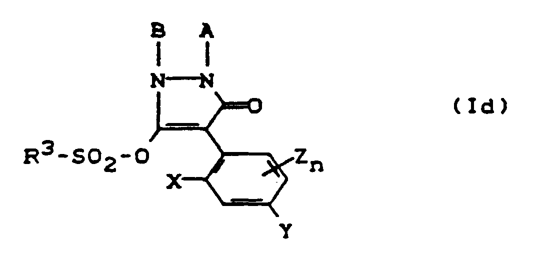 in welcher
   - A, B, X, Y, Z, R³ und n: die oben angegebene Bedeutung haben,
   wenn man Verbindungen der Formel (Ia) 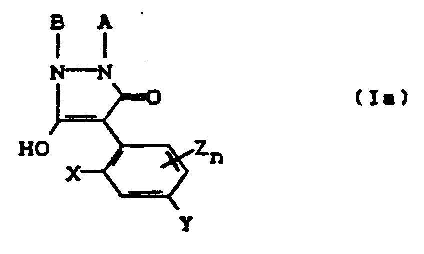 in welcher
   - A, B, X, Y, Z und n: die oben angegebene Bedeutung haben,
   mit Sulfonsäurechloriden der allgemeinen Formel (X)

   R³-SO₂-Cl (X)

   in welcher
   - R³: die oben angegebene Bedeutung hat,
   gegebenenfalls in Gegenwart eines Verdünnungsmittels und gegebenenfalls in Gegenwart eines Säurebindemittels,
   umsetzt;
   oder
(F) man erhält die Verbindungen der Formel (Ie) 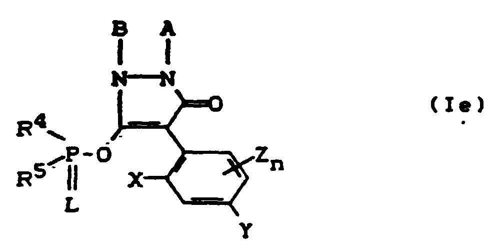 in welcher
   - A, B, L, X, Y, Z, R⁴, R⁵ und n: die oben angegeben. Bedeutung haben,
   wenn man
   3-Hydroxy-4-aryl-5-oxo-pyrazoline der Formel (Ia) 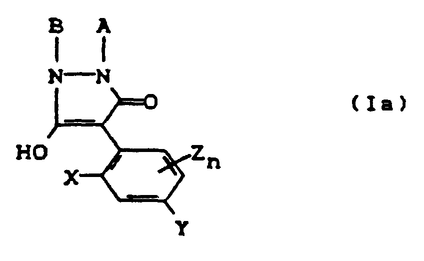 in welcher
   - A, B, X, Y, Z und n: die oben angegebene Bedeutung haben,
   mit Phosphorverbindungen der allgemeinen Formel (XI) 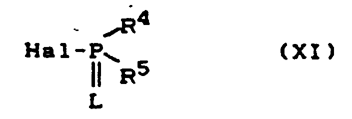 in welcher
   - L, R⁴ und R⁵: die oben angegebene Bedeutung haben
   und
   - Hal: für Halogen, insbesondere Chlor und Brom steht,
   gegebenenfalls in Gegenwart eines Verdünnungsmittels und gegebenenfalls in Gegenwart eines Säurebindemittels umsetzt;
   oder
(G) man erhält Verbindungen der Formel (If) 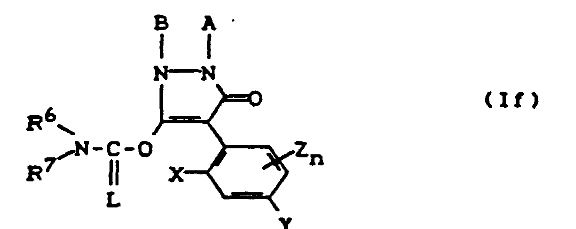 in welcher
   - A, B, L, X, Y, Z, R⁶, R⁷ und n: die oben angegebene Bedeutung haben,
   wenn man Verbindungen der Formel (Ia) 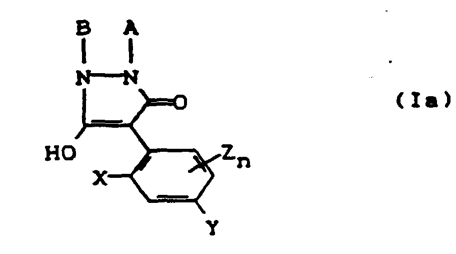 in welcher
   - A, B, X, Y, Z und n: die oben angegebene Bedeutung haben,

   α) mit Isocyanaten der allgemeinen Formel (XII)

      R⁶-N=C=O (XII)

      in welcher
      - R⁶: die oben angegebene Bedeutung hat,
      gegebenenfalls in Gegenwart eines Verdünnungsmittels und gegebenenfalls in Gegenwart eines Katalysators umsetzt,
      oder
   β) mit Carbamidsäurechloriden oder Thiocarbamidsäurechloriden der allgemeinen Formel (XIII) 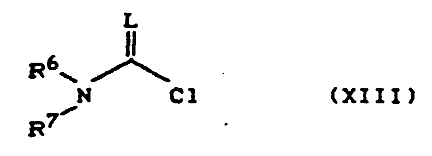 in welcher
      - L, R⁶ und R⁷: die oben angegebene Bedeutung haben,
      gegebenenfalls in Gegenwart eines Verdünnungsmittels und gegebenenfalls in Gegenwart eines Saurebindemittels umsetzt,
   oder
(H) man erhalt Verbindungen der Formel (Ig) 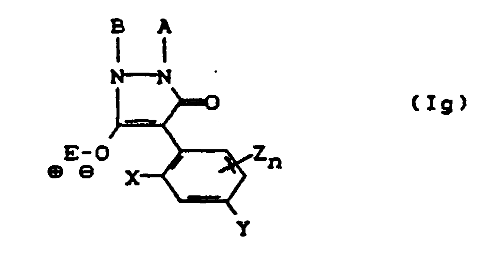 in welcher
   - A, B, X, Y, Z und n: die oben angegebene Bedeutung haben,
   und
   - E: für ein Metallionäquivalent oder für ein Ammoniumion steht,
   wenn man Verbindungen der Formel (Ia) 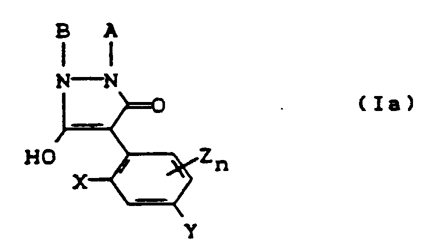 in welcher
   - A, B, X, Y, Z und n: die oben angegebene Bedeutung haben,
   mit Metallhydroxiden oder Aminen der allgemeinen Formeln (XIV) und (XV) 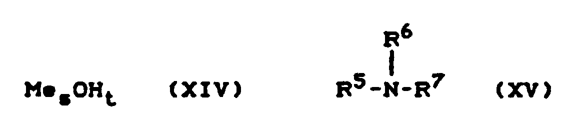 in welchen
   - Me: für ein- oder zweiwertige Metallionen,
   - s und t: für die Zahl 1 und 2 und
   - R⁵, R⁶ und R⁷: unabhängig voneinander für Wasserstoff und Alkyl
   stehen,
   gegebenenfalls in Gegenwart eines Verdünnungsmittels, umsetzt.

Weiterhin wurde gefunden, daß sich die neuen 3-Hydroxy-4-aryl-5-oxo-pyrazolin-Derivate der Formel (I) durch hervorragende insektizide, akarizide und herbizide Wirkungen auszeichnen.

Verwendet man beispielsweise gemäß Verfahren (A-α) (Chlorcarbonyl)-2,4,6-trimethylphenylketen und 1,2-Diazacyclopentan als Ausgangsverbindungen, so kann der Verlauf des erfindungsgemäßen Verfahrens durch folgendes Reaktionsschema wiedergegeben werden:

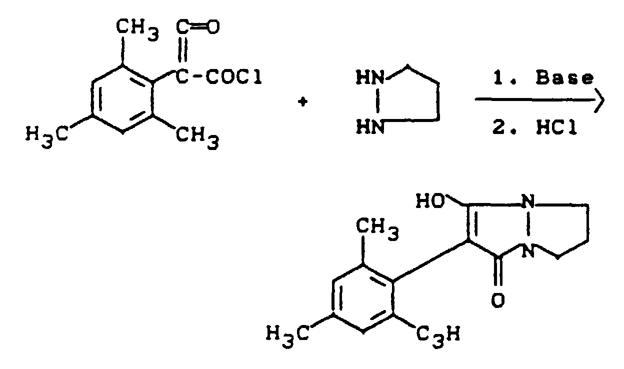

Verwendet man beispielsweise gemäß Verfahren (A-β) Mesitylmalonsäurediethylester und 1,2-Diazacyclopentan als Ausgangsverbindungen, so kann der Verlauf des erfindungsgemäßen Verfahrens durch das folgende Reaktionsschema wiedergegeben werden:

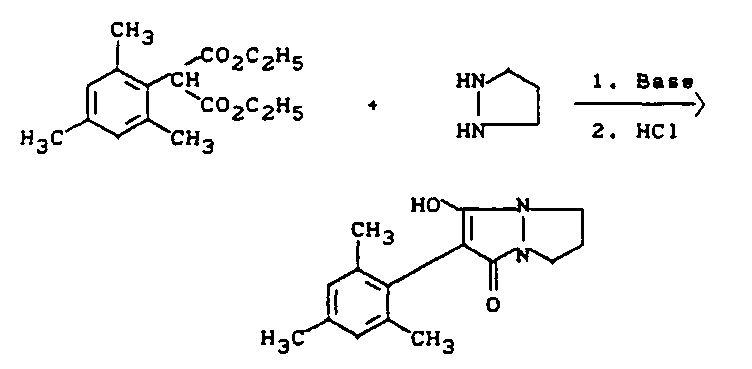

Verwendet man beispielsweise gemäß Verfahren (B-α) 3-(2,4,6-Trimethylphenyl)-1,5-diaza-bicyclo-(3,3,0^{1,5})-oktan-2,4-dion und Pivaloylchlorid als Ausgangsstoffe, so kann der Verlauf des erfindungsemäßen Verfahrens durch das folgende Formelschema wiedergegeben werden.

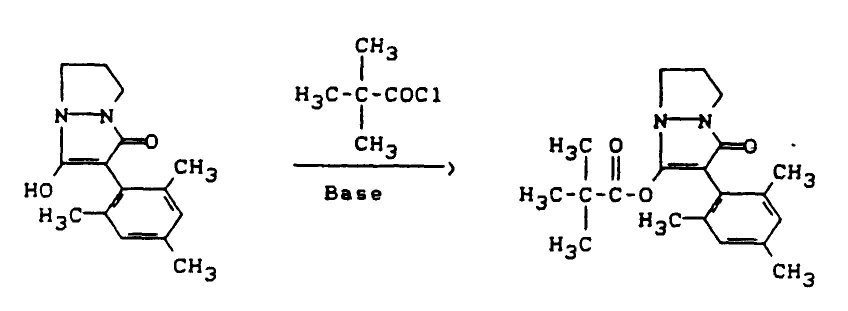

Verwendet man gemäß Verfahren (B-β) 8-(2,4,5-Trimethylphenyl)-1,6-diaza-bicyclo-(4,3,0^{1,6})-nonan-7,9-dion und Acetanhydrid als Ausgangsverbindungen, so kann der Verlauf des erfindungsgemäßen Verfahrens durch folgendes Reaktionsschema wiedergegeben werden.

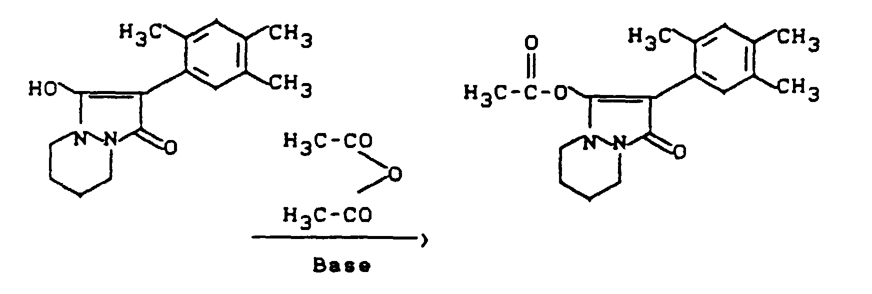

Verwendet man gemäß Verfahren (C) 8-(2,4-Dichlorphenyl)-1,6-diaza-bicyclo-(4,3,0^{1,6})-nonan-7,9-dion und Chlorameisensäureethoxyethylester als Ausgangsverbindungen, so kann der Verlauf des erfindungsgemäßen Verfahrens durch folgendes Reaktionsschema wiedergegeben werden.

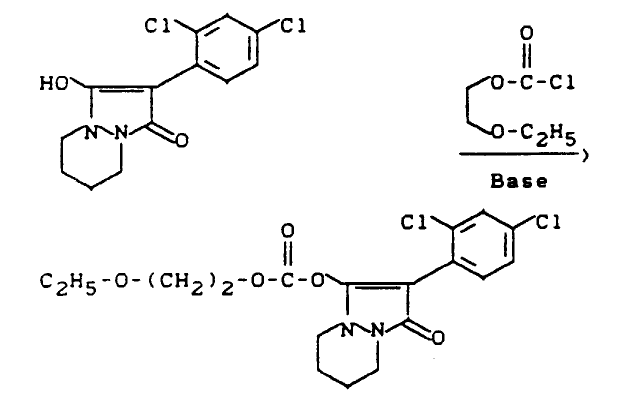

Verwendet man gemäß Verfahren (D-α) 3-(2,4,6-Trimethylphenyl)-1,5-diaza-bicyclo-(3,3,0^{1,5})-octan-2,4-dion und Chlormonothioameisensäuremethylester als Ausgangsprodukte, so kann der Reaktionsverlauf wie folgt wiedergegeben werden:

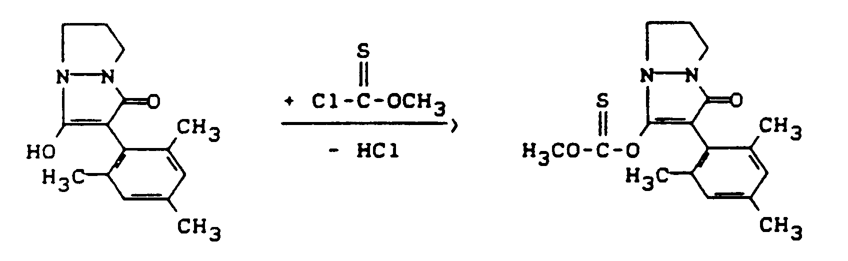

Verwendet man gemäß Verfahren (D-β) 3-(2,4,6-Trimethylphenyl)-1,5-diaza-bicyclo-(3,3-0^{1,5})-octan-2,4-dion, Schwefelkohlenstoff und Methyliodid als Ausgangskomponenten, so kann der Reaktionsverlauf wie folgt wiedergegeben werden:

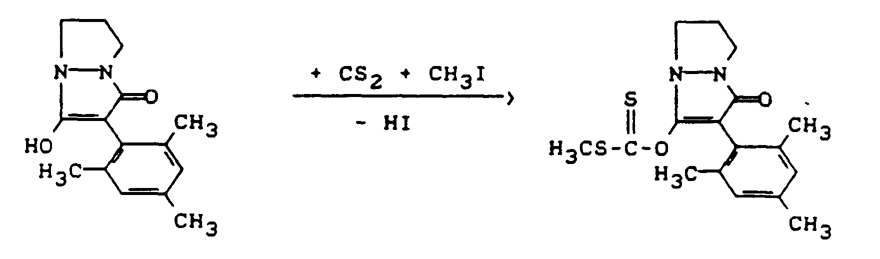

Verwendet man gemäß Verfahren (E) 3-(2,4,6-Trimethylphenyl)-1,5-diaza-bicyclo-(3,3,0^{1.5})-nonan-2,4-dion und Methansulfonsäurechlorid als Ausgangsprodukt, so kann der Reaktionsverlauf durch folgendes Reaktionsschema wiedergegeben werden:

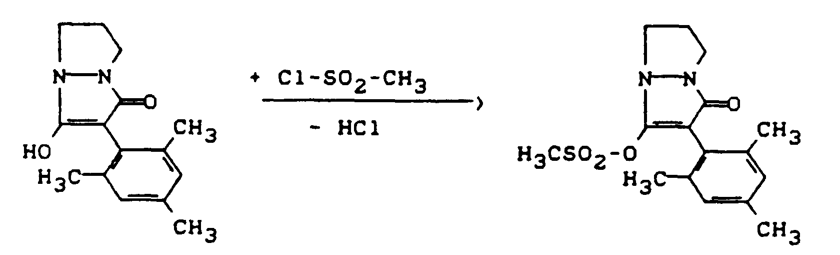

Verwendet man gemäß Verfahren (F) 3-(2,4,6-Trimethylphenyl)-1,5-diaza-bicyclo-(3,3,0^{1,5})-octan-2,4-dion und Methanthio-phosphonsäurechlorid-(2,2,2-trifluorethylester) als Ausgangsprodukte, so kann der Reaktionsverlauf durch folgendes Reaktionsschema wiedergegeben werden:

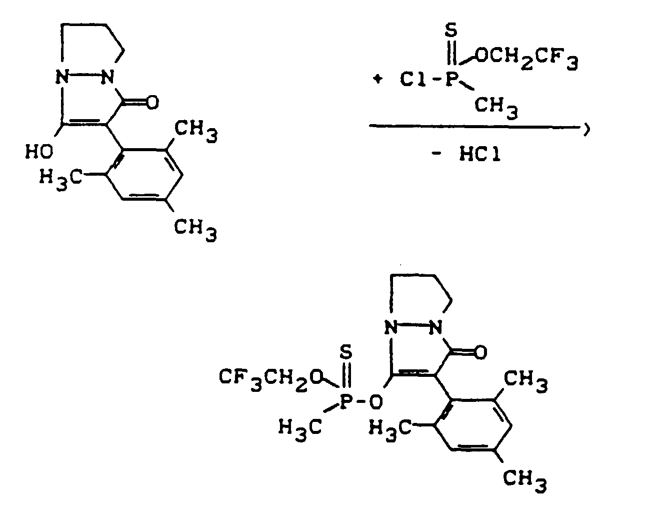

Verwendet man gemäß Verfahren (G-α) 3-(2,4,6-Trimethylphenyl)-1,5-diaza-bicyclo-(3,3,0^{1,5})-octan-2,4-dion und Ethylisocyanat als Ausgangsstoffe, so kann der Reaktionsverlauf durch das folgende Reaktionsschema wiedergegeben werden:

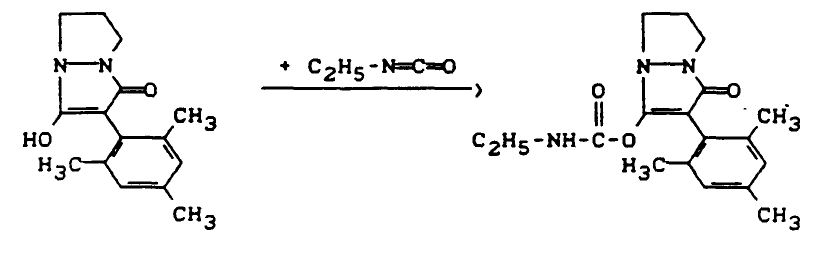

Verwendet man gemäß Verfahren (G-β) 3-(2,4,6-Trimethylphenyl)-1,5-diaza-bicyclo-(3,3,0^{1,5})-octan-2,4-dion und Dimethylcarbamidsäurechlorid als Ausgangsprodukte, so kann der Reaktionsverlauf durch folgendes Reaktionsschema wiedergegeben werden:

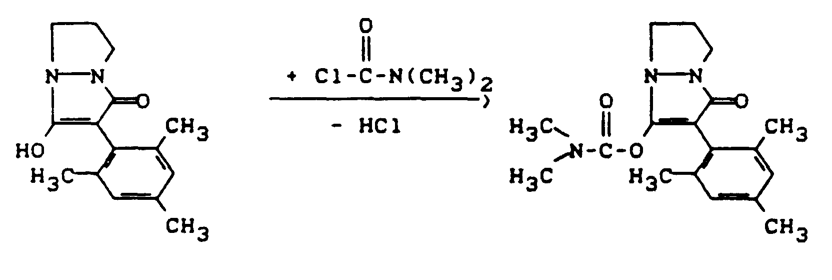

Verwendet man gemäß Verfahren (H) 3-(2,4,6-Trimethylphenyl)-1,5-diaza-bicyclo-(3,3,0^{1,5})-octan-2,4-dion und NaOH als Komponenten, so kann der Verlauf des erfindungegemäßen Verfahrens durch folgendes Reaktionsschema wiedergegeben werden:

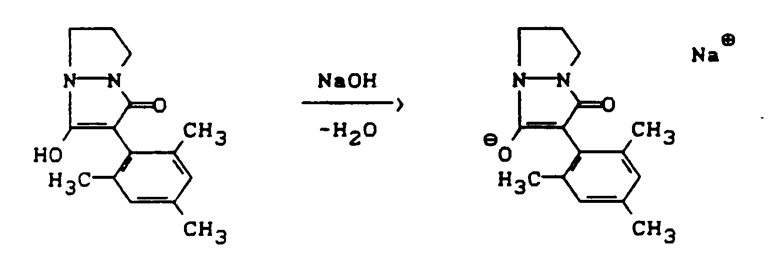

Die zur Durchführung des erfindungsgemäßen Verfahrens (A-α) als Ausgangsstoffe benötigten Halogencarbonylketene sind durch die Formel (II) allgemein definiert. In dieser Formel (II) stehen X, Y, Z, n und Hal vorzugsweise für diejenigen Reste, die bereits im Zusammenhang mit der Beschreibung der erfindungsgemäßen Stoffe der Formel (I) als bevorzugt für diese Substituenten genannt wurden.

Die Verbindungen der Formel (II) sind teilweise bekannt (vgl. beispielsweise Org. Prep. Proced.Int., 7(4), 155-8, 1975 und DE 19 45 703). Die noch nicht bekannten Verbindungen lassen sich aber nach im Prinzip bekannten Methoden in einfacher Weise analog herstellen. So erhält man z.B. Halogencarbonylketone der Formel (II), wenn man Arylmalonsäuren der Formel (XVI) 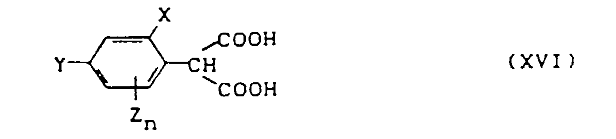 in welcher
- X, Y, Z und n: die oben angegebene Bedeutung haben,
mit Säurehalogeniden, wie beispielsweise Thionylchlorid, Phosphor(V)chlorid, Phosphor(III)chlorid, Oxalylchlorid, Phosgen oder Thionylbromid gegebenenfalls in Gegenwart von Katalysatoren, wie beispielsweise Diethylformamid, Methl-Sterylformamid oder Triphenylphosphin, umsetzt.

Die Arylmaionsäuren der Formel (XVI) sind z.T. allgemein bekannte Verbindungen der organischen Chemie (vgl. z.B. Organikum, VEB Deutscher Verlag der Wissenschaften, Berlin 1977, S.517 ff).

Neue Halogencarbonylketene besitzen die folgende Fomel (IIa) 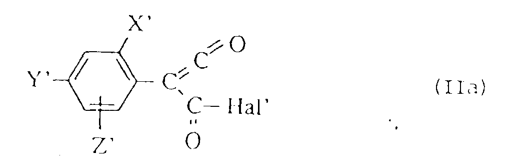 in welcher
- Hal': für Halogen steht
- X': für C₁-C₄-Alkyl, Halogen oderTrifluoromethyl steht
- Y': für Wasserstoff, C₁-C₄-Alkyl, Halogen oderTrifluoromethyl steht
und
- Z': für Wasserstoff, C₁-C₄-Alkyl, Halogen oderTrifluormethyl steht,
mit der Maßgabe, daß Z' nicht für Wasserstoff steht, wenn Y' für Wasserstoff steht.

Bevorzugte neue Halogencarbonylketene sind solche der Formel (IIa) in welcher
- Hal': für Brom oder Chlor steht
- X': für Methyl, Fluor, Chlor, Brom oderTrifluoromethyl steht,
- Y': für Wasserstoff, Methyl, Ethyl, n-Propyl, i-Propyl, n-Butyl, i-Butyl, tert.-Butyl, Fluor, Chlor, Brom oder Trifluormethyl steht
und
- Z': für Wasserstoff, Methyl, Fluor, Chlor, Brom oder Trifluormethyl steht,
mit der Maßgabe, daß Z' nicht für Wasserstoff steht, wenn Y' Wasserstoff bedeutet.

Besonders bevorzugte Halogencarbonylketene sind solche der Formel (IIa)
in welcher
entweder
X', Y' und Z' jeweils für Methyl stehen oder X' und Y' für Chlor stehen und gleichzeitig Z' Wasserstoff bedeutet.

Die neuen Halogencarbonylketene der Formel (IIa), in welcher X', Y', Z' und Hal' die oben angegebene Bedeutung haben, können durch Umsetzung von Arylmalonsäuren der Formel (XVIa) 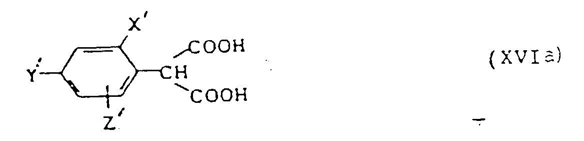 mit Säurehalogeniden wie beispielsweise Thionylchlorid, Phosphor(V)chlorid, Phosphor(III)chlorid, Oxalylchlorid, Phosgen oder Thionylbromid gegebenenfalls in Gegenwart von Katalysatoren, wie z. B. Diethylformamid, Methyl-stearylformamid der Triphenylphosphin erhalten werden.

Weiterhin sind die Ausgangsverbindungen Arylmalonsäuren der Formel (XVIa) in welcher X', Y' und Z' die oben angegebene Bedeutung haben, bisher noch nicht bekannte Verbindungen.

Die zur Durchführung des erfindungsgemäßen Verfahrens (A-β) als Ausgangsstoffe benötigten Malonsäureester sind durch die Formel (III) allgemein definiert. In dieser Formel (III) stehen X, Y, Z und n vorzugsweise für diejenigen Reste, die bereits im Zusammenhang mit der Beschreibung der erfindungsgemäßen Stoffe der Formel (I) als bevorzugt für diese Substituenten genannt wurden.

Die Aryimaionsäureester der Formel (III) sind allgemein bekannte Verbindungen der organischen Chemie (vgl. z.B. Tetrahedron Lett. 27, 2763 (1986) und Organikum VEB. Deutscher Verlag der Wissenschaften, Berlin 1977, S,587ff).

Die zur Durchführung der erfindungsgemäßen Verfahren (A-α) und (A-β) außerdem als Ausgangsstoffe benötigten Hydrazine der Formel (IV)

A-NH-NH-B (IV)

in welcher
- A und B: die oben angegebene Bedeutung haben,
sind teilweise bekannt und/oder nach literaturbekannten Methoden analog herstellbar (vgl. beispielsweise Liebigs Ann. Chem. 585, 6 (1954); Reaktionen der organischen Synthese, C.Ferri, Seite 212, 513; Georg Thieme Verlag Stuttgart, 1978; Liebigs Ann. Chem. 443, 242 (1925); Chem. Ber. 98, 2551 (1965)).

Die zur Durchführung der erfindungsgemäßen Verfahren (A-α) und (A-β) außerdem als Ausgangsstoffe benötigten Hydrazine der Formel (IVa) 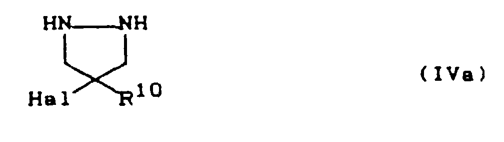 in welcher
- Hal: für Fluor oder Chlor steht und
- R¹⁰: für gegebenenfalls durch Halogen substituiertes C₁-C₈-Alkyl, C₂-C₈-Alkenyl, C₁-C₄-Alkoxy-C₂-C₄-alkyl steht
sind neu und Gegenstand der Erfindung.

Man erhält die neuen Verbindungen der Formel (IVa) 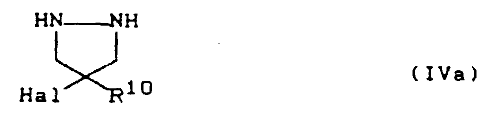 in welcher
- Hal und R¹⁰: die oben angegebene Bedeutung haben,
wenn man Verbindungen der Formel (XVII) 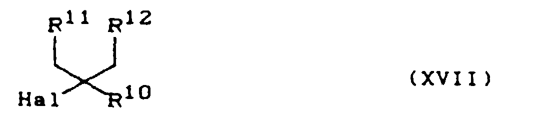 in welcher
- Hal und R¹⁰: die oben angegebene Bedeutung haben und
- R¹¹ und R¹²: für eine geeignete Abgangsgruppe, vorzugsweise für Halogen, insbesondere Chlor, Brom oder Iod, für Dialkylamino, wie beispielsweise Dimethylamino oder für jeweils gegebenenfalls substituiertes Alkylsulfonyloxy, Alkoxysulfonyloxy oder Arylsulfonyloxy, wie beispielsweise Methansulfonyloxy, Trifluormethansulfonyloxy, Methoxysulfonyloxy, Ethoxysulfonyloxy oder p-Toluolsulfonyloxy, stehen,
mit Hydrazinen bei 50 bis 150°C umsetzt (vgl. Herstellungsbeispiel).

Die Verbindungen der Formel (XVII) sind bekannt (vgl. beispielsweise Zh. Org. Khim. 19, 1107 (1983)).

Die zur Durchführung der erfindungsgemäßen Verfahren (B), (C), (D), (E), (F), (G) und (H) als Ausgangsstoffe benötigten 4-Aryl-pyrazolidin-3,5-dione bzw. deren Enole sind durch die Formel (Ia) allgemein definiert. In dieser Formel (Ia) stehen A, B, X, Y, Z und n vorzugsweise für diejenigen Reste, die bereits im Zusammenhang mit der Beschreibung der erfindungsgemäßen Stoffe der Formel (I) als bevorzugt für diese Substituenten genannt wurden.

Die Verbindungen der Formel (Ia) sind neu und Gegenstand der vorliegenden Erfindung. Sie sind erhältlich nach Verfahren (A).

Die zur Durchführung der erfindungsgemäßen Verfahren (B), (C), (D), (E), (F), (G) und (H) außerdem als Ausgangsstoffe benötigten Säurehalogenide der Formel (V), Carbonsäureenhydride der Formel (VI), Chlorameisensäureester oder Chlorameisensäurethioester der Formel (VII), Chlormonothioameisensäureester oder Chlordithioameisensäureester der Formel (VIII), Alkylhalogenide der Formel (IX), Sulfonsäurechloride der Formel (X), Phosphorverbindungen der Formel (XI) Isocyanate der Formel (XII), Carbamidsäurechloride der Formel (XIII) und Metailhydroxide oder Amine der Formel (XIV) und (XV) sind allgemein bekannte Verbindungen der organischen bzw. anorganischen Chemie.

Die Verfahren (A-α) und (A-β) sind dadurch gekennzeichnet, daß man Verbindungen der Formeln (II) oder (III) in welcher X, Y, Z, n und Hal die oben angegebene Bedeutung haben und Verbindungen der Formel (IV), in welcher A und B die oben angegebene Bedeutung haben, in Gegenwart von Basen einer Kondensation unterwirft.

Als Verdünnungsmittel können bei den erfindungsgemäßen Verfahren (A-α) und (A-β) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylother, außerdem polare Lösungsmittel, wie Dimethylsuifoxid, Sulfolan, Dimethylformamid und N-Methyl-pyrrolidon, sowie Alkohole wie Methanol, Ethanol, Propanol, Isopropanol, Butanol, iso-Butanol und tert.-Butanol.

Als Basen (Deprotonierungsmittel) können bei der Durchführung der erfindungsgemäßen Verfahren (A-α) und (A-β) alle üblichen Protononakzeptoren eingesetzt werden. Vorzugsweise verwendbar sind Alkalimetail- und Erdalkalimetall-oxide, -hydroxide und -carbonate, wie Natrium-hydroxid, Kaliumhydroxid, Magnesiumoxid, Calciumoxid, Natriumcarbonat, Kaliumcarbonat und Calciumcarbonat, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 oder TDA 1 eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natriumethylat und Kalium-tert.-butylat einsetzbar. Die Reaktionstemperaturen können bei der Durchführung der erfindungsgemäßen Verfahren (A-α) und (A-β) innerhalb eines größeren Bereiches variiert werden. Im allgemeinen arbeitet man bei Temperaturen zwischen -20°C und 250°C, vorzugsweise zwischen 0°C und 150°C.
Adogen 464 = Methyltrialkyl(C₈-C₁₀)ammoniumchlorid
TDA 1 = Tris-(methoxyethoxyethyl)-amin

Die erfindungsgemäßen Verfahren (A-α) und (A-β) werden im allgemeinen unter Normaldruck durchgeführt.

Bei der Durchführung der erfindungsgemäßen Verfahren (A-α) und (A-β) setzt man die Reaktionskomponenten der Formeln (II) und (IV) oder (III) und (IV) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (B-α) ist dadurch gekennzeichnet, daß man Verbindungen der Formel (Ia) mit Carbonsäurehalogeniden der Formel (V) umsetzt.

Als Verdünnungsmittel können bei dem erfindungsgemäßen Verfahren (Bα) bei Verwendung der Säurehalogenide alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Verwendet man die entsprechenden Carbonsäurehalogenide so kommen als Säurebindemittel bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, Diazabiyclooctan (DABCO), Diazabicycloundecan (DBU), Diszabicyclononen (DBN), Hüning-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat.

Die Reaktionstemperaturen können auch bei dem erfindungsgemäßen Verfahren (Bα) auch bei der Verwendung von Carbonsäurehalogeniden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (B-α) werden die Ausgangsstoffe der Formel (Ia) und das Carbonsäurehalogenid der Formel (V) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Das Verfahren (B-β) ist dadurch gekennzeichnet, daß man Verbindungen der Formel (Ia) mit Carbonsäurehydriden der Formel (VI) umsetzt.

Verwendet man bei dem erfindungsgemäßen Verfahren (B-β) als Reaktionskomponente der Formel (VI) Carbonsäureanhydride, so können als Verdünnungsmittel vorzugsweise diejenigen Verdünnungsmittei verwendet werden, die auch bei der Verwendung von Säurehalogeniden vorzugsweise in Betracht kommen. Im übrigen kann auch ein im Überschuß eingesetztes Carbonsäurehydrid gleichzeitig als Verdünnungsmittel fungieren.

Die Reaktionstemperaturen können bei dem erfindungsgemäßen Verfahren (B-β) auch bei der Verwendung von Carbonsäureanhydriden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (Ia) und das Carbonsäureanhydrid der Formel (VI) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisonsäureestern oder Chlorameisensäurethiolostern der Formel (VII) umsetzt.

Verwendet man die entsprechenden Chlorameisensäureester bzw. Chlorameisensäurethiolester so kommen als Säurebindemittel bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calcium-oxid, außerdem Alkali- und Erdalkalimetall-carbonate, wie Natriumcarbonat, Kaliumcarbonat und Calciumcarbonat.

Als Verdünnungsmittel können bei dem erfindungsgemäßen Verfahren (C) bei Verwendung der Chiorameisensäureester bzw. Chlorameisensäurethiolester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan.

Bei Verwendung der Chlorameisensäureester bzw. Chlorameisensäurethiolester als Carbonsäure-Derivate der Formel (VII) können die Reaktionstemperaturen bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende Chlorameisensäureester bzw. Chlorameisensäurethiolester der Formel (VII) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Beim Herstellungsverfahren (D-α) setzt man pro Mol Ausgangsverbindung der Formel (Ia) ca. 1 Mol Chlormonothioameisensäureester bzw. Chlordithioameisensäureester der Formel (VII) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindung Ia dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (D-β) setzt man pro Mol Ausgangsverbindung der Formel (Ia) die äquimolare Menge bzw. einen Überschuß Schwefelkohlanstoff zu. Man arbeitet hierbei vorzugsweise bei Temperaturen von 0 bis 50°C und insbesondere bei 20 bis 30°C.

Oft ist es zweckmäßig zunächst aus der Verbindung der Formel (Ia) durch Zusatz eines Deprotonierungsmittels (wie z.B. Kaliumtertiärbutylat oder Natriumhydrid) das entsprechende Salz herzustellen. Man setzt die Verbindung (Ia) solange mit Schwefeikohlenstoff um, bis die Bildung der Zwischenverbindung abgeschlossen ist, z.B. nach mehrstündigem Rühren bei Raumtemperatur.

Die weitere Umsetzung mit dem Alkylhalogenid der Formel (IX) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Beim Herstellungsverfahren (E) setzt man pro Mol Ausgangsverbindung der Formel (Ia) ca. 1 Mol Sulfonsäurechlorid (X) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung Ia dar, kann auf den weitern Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (E) kann gegebenenfalls unter Phasen-Transfer-Bedingungen gearbeitet werden (W.J. Spillane et. al.; J. Chem. Soc., Perkin Trans I, (3) 677-9 (1982)). In diesem Fall setzt man pro Mol Ausgangsverbindung der Formel Ia) 0,3 bis 1,5 mol Sulfonsäurechlorid (X), bevorzugt 0,5 mol bei 0° bis 150°C, vorzugsweise bei 20 bis 70°C um.

Als Phasen-Transfer-Katalysatoren können alle quartären Ammoniumsalze verwendet werden, vorzugsweise Tetraoctylammoniumbromid und Benzyltriethylammoniumchlorid. Als organische Lösungsmittel können in diesem Fall alle unpolaren inerten Lösungsmittel dienen, bevorzugt werden Benzol und Toluol eingesetzt.

Beim Herstellungsverfahren (F) setzt man zum Erhalt von Verbindungen der Struktur (Ie) auf 1 Mol der Verbindung (Ia), 1 bis 2, vorzugsweise 1 bis 1,3 Mol der Phosphorverbindung der Formel (XI) bei Temperaturen zwischen -40°C und 150°C, vorzugsweise zwischen -10 und 110°C um.

Als gegebenenfalls zugesetzte Verdünnungsmittel kommen aller inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Dimethylsulfid eingesetzt.

Als gegebenenfalls zugesetzte Säurebindemittel kommen übliche anorganische oder organische Basen in Frage wie Hydroxide, Carbonate. Beispielhaft seien Natriumhydroxid, Natriumcarbonat, Kaliumcarbonat, Pyridin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillioren", d.h. Entfernung der flüchtigen Bestandteile im Vakuum.

Bei Herstellungsverfahren (G-α) setzt man pro Mol Ausgangsverbindung der Formel (Ia) ca. 1 Mol Isocyanat der Formel (XII) bei 0 bis 100°C, vorzugsweise bei 20 bis 50°C um.

Als gegebenenfalls zugesetzte Verdünnungsmittel kommen alle inerte organische Lösungsmittel infrage, wie Ether, Amide, Nitrile, Sulfone und Sulfoxide.

Gegebenenfalls können Katalysatoren zur Beschleunigung der Reaktion zugesetzt werden. Als katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (G-β) setzt man pro Mol Ausgangsverbindung der Formel (Ia) ca. 1 Mol Carbamidsäurechlorid bzw. Thiocarbamidsäurechlorid der Formel (XIII) bei 0 bis 150°C, vorzugsweise bei 20 bis 70°C um.

Als gegebenenfalls zugesetzte Verdünnungsmittel kommen alle inerten polaren organischen Lösungsmittel infrage, wie Ether, Amide, Alkohole, Sulfone und Sulfoxide.

Vorzugsweise werden Dimethylsulfoxid, Tetrahydrofuran, Dimethylformamid und Dimethylsulfoxid eingesetzt.

Stellt man in einer bevorzugten Ausführungsform durch zusatz von starken Deprotonierungsmitteln (wie z.B. Natriumhydrid oder Kaliumtertiärbutylat) das Enolatsalz der Verbindung (Ia) dar, kann auf den weiteren Zusatz von Säurebindemitteln verzichtet werden.

Werden Säurebindemittel eingesetzt, so kommen übliche anorganische oder organische Basen infrage, beispielhaft seien Natriumhydroxid, Natriumcarbonat, Kaliumcarbonat und Pyridin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das Verfahren (H) ist dadurch gekennzeichnet, daß man Verbindungen der Formel (Ia) mit Metallhydroxiden (XIV) oder Aminen (XV) umsetzt.

Als Verdünnungsmittel können bei dem erfindungsgemäßen Verfahren vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (H) wird im allgemeinen unter Normaldruck durchgeführt. Die Reaktionstemperaturen liegen im allgemeinen zwischen -20°C und 100°C, vorzugsweise zwischen 0°C und 50°C.

Bei der Durchführung des orfindungsgemäßen Verfahrens (H) werden die Ausgangsstoffe der Formel (Ia) bzw. (XIV) oder (XV) im allgemeinen in angenähert äquimolaren Mengen verwendet. Es ist jedoch auch möglich, die eine oder andere Komponente in einem größeron Überschuß (bis zu 2 Mol) einzusetzen. Im allgemeinen geht man so vor, daß man das Reaktionsgemisch durch Abziehen des Verdünnungsmittel einengt.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletig blancardella, Hyponomeuta padella, Plutella maculipennis, Malacosoma neustrim, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Raupen der Kohlschabe (Plutella maculipennis) oder gegen pflanzenschädigenden Milben, wie spielsweise gegen die gemeine Spinnmilbe oder die Bohnenspinnmilbe (Tetranychus urticae) einsetzen.

Darüber hinaus zeigen die erfindungsgemäßen Wirkstoffe außerdem eine nematizide Wirkung.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endparasitischlebende Würmer.

Sie sind gegen normalsensible und resistente Arten und Stämme sowie gegen alle parasitierenden und nicht parasitierenden Entwicklungsstadien der Ekto- und Endoparasiten wirksam.
Die erfindungsgemäßen Verbindungen weisen auch eine Wirksamkeit gegenüber Protozoen und insbesondere eine coccidiostatische Wirksamkeit auf.
Die erfindungsgemäßen Wirkstoffe können weiterhin als Defoliants, Desiccants, Krautabtötungsmittel und insbesondere als Unkrautvernichtungsmittel verwendet erden. Unter Unkraut im weitesten Sinne sind alle Fflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.
Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulata, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeapsis, Papaver, Centaurea, Trifclium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphehoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachauflaufverfahren. Sie können beispielsweise in Soja oder Zuckerrüben mit sehr gutem Erfolg zur Bekämpfung von Schadgräsern eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. Weiterhin kommen 5-(2-Chlor-4-trifluormethyl-phenoxy)-2-nitrobenzoesäure (ACIFLUORFEN); 3-Isopropyl-2,1,3-benzothiadiazin-4-on-2,2-dioxid (BENTAZON); Methyl-5-(2,4-dichlorphenoxy)-2-nitrobenzoat (BIFENOX); 5-Amino-4-chlor-2-phenyl-2,3-dihydro-3-oxy-pyridazin (CHLORIDAZON); ethyl-2-{[(4-chlor-6-methoxy-2-pyrimidinyl)-aminocarbonyl]-aminosulfonyl}-benzoat (CHLORIMURON); 4-Amino-6-t-butyl-3-ethylthio-1,2,4-triazin-5(4H)-on (ETHIOZIN); 5-(2-Chlor-4-trifluormethyl-phenoxy)-N-methylsulfonyl-2-nitrobenzamid (FOMESAFEN); 2-[5-Methyl-5-(1-methylethyl)-4-oxo-2-imidazolin-2-yl]-3-chinolincarbonsäure (IMAZAQUIN); N-Methyl-2-(1,3-benzthiazol-2-yloxy)-acetanilid (MEFENACET); 1-(3-Trifluormethyl-phenyl)-4-methylamino-5-chlor-6-pyridazon (NORFLURAZON); N-(1-Ethylpropyl)-3,4-dimethyl-2,6-dinitroanilin (PENDIMETHALIN); 3-(Ethoxycarbonylaminophenyl)-N-(3'-methylphenyl)-carbamat (PHENMEDIPHAM); 3-[[[[(4-Methoxy-6-methyl-1,3,5-triazin-2-yl)-amino]-carbonyl]-amino]-sulfonyl]-thiophen-2-carbonsäure-methylester (THIAMETURON); 2,6-Dinitro-4-trifluormethyl-N,N-dipropylanilin (TRIFLURALIN) infrage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und Verwendung der erfindungsgemäßen Wirkstoffe geht aus den nachfolgenden Beispielen hervor, wobei nach den Beispielen 1, 20 ,47 und 51 keine erfindungsgemäßen Verbindungen erhälten werden.

### Herstellungsbeispiele:

### Beispiel 1

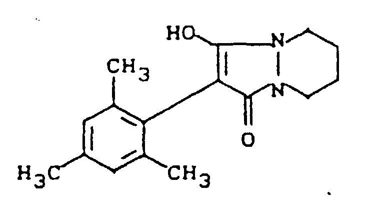

22,5 g (0,1 Mol) Mesitylchlorcarbonylketen werden in 200 ml Ether vorgelegt und bei 20°C mit 8,7 g (0,1 Mol) Piperidazin, 16,5 ml (0,11 Mol) Triethylamin und 10 ml Ether versetzt. Man rührt einen Tag bei 50°C, filtriert das entstandene Hydrochlorid ab und destilliert das Lösungsmittel im Wasserstrahlvakuum ab. Anschließend wird der Rückstand in 50 ml Tetrahydrofuran aufgenommen und diese Lösung in 1000 ml einer HCl/Eis-Mischung eingerührt. Nach Absaugen des Feststoffes und anschließender Trocknung erhält man 18,4 g (66 % der Theorie) 3-Hydroxy-4-mesityl-5-oxo-1,2-tetramethylenpyrazolin vom Schmelzpunkt 190°C.

### Beispiel 2

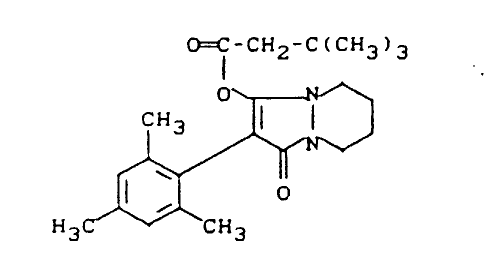

27,3 g (0,1 Mol) 3-Hydroxy-4-mesityl-5-oxo-1,2-tetramethylen-pyrazolin und 30 ml Triethylamin (0,2 Mol) werden in 500 ml Tetrahydrofuran gelöst und bei 20°C mit 13 g (0,12 Mol) 3,3-Dimethylbuttersäurechlorid versetzt. Man rührt 4 Stunden bei 40°C und gießt dann die Reaktionsmischung in 1000 ml Wasser. Der entstandene Feststoff wird abgesaugt und getrocknet.
Man erhält 34 g (92 % der Theorie) 5-Oxo-3-neopentylcarbonyloxy-4-mesityl-1,2-tetramethylen-pyrazolin vom Schmelzpunkt 76°C.

In analoger Weise zu Beispiel 1 und 2 und unter Berücksichtigung der Angaben in der Beschreibung zu den erfindungsgemäßen Verfahren, werden die nachfolgend in Tabelle 1 aufgeführten Endprodukte der Formel (I) 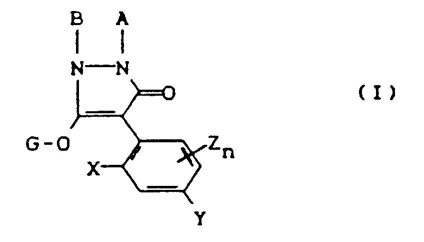 erhalten.

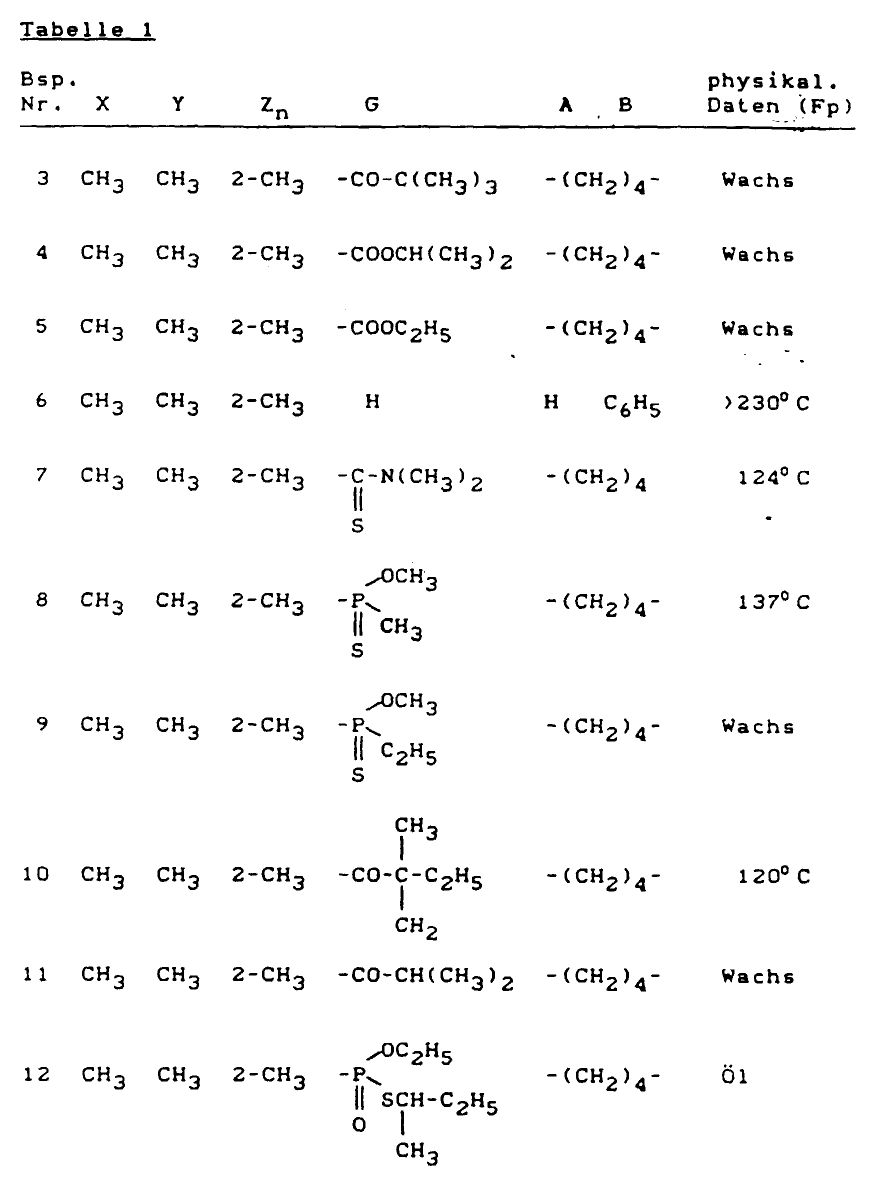

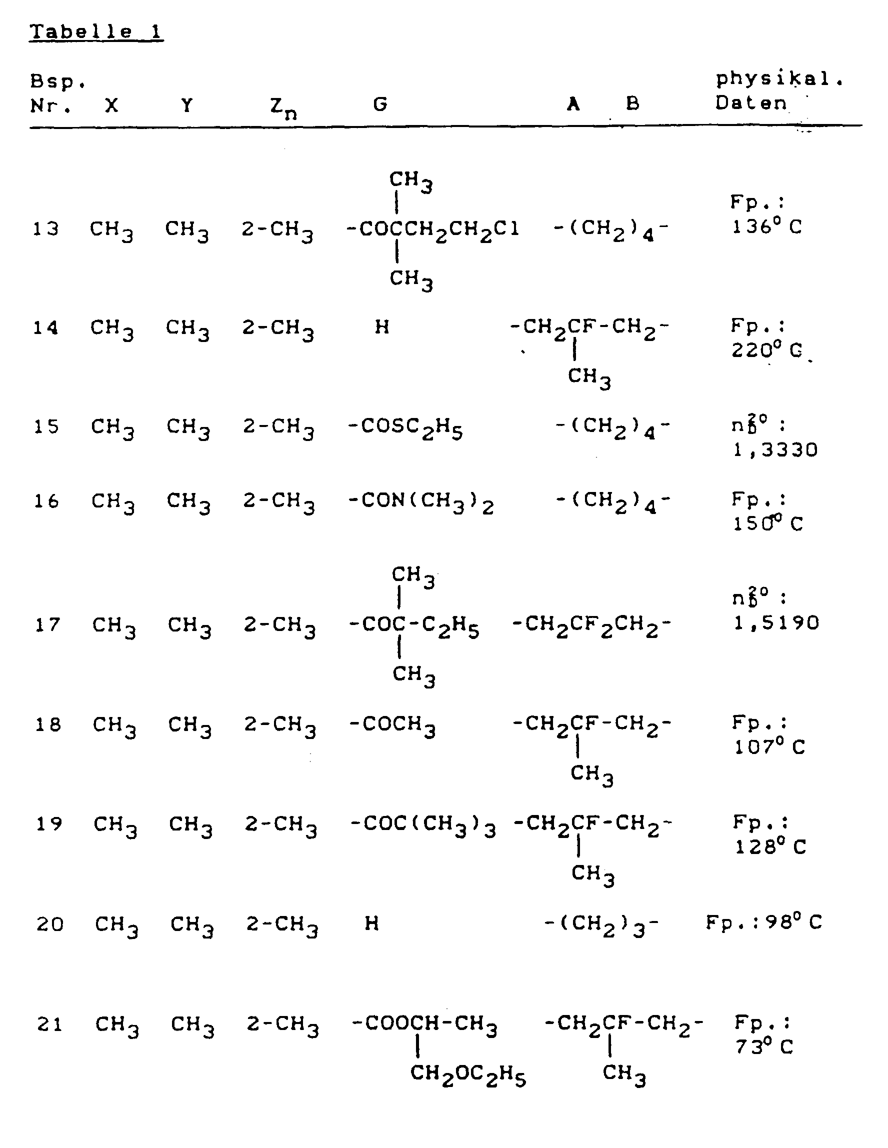

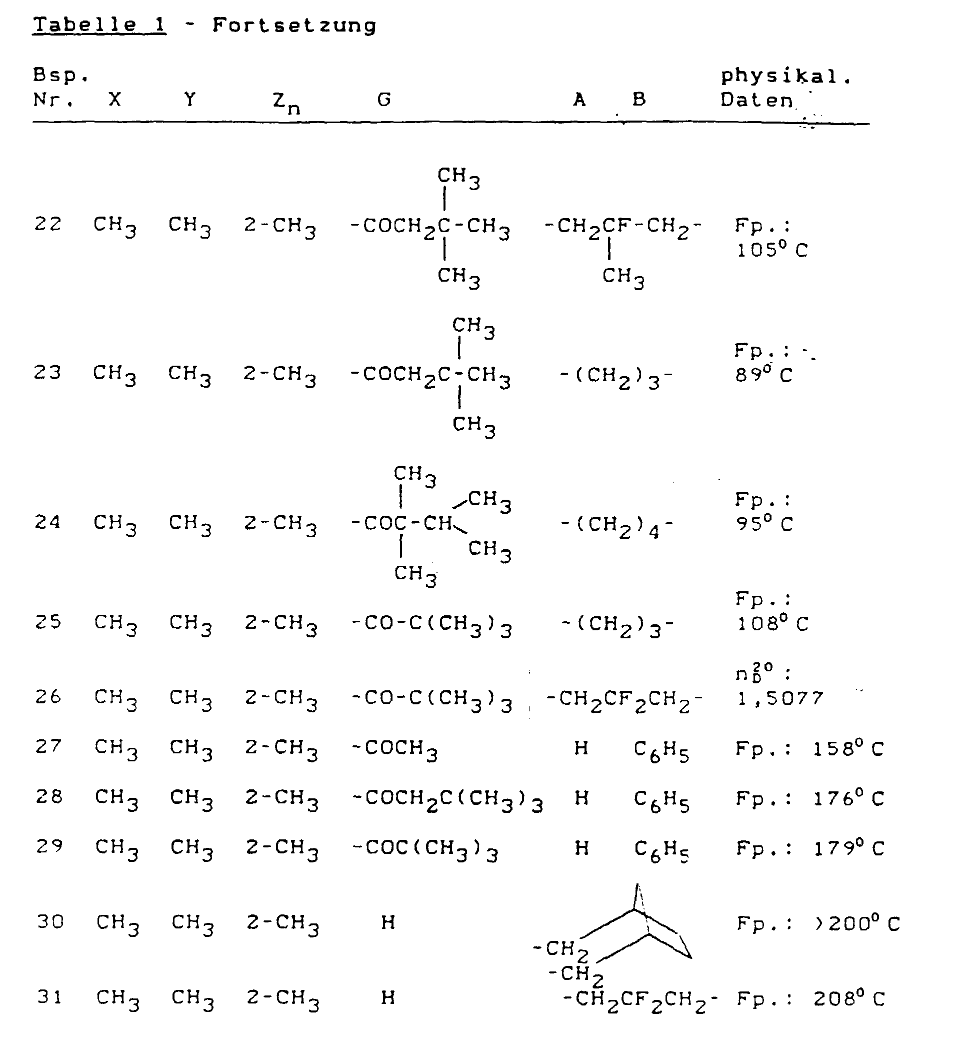

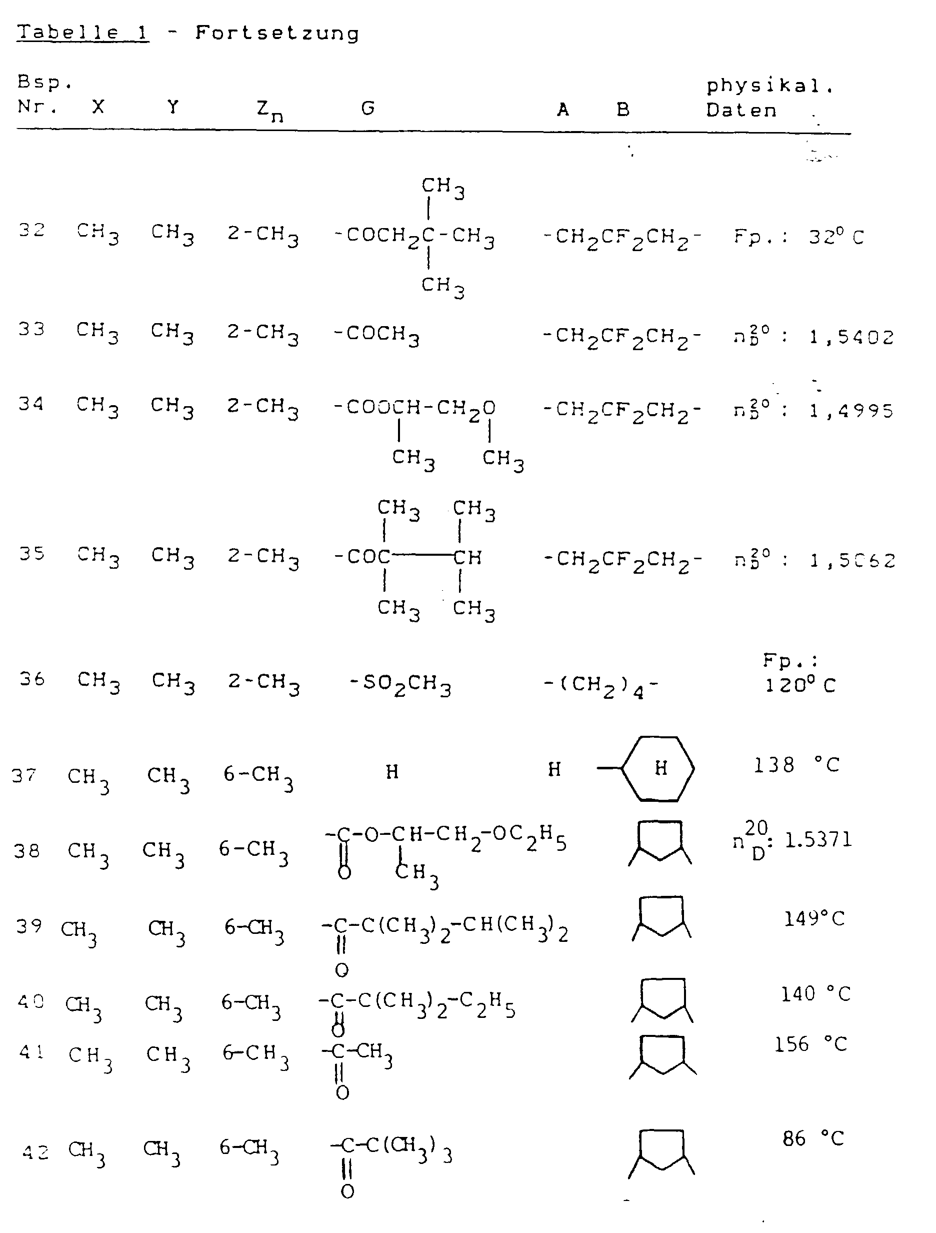

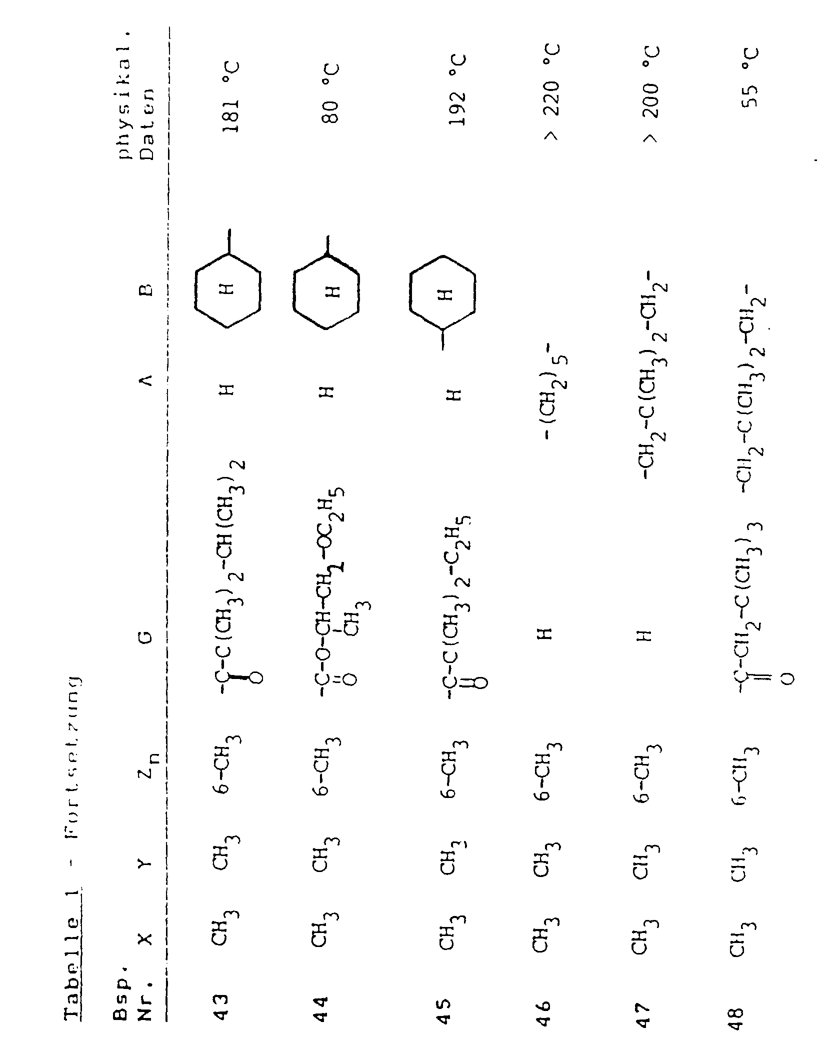

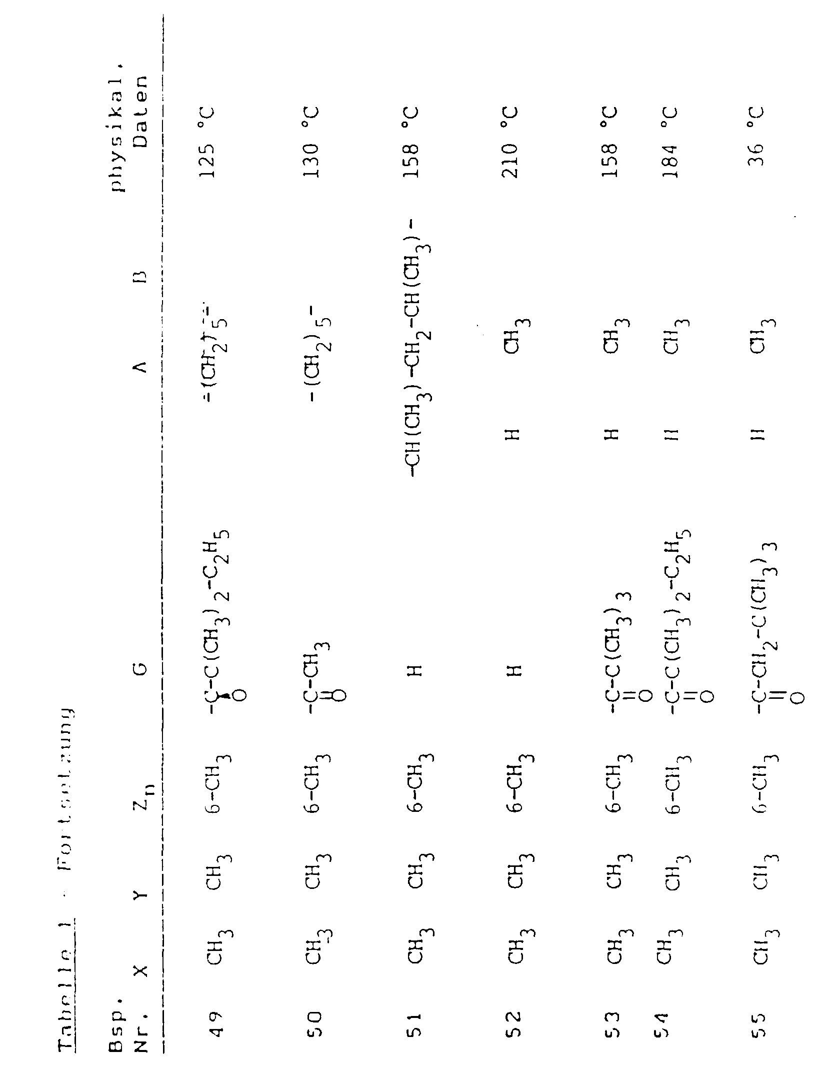

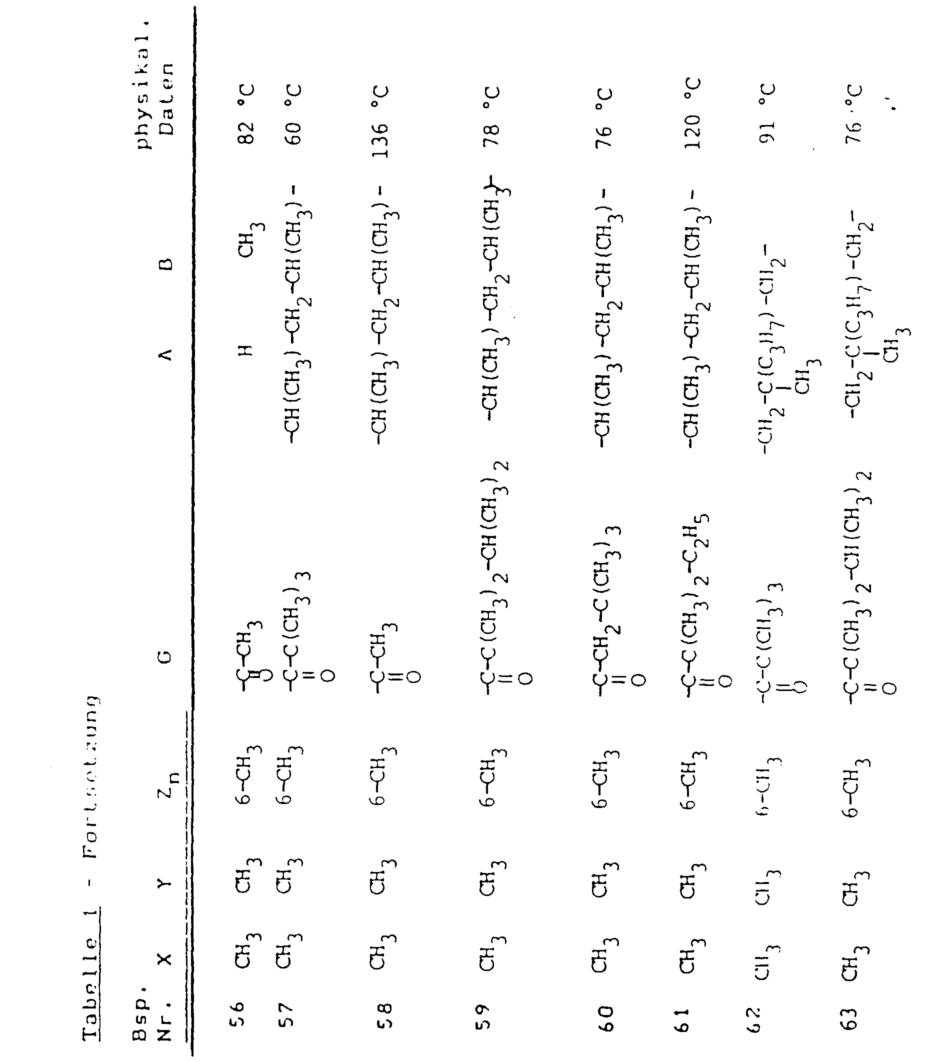

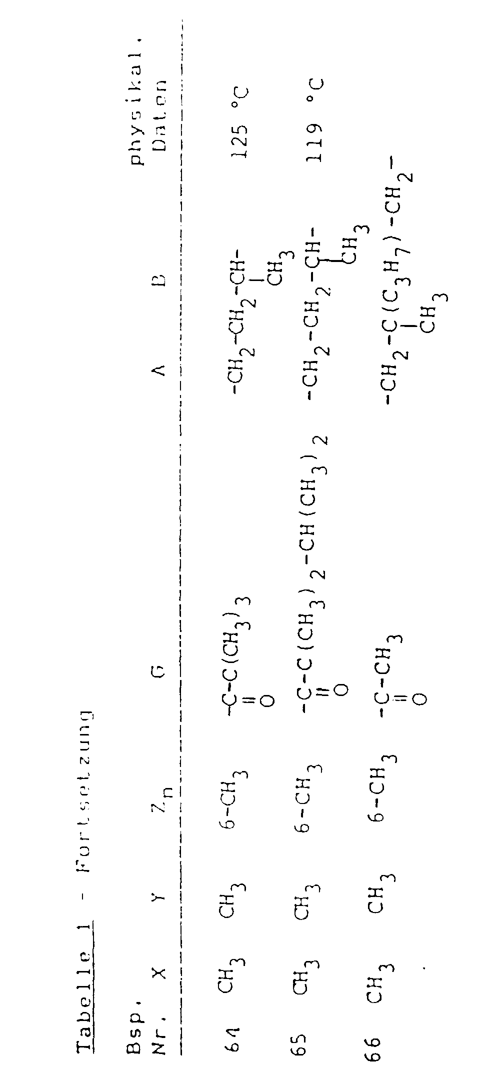

### Herstellung der Ausgangsverbindungen:

### Beispiel (II-1)

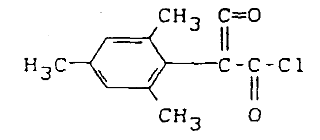

444,5 g (2 Mol) Mesitylmalonsäure werden in 1000 ml Methylcyclohexan bei 75-80°C suspendiert und 714 g (6 Mol) Trionylchlorid innerhalb von 3 Stunden zugetropft. Dann wird langsam weiter erwärmt und 8 Stunden unter Rückflußkühlung bei 110-120°C Badtemperatur nachgerührt.
Überschüssiges Thionylchlorid wird zusammen mit dem Lösungsmittel bei 10 mbar bis 80°C Badtemperatur abdestilliert, der Rückstand nach dem Erkalten mit der 3-fachen Mengen Petrolether verdünnt, filtriert, eingeengt und destilliert.
Man erhält 373 g (84 % der Theorie) Mesitylchlorcarbonylketen vom Siedepunkt 96°/0,45 mbar.

In analoger Weise zu Beispiel (II-1) und unter Berücksichtigung der Angaben in der Beschreibung zu den erfindungsgemäßen Verfahren, werden die nachstehend in Tabelle 2 aufgeführten Ausgangsverbindungen der Formel (II) 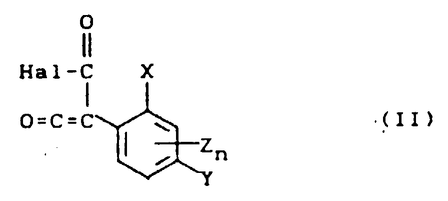 erhalten:

| Bsp.Nr. | X | Y | Zn | physikal.Konst. Kp [°C/mbar] |
|---|---|---|---|---|
| II-2 | H | H | 3-CF₃ | 65-69/0,05 |
| II-3 | Cl | H | H | 93-95/0,15 |
| II-4 | H | C(CH₃)₃ | H | 105-110/0,5 |

### Herstellung der Vorprodukte:

### Beispiel (XV-1)

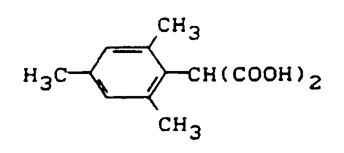

881 g (3,52 Mol) Mesitylmalonsäure-dimethylester und 690 g (12,32 Mol) Kaliumhydroxid werden in einer Mischung aus 880 ml Wasser und 1760 ml Methanol 12 Stunden gekocht, auf Raumtemperatur abgekühlt und bei einer Badtemperatur von ≤ 30°C eingeengt. Der zähe Rückstand wird in 2 l Wasser aufgenommen, die klare Lösung mit 1060 ml konzentrierter Salzsäure auf pH 1 gebracht und im Eisbad gekühlt. Der ausgefallene kristalline Niederschlag wird abgesaugt, mehrmals mit kaltem Wasser gewaschen, über Nacht an der Luft und anschließend über Kaliumhydroxid bei 40°C und 10 mbar getrocknet .

Man erhält 757 g (97 % der Theorie) Mesitylmalonsäure vom Schmelzpunkt 182-184°C (Zers.).

In analoger Weise zu Beispiel (XV-1) und unter Berücksichtigung der Angaben in der Beschreibung zu den erfindungsgemäßen Verfahren, werden die nachfolgend in Tabelle 3 aufgeführten Vorprodukte der Formel (XV). 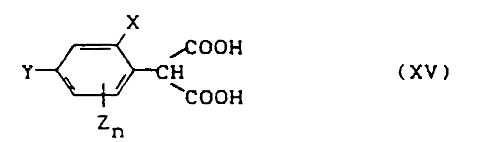 erhalten:

| Bsp.Nr. | X | Y | Zn | physikal.Konst. |
|---|---|---|---|---|
| XV-2 | H | H | 3-CF₃ | Fp.:123°C(Zers.) |
| XV-3 | Cl | H | H | Fp.:78-81°C (Zers.) |
| XV-4 | H | C(CH₃)₃ | H | Fp.:150-151°C (Zers.) |

284 g (1,5 Mol) 2-Fluor-2-chlormethyl-1-brompropan werden bei Raumtemperatur zu einer Mischung von 144 g (4,5 Mol) reinem Hydrazin und 1000 ml Hydrazinhydrat gegeben und 3 Stunden bei 100°C gerührt. nach dem Abkühlen wird das Reaktionsgemisch 18 Stunden mit Diethylether im Perforator extrahiert. Die Etherlösung wird mit Kaliumhydroxid-Plätzchen getrocknet und eingeengt. Der Rückstand wird bei 10 mbar destilliert.
Man erhält 118 g (68 % der Theorie) 4-Fluor-4-methylpiperidazin (98 %ig nach Gaschromatographie).

### Anwendungsbeispiele:

In den folgenden Anwendungsbeispielen wurde die nachstehend aufgeführte Verbindung als Vergleichssubstanz eingesetzt: 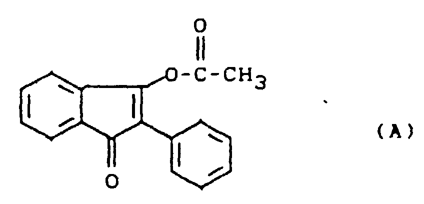 3-(Acetyloxy)-2-phenyl-1H-inden-1-on (bekannt aus US 410 40 43)

### Beispiel A

### Tetranychus-Test (resistent)

- Lösungsmittel:: 7 Gewichtsteile Dimethylformamid
- Emulgator:: 1 Gewichtsteil Alkylarylpolyglykolether

Zur Herstellung einer zwechmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 1, 3, 4, 5 und 6.

### Beispiel B

### Tetranychus-Test (OP-resistent)

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden mit einer Wirkstoffzubereitung der gewünschten Konzentration tropfnass gespritzt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 2, 8, 9, 10, 11 und 12.

### Beispiel C

### Plutella-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigten z.B. die fogenden Verbindungen der Herstellungsbeispiele überlegene Wirksamkeit gegenüber dem Stand der Technik: 2, 4, 5, 8, 9, 10 und 13.

### Beispiel D

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
- 0 % =: keine Wirkung (wie unbehandelte Kontrolle)
- 100 % =: totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele: 1, 2, 3, 4, 5, 8, 9, 10 und 11.

### Beispiel E

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
- 0 % =: keine Wirkung (wie unbehandelte Kontrolle)
- 100 % =: totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele: 1, 3, 4, 5, 8, 9, 10 und 11.

### Beispiel F

### Test mit Lucilia cuprina resistent-Larven

- Emulgator:: 35 Gewichtsteile Ethylenglykolmonomethylether
35 Gewichtsteile Nonylphenyolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die jeweils gewünschte Konzentration.

Etwa 20 Lucilia cuprina res.-Larven werden in ein Test-röhrchen gebracht, welches ca. 1 cm³ Pferdefleisch und 0,5 ml der Wirkstoffzubereitung enthält. Nach 24 Stunden wird der Abtötungsgrad bestimmt.

Hierbei zeigten die Wirkstoffe gemäß den Beispielen Nr. 1, 3, 4, 5, 7, 8, 9, 10, 11, 12, 13, 18, 19, 21, 22 und 24 bei einer Wirkstoffkonzentration von 1 % eine abtötende Wirkung von 100 %.

## Patentansprüche (Patentansprüche für folgende(n) Vertragsstaat(en): BE, CH, DE, FR, GB, GR, IT, LI, NL)

1. 3-Hydroxy-4-aryl-5-oxo-pyrazolin-Derivate der Formel (I) 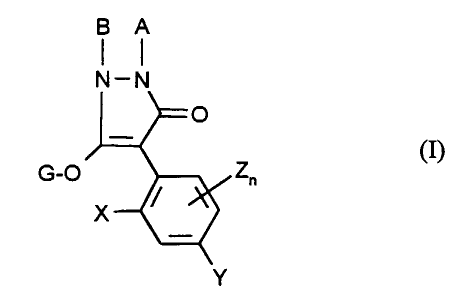 in welcher
A und B gleich oder verschieden sind und unabhängig voneinander jeweils für C₁-C₄-Alkyl, C₂-C₄-Alkenyl_{,} C₂-C₄-Alkinyl_{,} C₁-C₄-Alkoxy-C₁-C₄-alkyl, C₁-C₄-Alkylthio-C₁-C₄-alkyl, Cycloalkyl mit 3 bis 7 Kohlenstoffatomen oder für gegebenenfalls einfach bis dreifach, gleich oder verschieden substituiertes Phenyl stehen,
wobei als Phenylsubstituenten die für R³, R⁴ und R⁵ aufgeführten Phenylsubstituenten in Frage kommen oder
A und B zusammen mit den beiden Stickstoffatomen des Pyrazolinringes für eine gegebenenfalls einfach bis dreifach, gleich oder verschieden substituierte Gruppierung der nachfolgend aufgeführten Formeln 1', 2', 3' oder 4' stehen 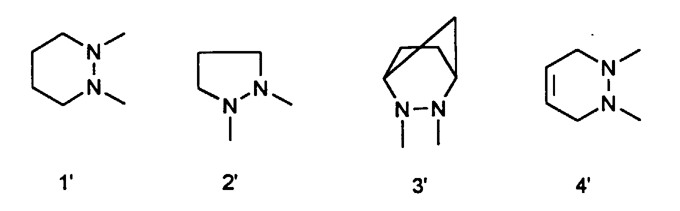 wobei als Substituenten Halogen, gegebenenfalls durch Fluor oder Chlor substituiertes C₁-C₈-Alkyl, C₂-C₈-Alkenyl und C₁-C₄-AlkoxyC₁-C₄-alkyl in Frage kommen,
X für Methyl, Ethyl, Propyl, i-Propyl, Fluor, Chlor, Brom, Methoxy und Ethoxy steht,
Y für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,
Z für Methyl, Ethyl, i-Propyl, Butyl, i-Butyl, tert.-Butyl, Fluor, Chlor, Brom, Methoxy und Ethoxy steht,
n für eine Zahl 0, 1, 2 oder 3 steht,
G für Wasserstoff (a) oder für die Gruppen 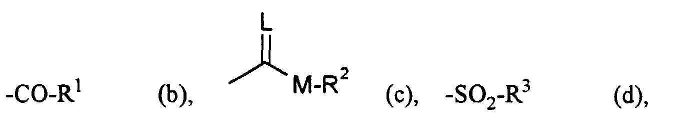 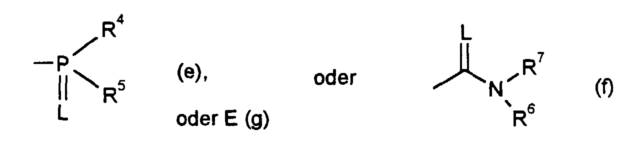 steht,
in welchen
E für ein Metallionäquivalent oder ein Ammoniumion steht,
L und M für Sauerstoff und/oder Schwefel stehen,
R¹ für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Alkylthio-C₂-C₆-alkyl, C₁-C₄-Polyalkoxy-C₂-C₄-alkyl oder Cycloalkyl mit 3 bis 6 Ringatomen, das durch 1 bis 2 Sauerstoff- und/oder Schwefelatome unterbrochen sein kann, steht,
für gegebenenfalls durch Fluor, Chlor, Brom, Methyl, Ethyl, Propyl, i-Propyl, Methoxy, Ethoxy, Trifluormethyl, Trifluormethoxy, Nitro substituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy substituiertes Phenyl-C₁-C₃-alkyl steht,
für gegebenenfalls durch Fluor, Chlor, Brom, Methyl, Ethyl substituiertes Pyridyl, Pyrimidyl, Thiazolyl und Pyrazolyl steht,
für gegebenenfalls durch Fluor, Chlor, Methyl, Ethyl substituiertes Phenoxy-C₁-C₄-alkyl steht,
für gegebenenfalls durch Fluor, Chlor, Amino, Methyl, Ethyl substituiertes Pyridyloxy-C₁-C₄-alkyl, Pyrimidyloxy-C₁-C₄-alkyl und Thiazolyloxy-C₁-C₄-alkyl steht,
R² für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Polyalkoxy-C₂-C₆-alkyl steht,
oder für gegebenenfalls durch Fluor, Chlor, Nitro, Methyl, Ethyl, Propyl, i-Propyl, Methoxy, Ethoxy, Trifluormethyl substituiertes Phenyl oder Benzyl steht,
R³, R⁴ und R⁵ unabhängig voneinander für gegebenenfalls durch Fluor oder Chlor substituiertes C₁-C₄-Alkyl, C₁-C₄-Alkoxy, C₁-C₄-Alkylamino, Di-(C₁-C₄)-alkylamino, C₁-C₄-Alkylthio, für gegebenenfalls durch Fluor, Chlor, Brom, Nitro, Cyano, C₁-C₂-Alkoxy, C₁-C₄-Fluoralkoxy, C₁-C₂-Chloralkoxy, C₁-C₂-Alkylthio, C₁-C₂-Fluoralkylthio, C₁-C₂-Chloralkylthio, C₁-C₃-Alkyl, C₁-C₃-Halogenalkyl substituiertes Phenyl, Phenoxy oder Phenylthio stehen,
R⁶ und R⁷ unabhängig voneinander für gegebenenfalls durch Fluor, Chlor, Brom substituiertes C₁-C₁₀-Alkyl, C₁-C₁₀-Alkoxy, C₁-C₁₀-Alkoxy-C₁-C₁₀-alkyl,
für gegebenenfalls durch Fluor, Chlor, Brom, C₁-C₂₀-Halogenalkyl, C₁-C₂₀-Alkyl oder C₁-C₄-Alkoxy substituiertes Phenyl,
für gegebenenfalls durch Fluor, Chlor, Brom, C₁-C₄-Alkyl, C₁-C₄-Halogenalkyl oder C₁-C₄-Alkoxy substituiertes Benzyl stehen,
mit der Maßgabe, daß
A und B unabhängig voneinander nicht für C₁-C₄-Alkyl, C₃-C₄-Alkenyl oder C₃-C₄-Alkinyl stehen oder
A und B zusammen nicht für einen gegebenenfalls bis zu dreifach durch C₁-C₄-Alkyl substituierten Rest aus der Reihe 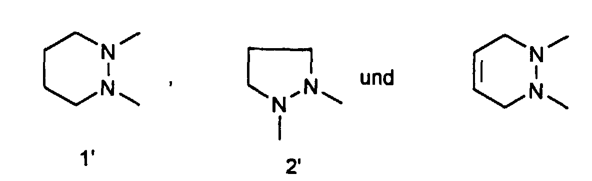 stehen,
wenn G für Wasserstoff steht.

2. Verfahren zur Herstellung von 3-Hydroxy-4-aryl-5-oxo-pyrazolin-Derivaten der Formel (I) gemäß Anspruch 1, dadurch gekennzeichnet,
(A) daß man zum Erhalt von 3-Hydroxy-4-aryl-5-oxo-pyrazolinen der Formel (Ia) 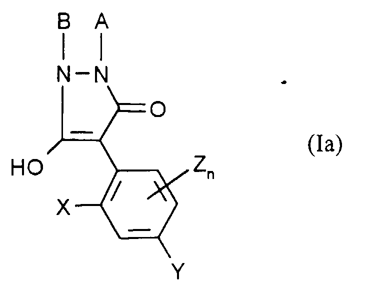 in welcher
A, B, X, Y, Z und n die oben angegebene Bedeutung haben,
(α) Halogencarbonylketene der Formel (II) 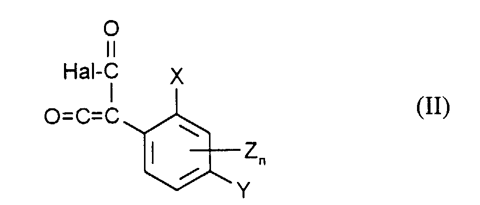 in welcher
X, Y, Z und n die oben angegebene Bedeutung haben,
und
Hal für Halogen, insbesondere Chlor oder Brom steht,
oder
(β) Malonsäurederivate der Formel (III) 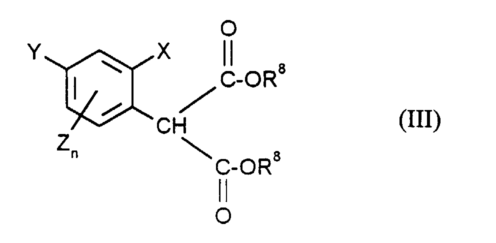 in welcher
X, Y, Z und n die oben angegebene Bedeutung haben,
und
R⁸ für Alkyl steht,
mit Hydrazinen der Formel (IV)
A-NH-NH-B (IV)
in welcher
A und B die oben angegebene Bedeutung haben,
gegebenenfalls in Gegenwart eines Verdünnungsmittels und gegebenenfalls in Gegenwart einer Base umsetzt;
oder
(B) daß man zum Erhalt von Verbindungen der Formel (Ib) 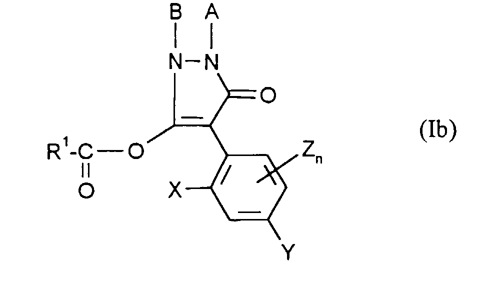 in welcher
A, B, X, Y, Z, R¹ und n die oben angegebene Bedeutung haben,
Verbindungen der Formel (Ia), 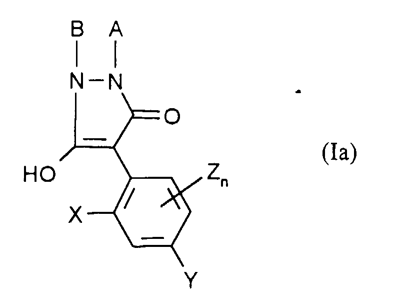 in welcher
A, B, X, Y, Z und n die oben angegebene Bedeutung haben,
(α) mit Säurehalogeniden der allgemeinen Formel (V) 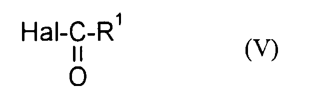 in welcher
R¹ die oben angegebenen Bedeutung hat,
und
Hal für Halogen, insbesondere Chlor und Brom steht,
gegebenenfalls in Gegenwart eines Verdünnungsmittels und gegebenenfalls in Gegenwart eines Säurebindemittels umsetzt,
oder
(β) mit Carbonsäureanhydriden der allgemeinen Formel (VI)
R¹-CO-O-CO-R¹ (VI)
in welcher
R¹ die oben angegebene Bedeutung hat,
gegebenenfalls in Gegenwart eines Verdünnungsmittels und gegebenenfalls in Gegenwart eines Säurebindemittels umsetzt;
oder
(C) daß man zum Erhalt von Verbindungen der Formel (Ic-1) 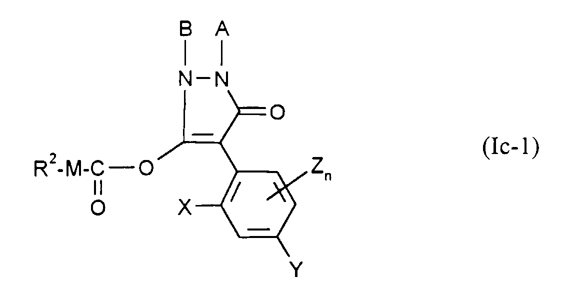 in welcher
A, B, X, Y, Z, R² und n die oben angegebene Bedeutung haben,
und
M für Sauerstoff oder Schwefel steht,
Verbindungen der Formel (Ia) 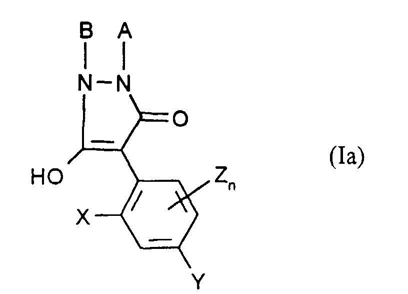 in welcher
A, B, X, Y, Z und n die oben angegebene Bedeutung haben,
mit Chlorameisensäureester oder Chlorameisensäurethiolester der allgemeinen Formel (VII)
R²-M-CO-Cl (VII)
in welcher
R² und M die oben angegebene Bedeutung haben,
gegebenenfalls in Gegenwart eines Verdünnungsmittels und gegebenenfalls in Gegenwart eines Säurebindemittels umsetzt;
oder
(D) daß man zum Erhalt von Verbindungen der Formel (Ic-2) 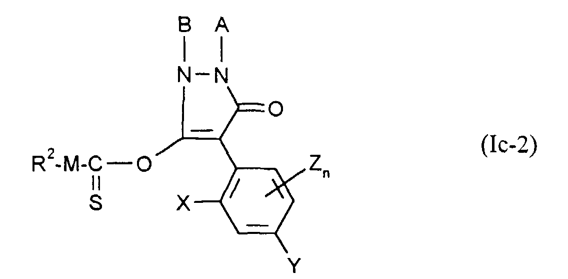 in welcher
A, B, R², X, Y, Z und n die oben angegebene Bedeutung haben,
und
M für Sauerstoff oder Schwefel steht,
Verbindungen der Formel (Ia) 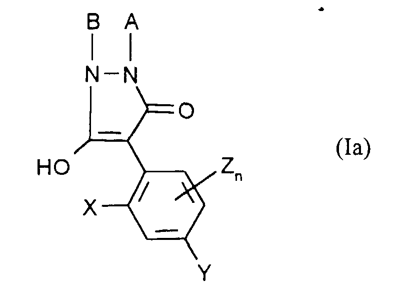 in welcher
A, B, X, Y, Z und n die oben angegebene Bedeutung haben,
(α) mit Chlormonothioameisensäureestern oder Chlordithioameisensäureestern der allgemeinen Formel (VIII) 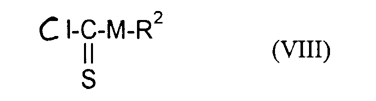 in welcher
M und R² die oben angegebene Bedeutung haben,
gegebenenfalls in Gegenwart eines Verdünnungsmittels und gegebenenfalls in Gegenwart eines Säurebindemittels umsetzt,
oder
(β) mit Schwefelkohlenstoff und anschließend mit Alkylhalogeniden der allgemeinen Formel (IX)
R²-Hal (IX)
in welcher
R² die oben angegebene Bedeutung hat,
und
Hal für Chlor, Brom Iod steht,
umsetzt;
oder
(E) daß man zum Erhalt von Verbindungen der Formel (Id) 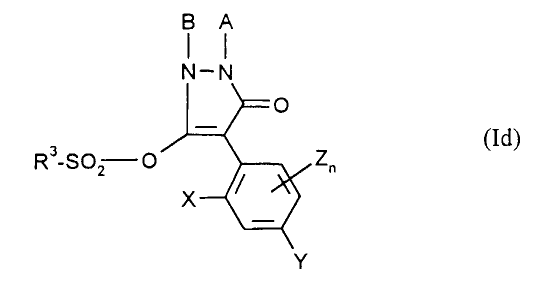 in welcher
A, B, X, Y, Z, R³ und n die oben angegebene Bedeutung haben,
Verbindungen der Formel (Ia) 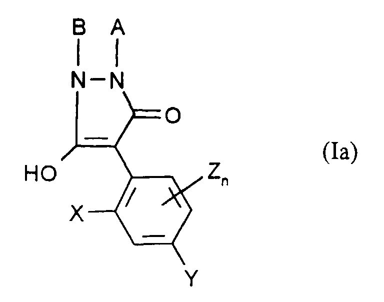 in welcher
A, B, X, Y, Z und n die oben angegebene Bedeutung haben,
mit Sulfonsäurechloriden der allgemeinen Formel (X)
R³-SO₂-Cl (X)
in welcher
R³ die oben angegebene Bedeutung hat,
gegebenenfalls in Gegenwart eines Verdünnungsmittels und gegebenenfalls in Gegenwart eines Säurebindemittels,
umsetzt;
oder
(F) daß man zum Erhalt von Verbindungen der Formel (Ie) 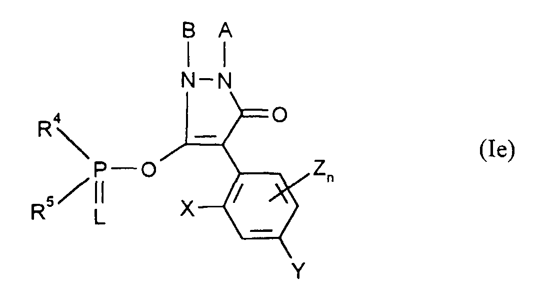 in welcher
A, B, L, X, Y, Z, R⁴, R⁵ und n die oben angegebene Bedeutung haben,
3-Hydroxy-4-aryl-5-oxo-pyrazoline der Formel (Ia) 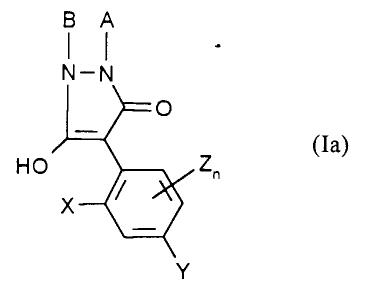 in welcher
A, B, X, Y, Z und n die oben angegebene Bedeutung haben,
mit Phosphorverbindungen der allgemeinen Formel (XI) 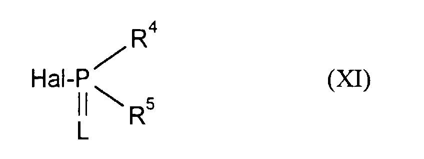 in welcher
L, R⁴ und R⁵ die oben angegebene Bedeutung haben,
und
Hal für Halogen, insbesondere Chlor oder Brom steht,
gegebenenfalls in Gegenwart eines Verdünnungsmittels und gegebenenfalls in Gegenwart eines Säurebindemittels umsetzt;
oder
(G) daß man zum Erhalt von Verbindungen der Formel (If) 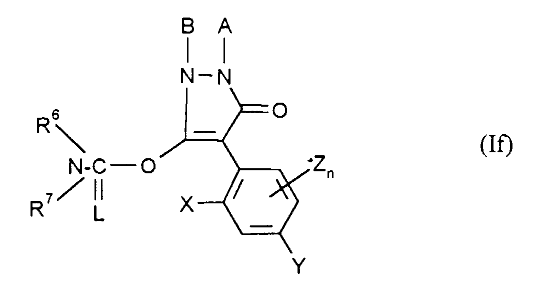 in welcher
A, B, L, X, Y, Z, R⁶, R⁷ und n die oben angegebene Bedeutung haben,
Verbindungen der Formel (Ia) 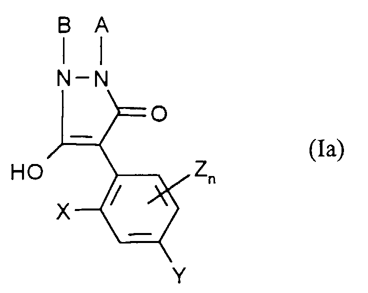 in welcher
A, B, X, Y, Z und n die oben angegebene Bedeutung haben,
(α) mit Isocyanaten der allgemeinen Formel (XII)
R⁶-N=C=O (XII)
in welcher
R⁶ die oben angegebene Bedeutung hat,
gegebenenfalls in Gegenwart eines Verdünnungsmittels und gegebenenfalls in Gegenwart eines Katalysators umsetzt,
oder
(β) mit Carbamidsäurechloriden oder Thiocarbamidsäurechloriden der allgemeinen Formel (XIII) 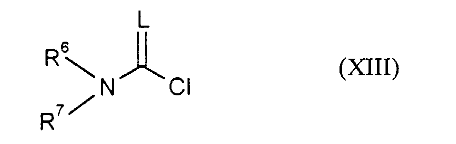 in welcher
L, R⁶ und R⁷ die oben angegebene Bedeutung haben,
gegebenenfalls in Gegenwart eines Verdünnungsmittels und gegebenenfalls in Gegenwart eines Säurebindemittels umsetzt,
oder
(H) daß man zum Erhalt von Verbindungen der Formel (Ig) 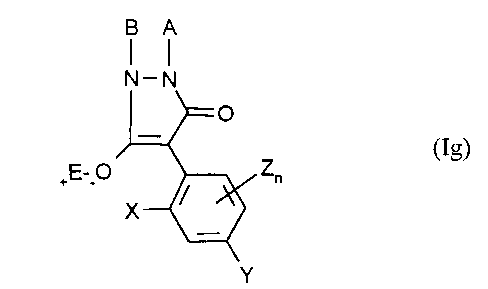 in welcher
A, B, X, Y, Z und n die oben angegebene Bedeutung haben,
und
E für ein Metallionäquivalent oder für ein Ammoniumion steht,
Verbindungen der Formel (Ia) 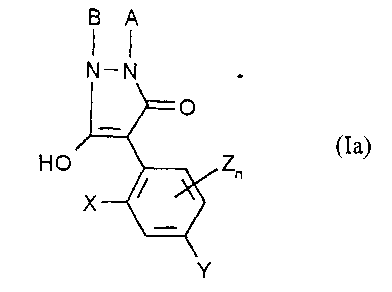 in welcher
A, B, X, Y, Z und n die oben angegebene Bedeutung haben,
mit Metallhydroxiden oder Aminen der allgemeinen Formeln (XIV) und (XV) 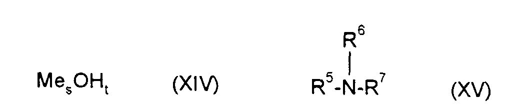 in welcher
Me für ein- oder zweiwertige Metallionen,
s und t für die Zahl 1 und 2 und
R⁵, R⁶ und R⁷ unabhängig voneinander für Wasserstoff und Alkyl
stehen,
gegebenenfalls in Gegenwart eines Verdünnungsmittels umsetzt.

3. Schädlingsbekämpfungsmittel, gekennzeichnet durch einen Gehalt an mindestens einem 3-Hydroxy-4-aryl-5-oxo-pyrazolin-Derivat der Formel (I) gemäß Anspruch 1.

4. Verfahren zur Bekämpfung von tierischen Schädlingen, dadurch gekennzeichnet, daß man substituierte 3-Hydroxy-4-aryl-5-oxo-pyrazolin-Derivate der Formel (I) gemäß Anspruch 1 auf tierische Schädlinge und/oder deren Lebensraum einwirken läßt.

5. Verwendung von substituierten 3-Hydroxy-4-aryl-5-oxo-pyrazolin-Derivaten der Formel (I) gemäß Anspruch 1 zur Bekämpfung von tierischen Schädlingen.

6. Verfahren zur Herstellung von Schädlingsbekämpfungsmitteln, dadurch gekennzeichnet, daß man substituierte 3-Hydroxy-4-aryl-5-oxo-pyrazolin-Derivate der Formel (I) gemäß Anspruch 1 mit Streckmitteln und/oder oberflächenaktiven Mitteln vermischt.

7. Herbizide Mittel, gekennzeichnet durch einen Gehalt an mindestens einem 3-Hydroxy-4-aryl-5-oxo-pyrazolin-Derivate der Formel (I) gemäß Anspruch 1.

8. Verfahren zur Bekämpfung von Unkräutern, dadurch gekennzeichnet, daß man 3-Hydroxy-4-aryl-5-oxo-pyrazolin-Derivate der Formel (I) gemäß Anspruch 1 auf Unkräuter und/oder deren Lebensraum einwirken läßt.

9. Verwendung von 3-Hydroxy-4-aryl-5-oxo-pyrazolin-Derivaten der Formel (I) gemäß Anspruch 1 zur Bekämpfung von Unkräutern.

10. Verfahren zur Herstellung von herbiziden Mitteln, dadurch gekennzeichnet, daß man 3-Hydroxy-4-aryl-5-oxo-pyrazolin-Derivate der Formel (I) gemäß Anspruch 1 mit Streckmitteln und/oder oberflächenaktiven Mitteln vermischt.

11. Halogencarbonylketene der Formel (IIa) 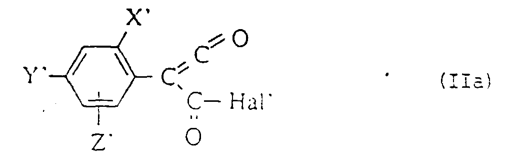 in welcher
Hal' für Halogen steht
X' für C₁-C₄-Alkyl, Halogen oder Trifluormethyl steht
Y' für Wasserstoff, C₁-C₄-Alkyl, Halogen oder Trifluormethyl steht
und
Z' für Wasserstoff, C₁-C₄-Alkyl, Halogen oder Trifluormethyl steht,
mit der Maßgabe, daß Z' nicht für Wasserstoff steht wenn Y' für Wasserstoff steht.

12. Halogencarbonylketene der Formel (IIa) gemäß Ansprüch 11
in welcher
Hal' für Brom oder Chlor steht
X' für Methyl, Fluor, Chlor, Brom oder Trifluormethyl steht.
Y' für Wasserstoff, Methyl, Ethyl, n-Propyl, i-Propyl, n-Butyl, i-Butyl, tert.-Butyl, Fluor, Chlor, Brom oder Trifluormethyl steht
und
Z' für Wasserstoff, Methyl, Fluor, Chlor, Brom oder Trifluormethyl steht,
mit der Maßgabe, daß Z' nicht für Wasserstoff steht, wenn Y' Wasserstoff bedeutet.

13. Halogencarbonylketene der Formel (IIa) gemäß Anspruch 11, in welcher
entweder
X', Y' und Z' jeweils für Methyl stehen oder X' und Y' für Chlor stehen und gleichzeitig Z' Wasserstoff bedeutet.

14. Verfahren zur Herstellung von Halogencarbonylketenen der Formel (IIa) 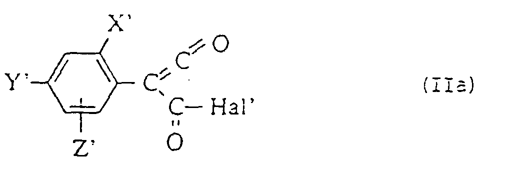 in welcher
Hal' für Halogen steht
X' für C₁-C₄-Alkyl, Halogen oder Trifluomethyl steht
Y' für Wasserstoff, C₁-C₄-Alkyl, Halogen oder Trifluormethyl steht
und
Z' für Wasserstoff, C₁-C₄-Alkyl, Halogen oder Trifluormethyl steht,
mit der Maßgabe, daß Z' nicht für Wasserstoff steht, wenn Y' für Wasserstoff steht,
dadurch gekennzeichnet,
daß man
Arylmalonsäuren der Formel (XVIa) 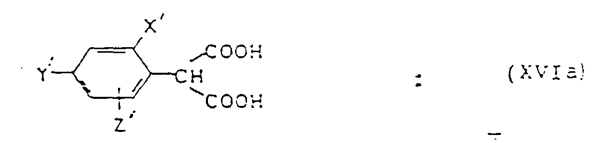 in welcher X', Y' und Z' die oben angegebene Bedeutung haben, mit Säurehalogeniden gegebenenfalls in Gegenwart von Katalysatoren umsetzt.

15. Arylmalonsäuren der Formel (XVIa) 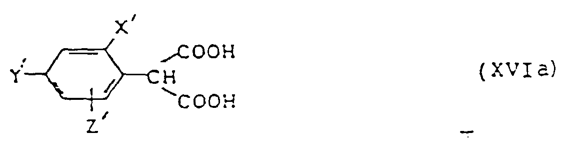 in welcher X', Y' und Z' die im Anspruch 14 angegebene Bedeutung haben.

## Patentansprüche (Patentansprüche für folgende(n) Vertragsstaat(en): ES)

1. Verfahren zur Herstellung von 3-Hydroxy4-aryl-5-oxo-pyrazolin-Derivaten der Formel (I) 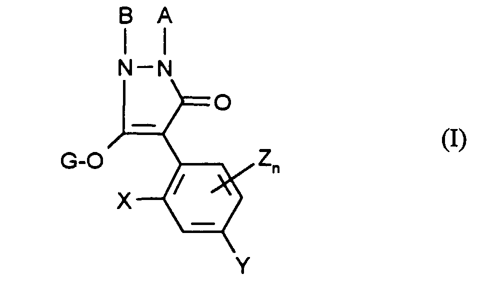 in welcher
A und B gleich oder verschieden sind und unabhängig voneinander jeweils für C₁-C₄-Alkyl, C₂-C₄-Alkenyl, C₂-C₄-Alkinyl, C₁-C₄-Alkoxy-C₁-C₄-alkyl, C₁-C₄-Alkylthio-C₁-C₄-alkyl, Cycloalkyl mit 3 bis 7 Kohlenstoffatomen oder für gegebenenfalls einfach bis dreifach, gleich oder verschieden substituiertes Phenyl stehen,
wobei als Phenylsubstituenten die für R³, R⁴ und R⁵ aufgeführten Phenylsubstituenten in Frage kommen oder
A und B zusammen mit den beiden Stickstoffatomen des Pyrazolinringes für eine gegebenenfalls einfach bis dreifach, gleich oder verschieden substituierte Gruppierung der nachfolgend aufgeführten Formeln 1', 2', 3' oder 4' stehen 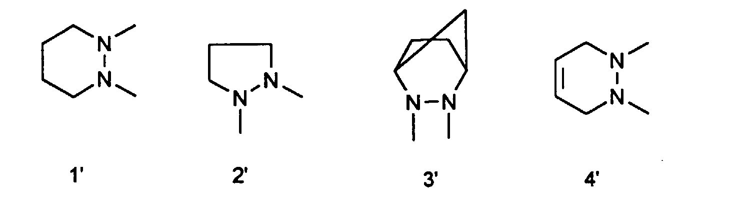 wobei als Substituenten Halogen, gegebenenfalls durch Fluor oder Chlor substituiertes C₁-C₈-Alkyl, C₂-C₈-Alkenyl und C₁-C₄-Alkoxy-C₁-C₄-alkyl in Frage kommen,
X für Methyl, Ethyl, Propyl, i-Propyl, Fluor, Chlor, Brom, Methoxy und Ethoxy steht,
Y für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,
Z für Methyl, Ethyl, i-Propyl, Butyl, i-Butyl, tert.-Butyl, Fluor, Chlor, Brom, Methoxy und Ethoxy steht,
n für eine Zahl 0, 1, 2 oder 3 steht,
G für Wasserstoff (a) oder für die Gruppen 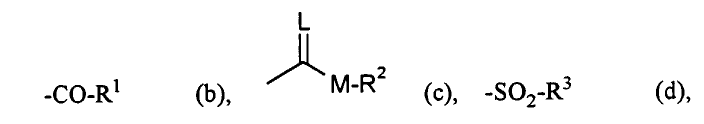 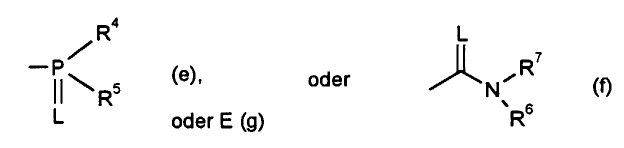 steht,
in welchen
E für ein Metallionäquivalent oder ein Ammoniumion steht,
L und M für Sauerstoff und/oder Schwefel steht,
R¹ für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Alkylthio-C₂-C₆-alkyl, C₁-C₄-Polyalkoxy-C₂-C₄-alkyl oder Cycloalkyl mit 3 bis 6 Ringatomen, das durch 1 bis 2 Sauerstoff- und/oder Schwefelatome unterbrochen sein kann, steht,
für gegebenenfalls durch Fluor, Chlor, Brom, Methyl, Ethyl, Propyl, i-Propyl, Methoxy, Ethoxy, Trifluormethyl, Trifluormethoxy, Nitro substituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy substituiertes Phenyl-C₁-C₃-alkyl steht,
für gegebenenfalls durch Fluor, Chlor, Brom, Methyl, Ethyl substituiertes Pyridyl, Pyrimidyl, Thiazolyl und Pyrazolyl steht,
für gegebenenfalls durch Fluor, Chlor, Methyl, Ethyl substituiertes Phenoxy-C₁-C₄-alkyl steht,
für gegebenenfalls durch Fluor, Chlor, Amino, Methyl, Ethyl substituiertes Pyridyloxy-C₁-C₄-alkyl, Pyridydoxy-C₁-C₄-alkyl und Thiazolyloxy-C₁-C₄-Alkyl steht,
R² für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Polyalkoxy-C₂-C₆-alkyl steht,
oder für gegebenenfalls durch Fluor, Chlor, Nitro, Methyl, Ethyl, Propyl, i-Propyl, Methoxy, Ethoxy, Trifluormethyl substituiertes Phenyl oder Benzyl steht,
R³, R⁴ und R⁵ unabhängig voneinander für gegebenenfalls durch Fluor oder Chlor substituiertes C₁-C₄-Alkyl, C₁-C₄-Alkoxy, C₁-C₄-Alkylamino, Di-(C₁-C₄)-alkylamino, C₁-C₄-Alkylthio, für gegebenenfalls durch Fluor, Chlor, Brom, Nitro, Cyano, C₁-C₂-Alkoxy, C₁-C₄-Fluoralkoxy, C₁-C₂-Chloralkoxy, C₁-C₂-Alkylthio, C₁-C₂-Fluoralkylthio, C₁-C₂-Chloralkylthio, C₁-C₃-Alkyl, C₁-C₃-Halogenalkyl substituiertes Phenyl, Phenoxy oder Phenylthio stehen,
R⁶ und R⁷ unabhängig voneinander für gegebenenfalls durch Fluor, Chlor, Brom substituiertes C₁-C₁₀-Alkyl, C₁-C₁₀-Alkoxy, C₁-C₁₀-Alkoxy-C₁-C₁₀-alkyl,
für gegebenenfalls durch Fluor, Chlor, Brom, C₁-C₂₀-Halogenalkyl,C₁-C₂₀-Alkyl oder C₁-C₄-Alkoxy substituiertes Phenyl,
für gegebenenfalls durch Fluor, Chlor, Brom, C₁-C₄-Alkyl, C₁-C₄-Halogenalkyl oder C₁-C₄-Alkoxy substituiertes Benzyl stehen,
mit der Maßgabe, daß
A und B unabhängig voneinander nicht für C₁-C₄-Alkyl, C₃-C₄-Alkenyl oder C₃-C₄-Alkinyl stehen oder
A und B zusammen nicht für einen gegebenenfalls bis zu dreifach durch C₁-C₄-Alkyl substituierten Rest aus der Reihe 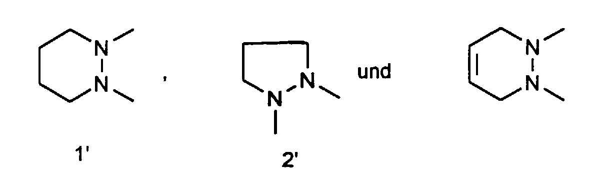 stehen,
wenn G für Wasserstoff steht,
dadurch gekennzeichnet,
(A) daß man zum Erhalt von 3-Hydroxy-4-aryl-5-oxo-pyrazolinen der Formel (Ia) 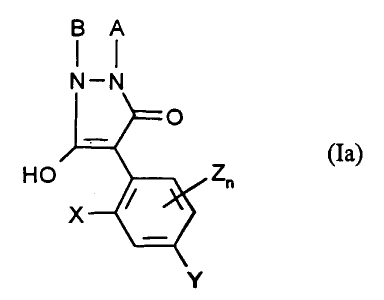 in welcher
A, B, X, Y, Z und n die oben angegebene Bedeutung haben,
(α) Halogencarbonylketene der Formel (II) 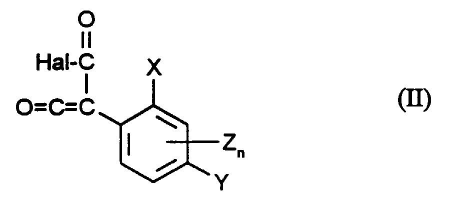 in welcher
X, Y, Z und n die oben angegebene Bedeutung haben,
und
Hal für Halogen, insbesondere Chlor oder Brom steht,
oder
(β) Malonsäurederivate der Formel (III) 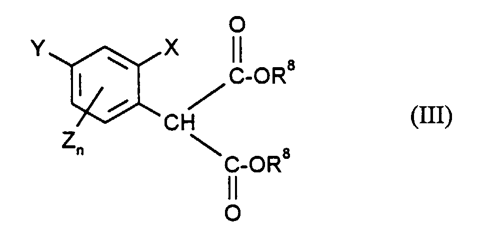 in welcher
X, Y, Z und n die oben angegebene Bedeutung haben,
und
R⁸ für Alkyl steht,
mit Hydrazinen der Formel (IV)
A-NH-NH-B (IV)
in welcher
A und B die oben angegebene Bedeutung haben,
gegebenenfalls in Gegenwart eines Verdünnungsmittels und gegebenenfalls in Gegenwart einer Base umsetzt;
oder
(B) daß man zum Erhalt von Verbindungen der Formel (Ib) 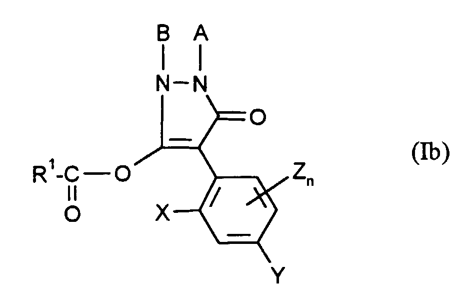 in welcher
A, B, X, Y, Z, R¹ und n die oben angegebene Bedeutung haben,
Verbindungen der Formel (Ia), 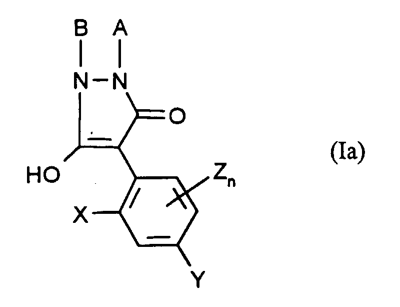 in welcher
A, B, X, Y, Z und n die oben angegebene Bedeutung haben,
(α) mit Säurehalogeniden der allgemeinen Formel (V) 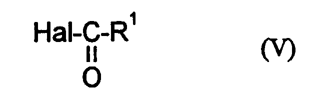 in welcher
R¹ die oben angegebenen Bedeutung hat,
und
Hal für Halogen, insbesondere Chlor und Brom steht,
gegebenenfalls in Gegenwart eines Verdünnungsmittels und gegebenenfalls in Gegenwart eines Säurebindemittels umsetzt,
oder
(β) mit Carbonsäureanhydriden der allgemeinen Formel (VI)
R¹-CO-O-CO-R¹ (VI)
in welcher
R¹ die oben angegebene Bedeutung hat,
gegebenenfalls in Gegenwart eines Verdünnungsmittels und gegebenenfalls in Gegenwart eines Säurebindemittels umsetzt;
oder
(C) daß man zum Erhalt von Verbindungen der Formel (Ic-1) 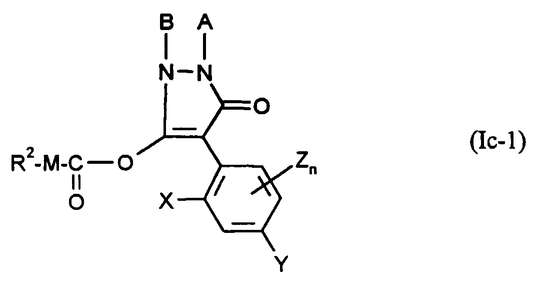 in welcher
A, B, X, Y, Z, R² und n die oben angegebene Bedeutung haben,
und
M für Sauerstoff oder Schwefel steht,
Verbindungen der Formel (Ia) 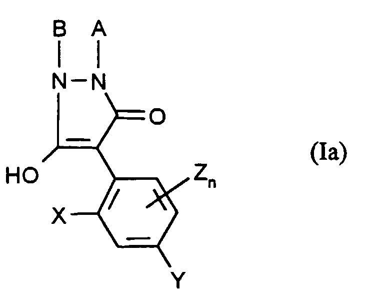 in welcher
A, B, X, Y, Z und n die oben angegebene Bedeutung haben,
mit Chlorameisensäureester oder Chlorameisensäurethiolester der allgemeinen Formel (VII)
R²-M-CO-Cl (VII)
in welcher
R² und M die oben angegebene Bedeutung haben,
gegebenenfalls in Gegenwart eines Verdünnungsmittels und gegebenenfalls in Gegenwart eines Säurebindemittels umsetzt;
oder
(D) daß man zum Erhalt von Verbindungen der Formel (Ic-2) 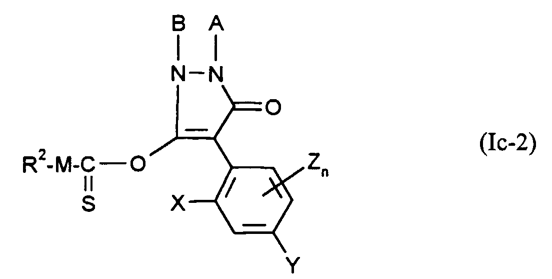 in welcher
A, B, R², X, Y, Z und n die oben angegebene Bedeutung haben,
und
M für Sauerstoff oder Schwefel steht,
Verbindungen der Formel (Ia) 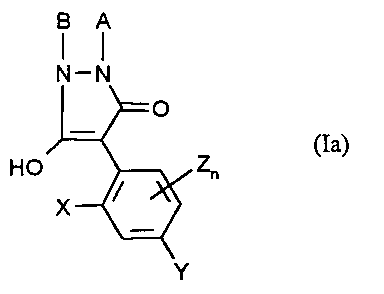 in welcher
A, B, X, Y, Z und n die oben angegebene Bedeutung haben,
(α) mit Chlormonothioameisensäureestern oder Chlordithioameisensäureestern der allgemeinen Formel (VIII) 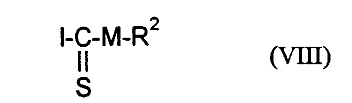 in welcher
M und R² die oben angegebene Bedeutung haben,
gegebenenfalls in Gegenwart eines Verdünnungsmittels und gegebenenfalls in Gegenwart eines Säurebindemittels umsetzt,
oder
(β) mit Schwefelkohlenstoff und anschließend mit Alkylhalogeniden der allgemeinen Formel (IX)
R²-Hal (IX)
in welcher
R² die oben angegebene Bedeutung hat,
und
Hal für Chlor, Brom Iod steht,
umsetzt;
oder
(E) daß man zum Erhalt von Verbindungen der Formel (Id) 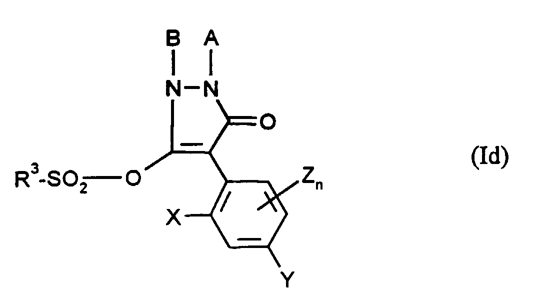 in welcher
A, B, X, Y, Z, R³ und n die oben angegebene Bedeutung haben,
Verbindungen der Formel (Ia) 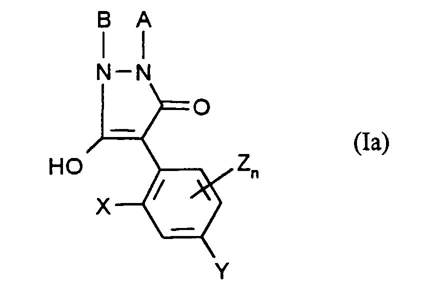 in welcher
A, B, X, Y, Z und n die oben angegebene Bedeutung haben,
mit Sulfonsäurechloriden der allgemeinen Formel (X)
R³-SO₂-Cl (X)
in welcher
R³ die oben angegebene Bedeutung hat,
gegebenenfalls in Gegenwart eines Verdünnungsmittels und gegebenenfalls in Gegenwart eines Säurebindemittels,
umsetzt;
oder
(F) daß man zum Erhalt von Verbindungen der Formel (Ie) 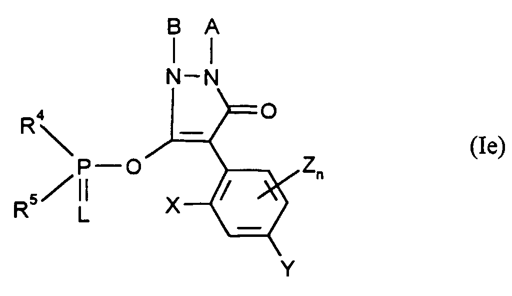 in welcher
A, B, L, X, Y, Z, R⁴, R⁵ und n die oben angegebene Bedeutung haben,
3-Hydroxy-4-aryl-5-oxo-pyrazoline der Formel (Ia) 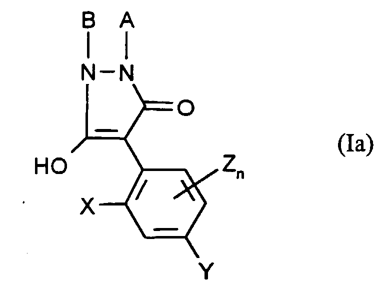 in welcher
A, B, X, Y, Z und n die oben angegebene Bedeutung haben,
mit Phosphorverbindungen der allgemeinen Formel (XI) 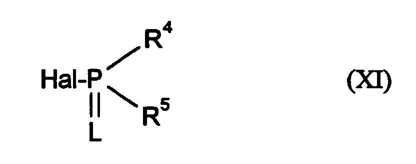 in welcher
L, R⁴ und R⁵ die oben angegebene Bedeutung haben,
und
Hal für Halogen, insbesondere Chlor oder Brom steht,
gegebenenfalls in Gegenwart eines Verdünnungsmittels und gegebenenfalls in Gegenwart eines Säurebindemittels umsetzt;
oder
(G) daß man zum Erhalt von Verbindungen der Formel (If) 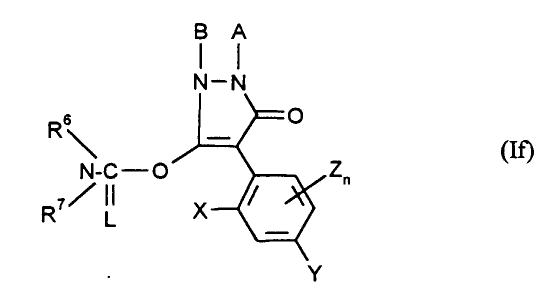 in welcher
A, B, L, X, Y, Z, R⁶, R⁷ und n die oben angegebene Bedeutung haben,
Verbindungen der Formel (Ia) 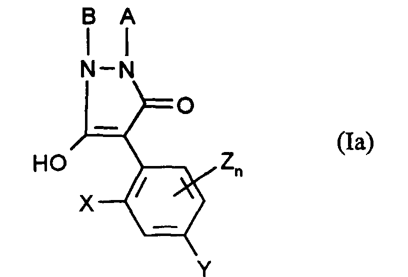 in welcher
A, B, X, Y, Z und n die oben angegebene Bedeutung haben,
(α) mit Isocyanaten der allgemeinen Formel (XII)
R⁶-N=C=O (XII)
in welcher
R⁶ die oben angegebene Bedeutung hat,
gegebenenfalls in Gegenwart eines Verdünnungsmittels und gegebenenfalls in Gegenwart eines Katalysators umsetzt,
oder
(β) mit Carbamidsäurechloriden oder Thiocarbamidsäurechloriden der allgemeinen Formel (XIII) 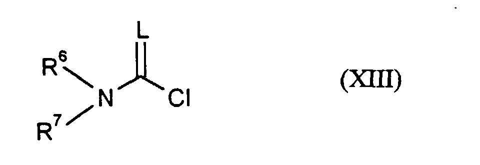 in welcher
L, R⁶ und R⁷ die oben angegebene Bedeutung haben,
gegebenenfalls in Gegenwart eines Verdünnungsmittels und gegebenenfalls in Gegenwart eines Säurebindemittels umsetzt,
oder
(H) daß man zum Erhalt von Verbindungen der Formel (Ig) 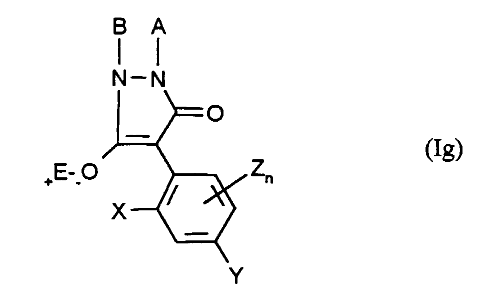 in welcher
A, B, X, Y, Z und n die oben angegebene Bedeutung haben,
und
E für ein Metallionäquivalent oder für ein Ammoniumion steht,
Verbindungen der Formel (Ia) 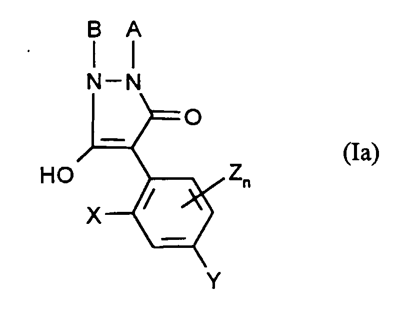 in welcher
A, B, X, Y, Z und n die oben angegebene Bedeutung haben,
mit Metallhydroxiden oder Aminen der allgemeinen Formeln (XIV) und (XV) 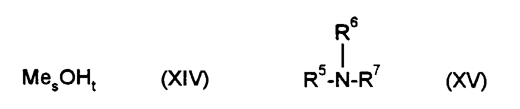 in welcher
Me für ein- oder zweiwertige Metallionen,
s und t für die Zahl 1 und 2 und
R⁵, R⁶ und R⁷ unabhängig voneinander für Wasserstoff und Alkyl
stehen,
gegebenenfalls in Gegenwart eines Verdünnungsmittels umsetzt.

2. Schädlingsbekämpfungsmittel, gekennzeichnet durch einen Gehalt an mindestens einem 3-Hydroxy-4-aryl-5-oxo-pyrazolin-Derivat der Formel (I).

3. Verfahren zur Bekämpfung von tierischen Schädlingen, dadurch gekennzeichnet, daß man substituierte 3-Hydroxy-4-aryl-5-oxo-pyrazolin-Derivate der Formel (I) auf tierische Schädlinge und/oder deren Lebensraum einwirken läßt.

4. Verwendung von substituierten 3-Hydroxy-4-aryl-5-oxo-pyrazolin-Derivaten der Formel (I) zur Bekämpfung von tierischen Schädlingen.

5. Verfahren zur Herstellung von Schädlingsbekämpfungsmitteln, dadurch gekennzeichnet, daß man substituierte 3-Hydroxy-4-aryl-5-oxo-pyrazolin-Derivate der Formel (I) gemäß Anspruch 1 mit Streckmitteln und/oder oberflächenaktiven Mitteln vermischt.

6. Herbizide Mittel, gekennzeichnet durch einen Gehalt an mindestens einem 3-Hydroxy-4-aryl-5-oxo-pyrazolin-Derivate der Formel (I) gemäß Anspruch 1.

7. Verfahren zur Bekämpfung von Unkräutern, dadurch gekennzeichnet, daß man 3-Hydroxy-4-aryl-5-oxo-pyrazolin-Derivate der Formel (I) gemäß Anspruch 1 auf Unkräuter und/oder deren Lebensraum einwirken läßt.

8. Verwendung von 3-Hydroxy-4-aryl-5-oxo-pyrazolin-Derivaten der Formel (I) gemäß Anspruch 1 zur Bekämpfung von Unkräutern.

9. Verfahren zur Herstellung von herbiziden Mitteln, dadurch gekennzeichnet, daß man 3-Hydroxy-4-aryl-5-oxo-pyrazolin-Derivate der Formel (I) gemäß Anspruch 1 mit Streckmitteln und/oder oberflächenaktiven Mitteln vermischt.

10. Verfahren zur Herstellung von Halogencarbonylketenen der Formel (IIa) 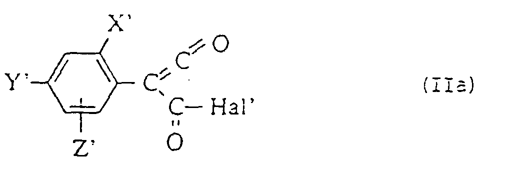 in welcher
Hal' für Halogen steht
X' für C₁-C₄-Alkyl, Halogen oder Trifluormethyl steht
Y' für Wasserstoff C₁-C₄-Alkyl, Halogen oder Trifluormethyl steht
und
Z' für Wasserstoff, C₁-C₄-Alkyl, Halogen oder Trifluormethyl steht,
mit der Maßgabe, daß Z' nicht für Wasserstoff steht, wenn Y' für Wasserstoff steht,
dadurch gekennzeichnet
daß man
Arylmalonsäuren der Formel (XVIa) 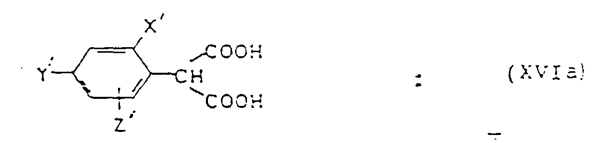 in welcher X', Y' und Z' die oben angegebene Bedeutung haben, mit Säurehalogeniden gegebenenfalls in Gegenwart von Katalysatoren umsetzt.

## Claims (Claims for the following Contracting State(s): BE, CH, DE, FR, GB, GR, IT, LI, NL)

1. 3-hydroxy-4-aryl-5-oxo-pyrazoline derivatives of the formula (I) 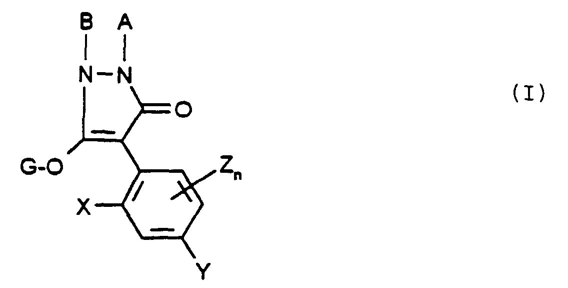 in which
A and B are identical or different and independently of one another in each case represent, C₁-C₄-alkyl, C₂-C₄-alkenyl, C₂-C₄-alkynyl, C₁-C₄-alkoxy-C₁-C₄-alkyl, C₁-C₄-alkylthio-C₁-C₄-alkyl or cyclo-alkyl having 3 to 7 carbon atoms, or represent phenyl which is optionally monosubstituted to trisubstituted by identical or different substituents, suitable phenyl substituents being the phenyl substituents mentioned in the case of R³, R⁴ and R⁵, or
A and B together with the two nitrogen atoms of the pyrazoline ring represent a group of the formulae 1', 2', 3', or 4' listed below 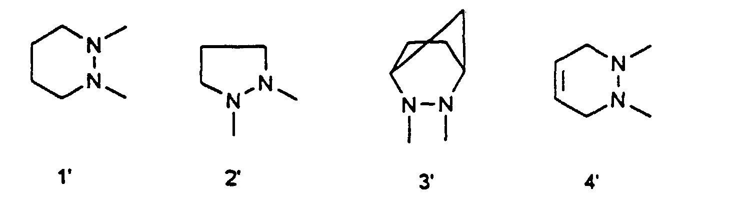
which is optionally monosubstituted to trisubstituted by identical or different substituents, suitable substituents being halogen, or C₁-C₈-alkyl, C₂-C₈-alkenyl and C₁-C₄-alkoxy-C₁-C₄-alkyl, which are optionally substituted by fluorine or chlorine,
X represents methyl, ethyl, propyl, i-propyl, fluorine, chlorine, bromine, methoxy and ethoxy
Y represents hydrogen, methyl, ethyl, propyl, i-propyl, butyl, i-butyl, tert-butyl, fluorine, chlorine, bromine, methoxy, ethoxy and trifluoromethyl,
Z represents methyl, ethyl, i-propyl, butyl, i-butyl, tert-butyl, fluorine, chlorine, bromine, methoxy and ethoxy,
n represents a number 0, 1, 2 or 3,
G represents hydrogen (a) or the groups 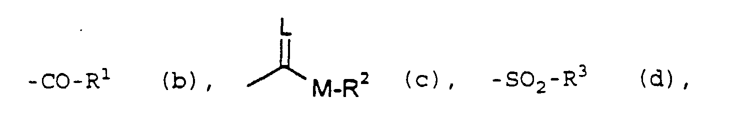 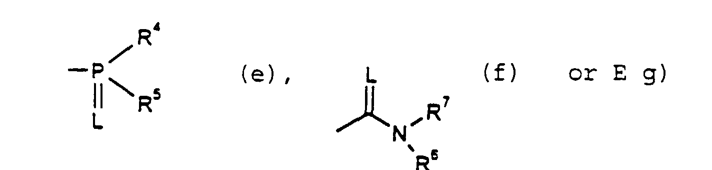
in which
E represents a metal ion equivalent or an ammonium ion,
L and M represent oxygen and/or sulphur,
R¹ represents optionally fluorine- or chlorine-substituted C₁-C₁₄-alkyl, C₂-C₁₄-alkenyl, C₁-C₄-alkoxy-C₂-C₆-alkyl, C₁-C₄-alkylthio-C₂-C₆-alkyl, C₁-C₄-polyalkoxy-C₂-C₄-alkyl or cycloalkyl which has 3 to 6 ring atoms and which can be interrupted by 1 to 2 oxygen and/or sulphur atoms, or represents phenyl which is optionally substituted by fluorine, chlorine, bromine, methyl, ethyl, propyl, i-propyl, methoxy, ethoxy, trifluoromethyl, trifluoromethoxy or nitro,
or represents phenyl-C₁-C₃-alkyl which is optionally substituted by fluorine, chlorine, bromine, methyl, ethyl, propyl i-propyl, methoxy, ethoxy, trifluoromethyl or trifluoromethoxy,
or represents pyridyl, pyrimidyl, thiazolyl and pyrazolyl which are optionally substituted by fluorine, chlorine, bromine, methyl or ethyl,
or represents phenoxy-C₁-C₄-alkyl which is optionally substituted by fluorine, chlorine, methyl or ethyl,
or represents pyridyloxy-C₁-C₄-alkyl, pyrimidyloxy-C₁-C₄-alkyl and thiazolyloxy-C₁-C₄-alkyl which are optionally substituted by fluorine, chlorine, amino, methyl or ethyl,
R² represents C₁-C₁₄-alkyl, C₂-C₁₄-alkenyl, C₁-C₄-alkoxy-C₂-C₆-alkyl or C₁-C₄-polyalkoxy-C₂-C₆-alkyl, which are optionally substituted by fluorine or chlorine,
or represents phenyl or benzyl which are optionally substituted by fluorine, chlorine, nitro, methyl, ethyl, propyl, i-propyl, methoxy, ethoxy, or trifluoromethyl,
R³, R⁴ and R⁵ independently of one another represent C₁-C₄-alkyl, C₁-C₄-alkoxy, C₁-C₄-alkylamino, di-(C₁-C₄)-alkylamino or C₁-C₄-alkylthio, which are optionally substituted by fluorine or chlorine, or represent phenyl, phenoxy or phenylthio which are optionally substituted by fluorine, chlorine, bromine, nitro, cyano, C₁-C₂-alkoxy, C₁-C₄-fluoroalkoxy, C₁-C₂-chloroalkoxy, C₁-C₂-alkylthio, C₁-C₂-fluoroalkylthio, C₁-C₂-chloroalkylthio, C₁-C₃-alkyl or C₁-C₃-halogenoalkyl,
R⁶ and R⁷ independently of one another represent C₁-C₁₀-alkyl, C₁-C₁₀-alkoxy or C₁-C₁₀-alkoxy-C₁-C₁₀-alkyl, which are optionally substituted by fluorine, chlorine or bromine, or represent phenyl which is optionally substituted by fluorine, chlorine, bromine, Cₗ-C₂₀-halogenoalkyl, C₁-C₂₀-alkyl or C₁-C₄-alkoxy,
or represent benzyl which is optionally substituted by fluorine, chlorine, bromine, C₁-C₄-alkyl, C₁-C₄-halogenoalkyl or C₁-C₄-alkoxy with the proviso that
A and B independently of one another do not represent C₁-C₄-alkyl,
C₃-C₄-alkenyl or C₃-C₄-alkinyl or
A and B together do not represent a radical from the series consisting of 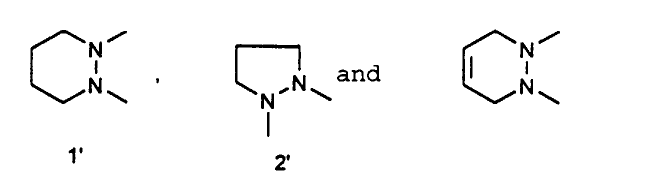 which is optionally up to trisubstituted by C₁-C₄-alkyl,
if G represents hydrogen.

2. Process for the preparation of 3-hydroxy-4-aryl-5-oxo-pyrazoline derivatives of the formula (I) according to Claim 1, characterised in that
(A) to obtain 3-hydroxy-4-aryl-5-oxo-pyrazolines of the formula (Ia) 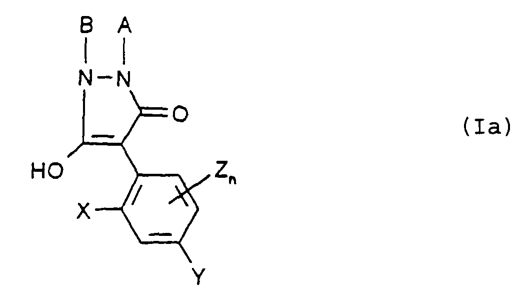 in which
A, B, X, Y, Z and n have the abovementioned meaning,
(α) halogenocarbonyl ketenes of the formula (II) 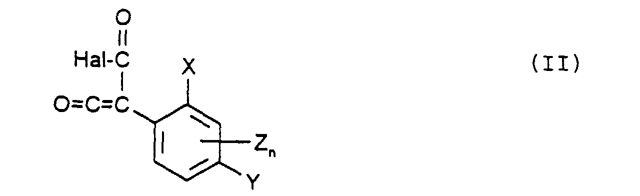 in which
X, Y, Z and n have the abovementioned meaning
and
Hal represents halogen, in particular chlorine or bromine,
or
(β) malonic acid derivatives of the formula (III) 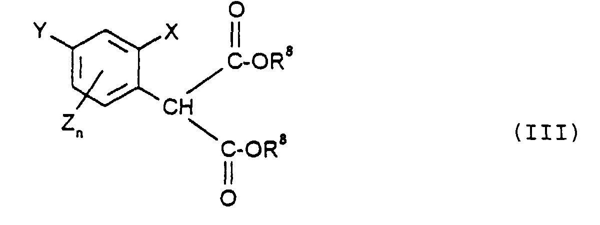 in which
X, Y, Z and n have the abovementioned meaning and
R⁸ represents alkyl,
are reacted with hydrazines of the formula (IV)
A-NH-NH-B (IV)
in which
A and B have the abovementioned meaning,
if appropriate in the presence of a diluent and
if appropriate in the presence of a base;
or
(B) to obtain compounds of the formula (Ib) 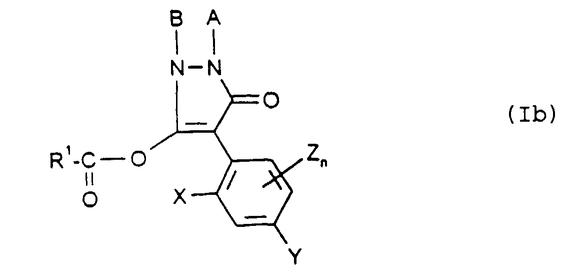 in which
A, B, X, Y, Z, R¹ and n have the abovementioned meaning,
compounds of the formula (Ia) 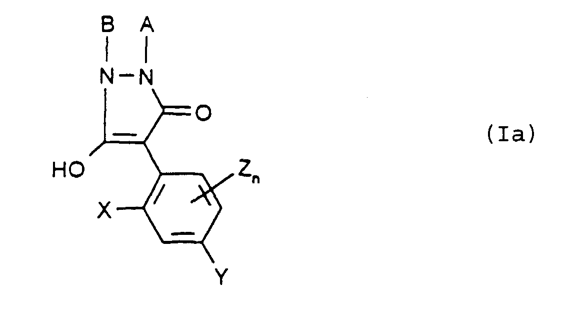 in which
A, B, X, Y, Z and n have the abovementioned meaning,
(α) are reacted with acid halides of the general formula (V) 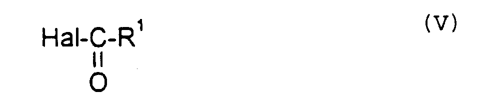 in which
R¹ has the abovementioned meaning
and
Hal represents halogen, in particular chlorine and bromine,
if appropriate in the presence of a diluent and if appropriate in the presence of an acid-binding agent,
or
(β) are reacted with carboxylic anhydrides of the general formula (VI)
R¹-CO-O-CO-R¹ (VI)
in which
R¹ has the abovementioned meaning,
if appropriate in the presence of a diluent and if appropriate in the presence of an acid-binding agent;
or
(C) to obtain compounds of the formula (Ic-1) 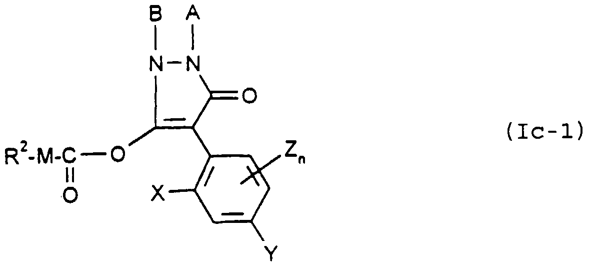 in which
A, B, X, Y, Z, R² and n have the abovementioned meaning,
and
M represents oxygen or sulphur,
compounds of the formula (Ia) 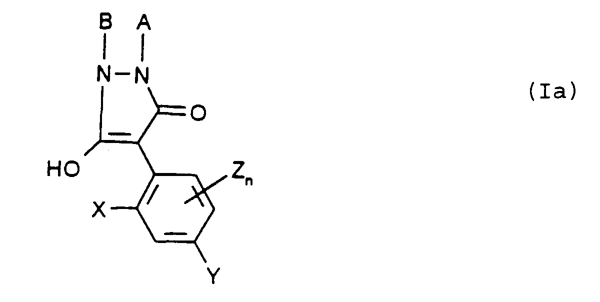 in which
A, B, X, Y, Z and n have the abovementioned meaning,
are reacted with chloroformic ester or chloroformic thioester of the general formula (VII)
R²-M-CO-Cl (VII)
in which
R² and M have the abovementioned meaning,
if appropriate in the presence of a diluent and if appropriate in the presence of an acid-binding agent;
or
(D) to obtain compounds of the formula (Ic-2) 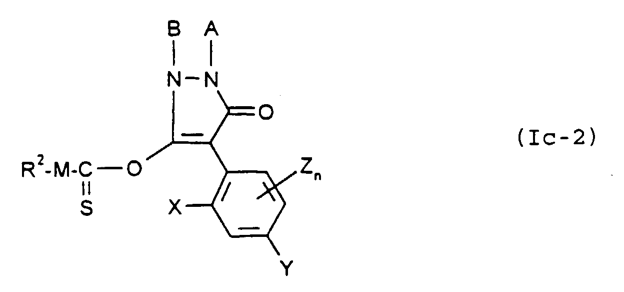 in which
A, B, R², X, Y, Z and n have the abovementioned meaning,
and
M represents oxygen or sulphur,
compounds of the formula (Ia) 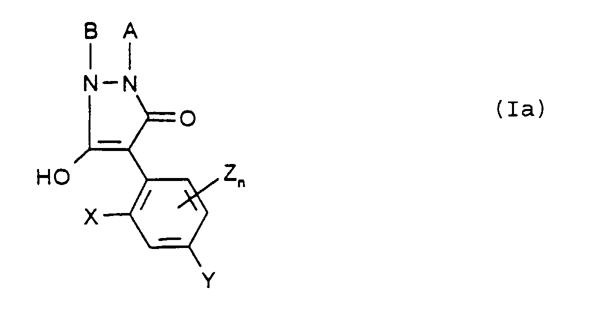 in which
A, B, X, Y, Z and n have the abovementioned meaning,
(α) are reacted with chloromonothioformic esters or chlorodithioformic esters of the general formula (VIII) 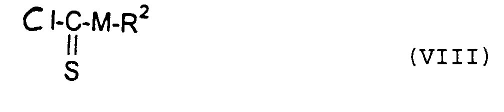 in which
M and R² have the abovementioned meaning,
if appropriate in the presence of a diluent and if appropriate in the presence of an acid-binding agent,
or
(β) are reacted with carbon disulphide and subsequently with alkyl halides of the general formula (IX)
R²-Hal (IX)
in which
R² has the abovementioned meaning
and
Hal represents chlorine, bromine or iodine;
or
(E) to obtain compounds of the formula (Id) 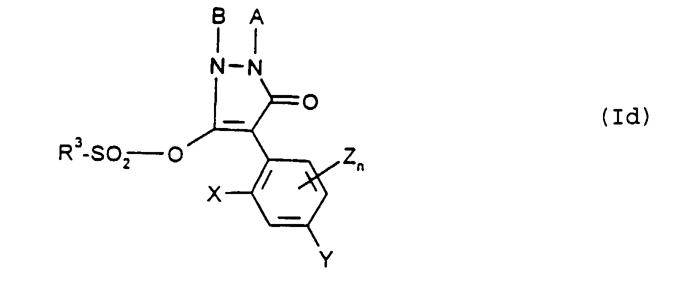 in which
A, B, X, Y, Z, R³ and n have the abovementioned meaning,
compounds of the formula (Ia) 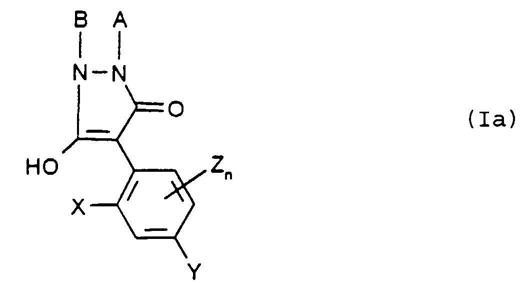 in which
A, B, X, Y, Z and n have the abovementioned meaning,
are reacted with sulphonyl chlorides of the general formula (X)
R³-SO₂-Cl (X)
in which
R³ has the abovementioned meaning,
if appropriate in the presence of a diluent and if appropriate in the presence of an acid-binding agent;
or
(F) to obtain compounds of the formula (Ie) 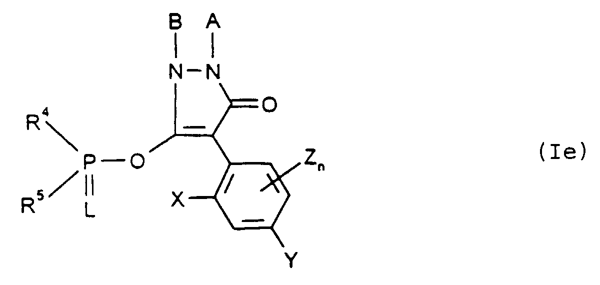 in which
A, B, L, X, Y, Z, R⁴, R⁵ and n have the above mentioned meaning,
3-hydroxy-4-aryl-5-oxo-pyrazolines of the formula (Ia) 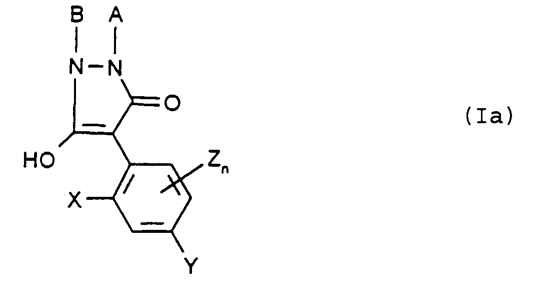 in which
A, B, X, Y, Z and n have the abovementioned meaning,
are reacted with phosphorus compounds of the general formula (XI) 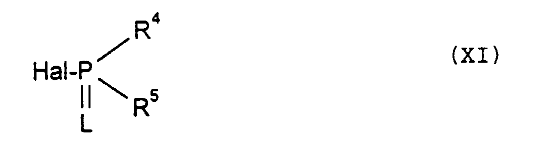 in which
L, R⁴ and R⁵ have the abovementioned meaning
and
Hal represents halogen, in particular chlorine and bromine,
if appropriate in the presence of a diluent and if appropriate in the presence of an acid-binding agent;
or
(G) to obtain compounds of the formula (If) 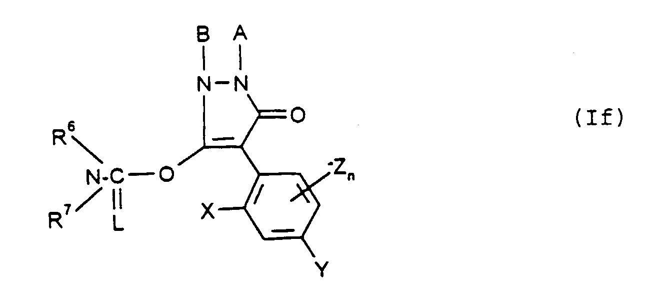 in which
A, B, L, X, Y, Z, R⁶, R⁷ and n have the above mentioned meaning,
compounds of the formula (Ia) 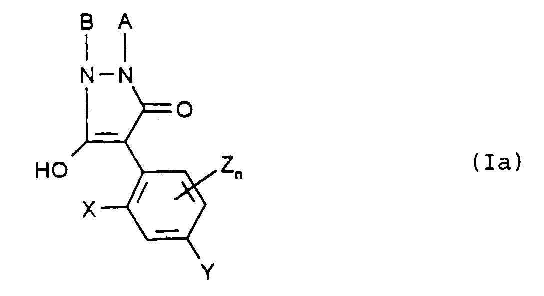 in which
A, B, X, Y, Z and n have the abovementioned meaning,
(α) are reacted with isocyanates of the general formula (XII)
R⁶-N=C=O (XII)
in which
R⁶ has the abovementioned meaning,
if appropriate in the presence of a diluent and if appropriate in the presence of a catalyst,
or
(β) are reacted with carbamoyl chlorides or thiocarbamoyl chlorides of the general formula (XIII) 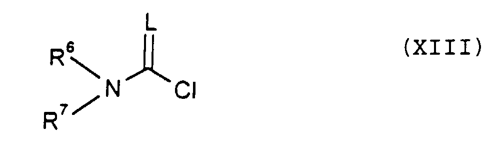 in which
L, R⁶ and R⁷ have the abovementioned meaning,
if appropriate in the presence of a diluent and if appropriate in the presence of an acid-binding agent,
or
(H) to obtain compounds of the formula (Ig) 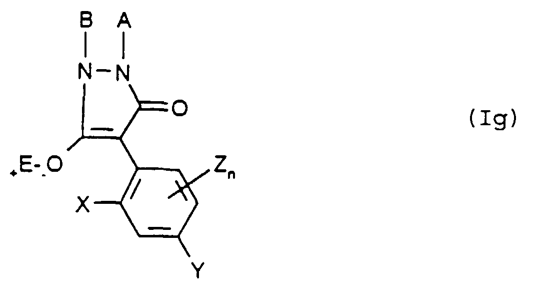 in which
A, B, X, Y, Z and n have the abovementioned meaning,
and
E represents a metal ion equivalent or an ammonium ion,
compounds of the formula (Ia) 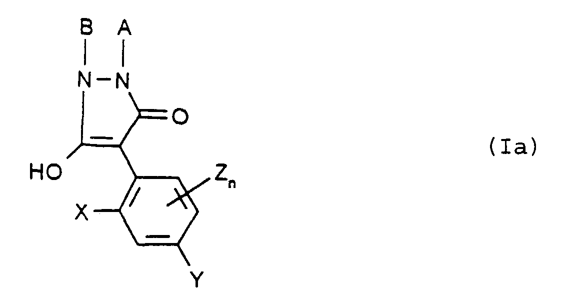 in which
A, B, X, Y, Z and n have the abovementioned meaning,
are reacted with metal hydroxides or amines of the general formulae (XIV) and (XV) 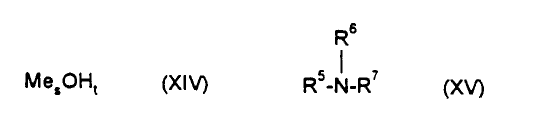 in which
Me represents monovalent or divalent metal ions,
s and t represent the number 1 and 2 and
R⁵, R⁶ and R⁷ independently of one another represent hydrogen and alkyl,
if appropriate in the presence of a diluent.

3. Pesticides, characterised in that they contain at least one 3-hydroxy-4-aryl-5-oxo-pyrazoline derivative of the formula (I) according to Claim 1.

4. Method of combating animal pests, characterised in that substituted 3-hydroxy-4-aryl-5-oxo-pyrazoline derivatives of the formula (I) according to Claim 1 are allowed to act on animal pests and/or their environment.

5. Use of substituted 3-hydroxy-4-aryl-5-oxo-pyrazoline derivatives of the formula (I) according to Claim 1 for combating animal pests.

6. Process for the preparation of pesticides, characterised in that substituted 3-hydroxy-4-aryl-5-oxo-pyrazoline derivatives of the formula (I) according to Claim 1 are mixed with extenders and/or surface-active agents.

7. Herbicidal agents, characterised in that they contain at least one 3-hydroxy-4-aryl-5-oxo-pyrazoline derivative of the formula (I) according to Claim 1.

8. Method of combating weeds, characterised in that 3-hydroxy-4-aryl-5-oxo-pyrazoline derivatives of the formula (I) according to Claim 1 are allowed to act on weeds and/or their environment.

9. Use of 3-hydroxy-4-aryl-5-oxo-pyrazoline derivatives of the formula (I) according to Claim 1 for combating weeds.

10. Process for the preparation of herbicidal agents, characterised in that 3-hydroxy-4-aryl-5-oxo-tpyrazoline derivatives of the formula (I) according to Claim 1 are mixed with extenders and/or surface-active agents.

11. Halogenocarbonyl ketenes of the formula (IIa) 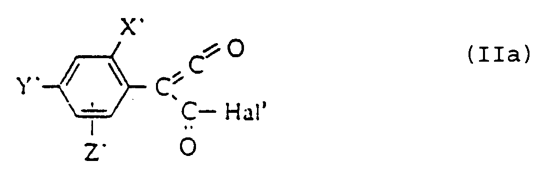 in which
Hal' represents halogen,
X' represents C₁-C₄-alkyl, halogen or trifluoromethyl,
Y' represents hydrogen, C₁-C₄-alkyl, halogen or trifluoromethyl and
Z' represents hydrogen, C₁-C₄-alkyl, halogen or trifluoromethyl,
with the proviso that Z' does not represent hydrogen when Y' represents hydrogen.

12. Halogenocarbonyl ketenes of the formula (IIa) according to Claim 11 in which
Hal' represents bromine or chlorine,
X' represents methyl, fluorine, chlorine, bromine or trifluoromethyl,
Y' represents hydrogen, methyl, ethyl, n-propyl, i-propyl, n-butyl, i-butyl, tert.-butyl, fluorine, chlorine, bromine or trifluoromethyl and
Z' represents hydrogen, methyl, fluorine, chlorine, bromine or trifluoromethyl,
with the proviso that Z' does not represent hydrogen when Y' represents hydrogen.

13. Halogenocarbonyl ketenes of the formula (IIa) according to Claim 11,
in which
either
X', Y' and Z' each represent methyl or X' and Y' represent chlorine and simultaneously Z' denotes hydrogen.

14. Process for the preparation of halogenocarbonyl ketenes of the formula (IIa) 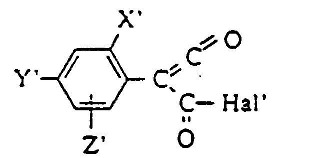 in which
Hal' represents halogen,
X' represents C₁-C₄-alkyl, halogen or trifluoromethyl,
Y' represents hydrogen, C₁-C₄-alkyl, halogen or trifluoromethyl and
Z' represents hydrogen, C₁-C₄-alkyl, halogen or trifluoromethyl,
with the proviso that Z' does not represent hydrogen when Y' represents hydrogen,
characterized in that
arylmalonic acids of the formula (XVIa) 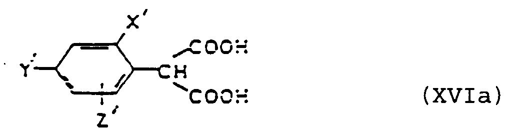 in which X', Y' and Z' of the abovementioned meaning are reacted with acid halides, if appropriate in the presence of catalysts.

15. Arylmalonic acids of the formula (XVIa) 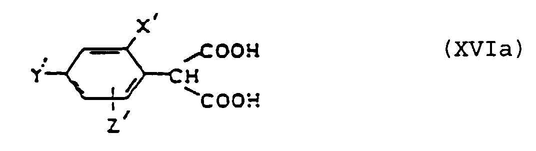 in which
X', Y' and Z' have the meaning given in Claim 14.

## Claims (Claims for the following Contracting State(s): ES)

1. Process for the preparation of 3-hydroxy-4-aryl-5-oxo-pyrazoline derivatives of the formula (I) 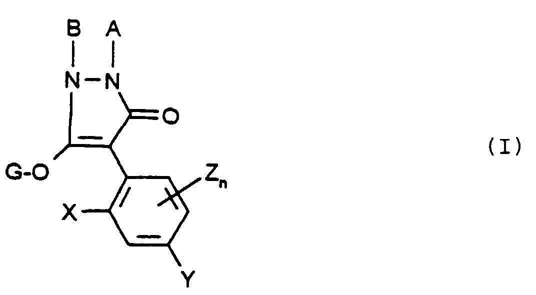 in which
A and B are identical or different and independently of one another in each case represent C₁-C₄-alkyl, C₂-C₄-alkenyl, C₂-C₄-alkynyl, C₁-C₄-alkoxy-C₁-C₄-alkyl, C₁-C₄-alkylthio-C₁-C₄-alkyl or cycloalkyl having 3 to 7 carbon atoms, or represent phenyl which is optionally monosubstituted to trisubstituted by identical or different substituents,
suitable phenyl substituents being the phenyl substituents mentioned in the case of R³, R⁴ and R⁵, or
A and B together with the two nitrogen atoms of the pyrazoline ring represent a group of the formulae 1', 2', 3' or 4' listed below 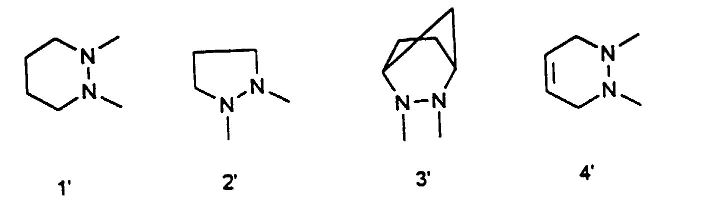 which is optionally monosubstituted to trisubstituted by identical or different substituents, suitable substituents being halogen, or C₁-C₈-alkyl, C₂-C₈-alkenyl and C₁-C₄-alkoxy-C₁-C₄-alkyl, which are optionally substituted by fluorine or chlorine,
X represents methyl, ethyl, propyl, i-propyl, fluorine, chlorine, bromine, methoxy and ethoxy
Y represents hydrogen, methyl, ethyl, propyl, i-propyl, butyl, i-butyl, tert-butyl, fluorine, chlorine, bromine, methoxy, ethoxy and trifluoromethyl,
Z represents methyl, ethyl, i-propyl, butyl, i-butyl, tert-butyl, fluorine, chlorine, bromine, methoxy and ethoxy,
n represents a number 0, 1, 2 or 3,
G represents hydrogen (a) or the groups 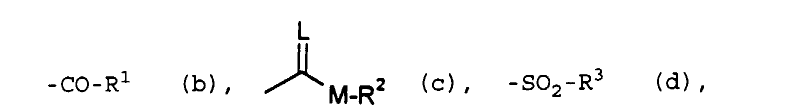 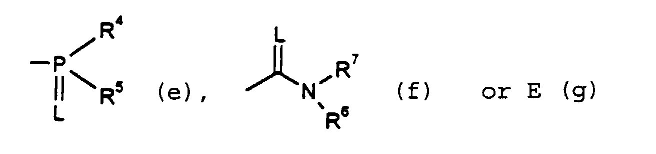 in which
E represent a metal ion equivalent or an ammonium ion,
L and M represent oxygen and/or sulphur,
R¹ represents optionally fluorine- or chlorine-substituted C₁-C₁₄-alkyl, C₂-C₁₄-alkenyl, C₁-C₄-alkoxy-C₂-C₆-alkyl, C₁-C₄-alkylthio-C₂-C₆-alkyl, C₁-C₄-polyalkoxy-C₂-C₄-alkyl or cycloalkyl which has 3 to 6 ring atoms and which can be interrupted by 1 to 2 oxygen and/or sulphur atoms,
or represents phenyl which is optionally substituted by fluorine, chlorine, bromine, methyl, ethyl, propyl, i-propyl, methoxy, ethoxy, trifluoromethyl, trifluoromethoxy or nitro,
or represents phenyl-C₁-C₃-alkyl which is optionally substituted by fluorine, chlorine, bromine, methyl, ethyl, propyl i-propyl, methoxy, ethoxy, trifluoromethyl or trifluoromethoxy,
or represents pyridyl, pyrimidyl, thiazolyl and pyrazolyl which are optionally substituted by fluorine, chlorine, bromine, methyl or ethyl, or represents phenoxy-C₁-C₄-alkyl which is optionally substituted by fluorine, chlorine, methyl or ethyl,
or represents pyridyloxy-C₁-C₄-alkyl, pyrimidyloxy-C₁-C₄-alkyl and thiazolyloxy-C₁-C₄-alkyl which are optionally substituted by fluorine, chlorine, amino, methyl- or ethyl,
R² represents C₁-C₁₄-alkyl, C₂-C₁₄-alkenyl, C₁-C₄-alkoxy-C₂-C₆-alkyl or C₁-C₄-polyalkoxy-C₂-C₆-alkyl, which are optionally substituted by fluorine or chlorine,
or represents phenyl or benzyl which are optionally substituted by fluorine, chlorine, nitro, methyl, ethyl, propyl, i-propyl, methoxy, ethoxy, or trifluoromethyl,
R³, R⁴ and R⁵ independently of one another represent C₁-C₄-alkyl,C₁-C₄-alkoxy, C₁-C₄-alkylamino, di-C₁-C₄-alkyl C₁-C₄-alkoxy, C₁-C₄-alkylamino, di-(C₁-C₄)-alkylamino or C₁-C₄-alkylthio, which are optionally substituted by fluorine or chlorine, or represent phenyl, phenoxy or phenylthio which are optionally substituted by fluorine, chlorine, bromine, nitro, cyano, C₁-C₂-alkoxy, C₁-C₄-fluoroalkoxy, C₁-C₂-chloroalkoxy, C₁-C₂-alkylthio, C₁-C₂-fluoroalkylthio, C₁-C₂-chloroalkylthio, C₁-C₃-alkyl or C₁-C₃-halogenoalkyl,
R⁶ and R⁷ independently of one another represent C₁-C₁₀-alkyl, C₁-C₁₀-alkoxy or C₁-C₁₀-alkoxy-C₁-C₁₀-alkyl, which are optionally substituted by fluorine, chlorine or bromine, or represents phenyl which is optionally substituted by fluorine, chlorine, bromine, C₁-C₂₀-halogenoalkyl, C₁-C₂₀-alkyl or C₁-C₄-alkoxy,
or represent benzyl which is optionally substituted by fluorine, chlorine, bromine, C₁-C₄-alkyl, C₁-C₄-halogenoalkyl or C₁-C₄-alkoxy,
with the proviso that
A and B independently of one another do not represent C₁-C₄-alkyl, C₃-C₄-alkenyl or C₃-C₄-alkinyl or
A and B together do not represent a radical from the series consisting of 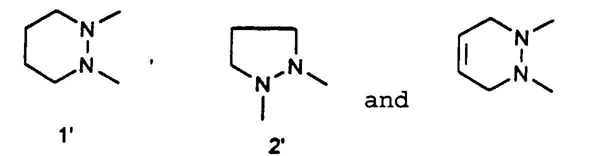 which is optionally up to trisubstituted by C₁-C₄-alkyl,
if G represents hydrogen,
characterised in that
(A) to obtain 3-hydroxy-4-aryl-5-oxo-pyrazolines of the formula (Ia) 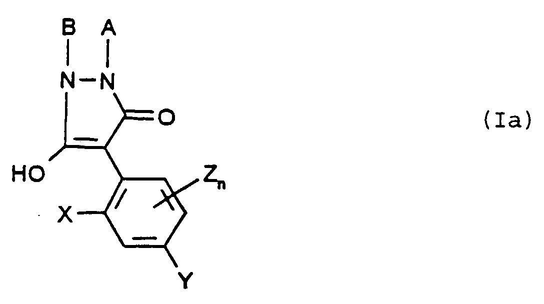 in which
A, B, X, Y, Z and n have the abovementioned meaning,
(α) halogenocarbonyl ketenes of the formula (II) 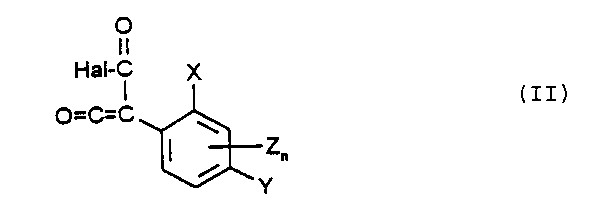 in which
X, Y, Z and n have the abovementioned meaning
and
Hal represents halogen, in particular chlorine or bromine,
or
(β) malonic acid derivatives of the formula (III) 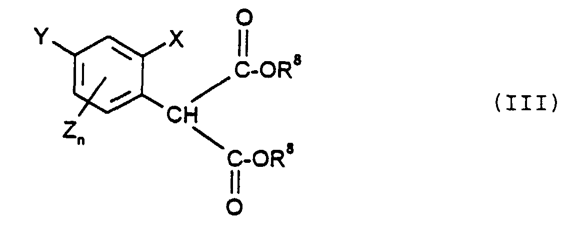 in which
X, Y, Z and n have the abovementioned meaning
and
R⁸ represents alkyl,
are reacted with hydrazines of the formula (IV)
A-NH-NH-B (IV)
in which
A and B have the abovementioned meaning,
if appropriate in the presence of a diluent and if appropriate in the presence of a base;
or
(B) to obtain compounds of the formula (Ib) 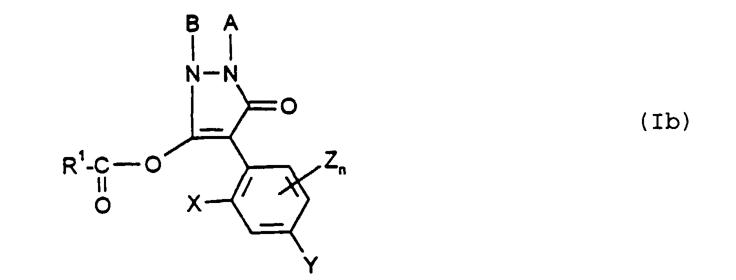 in which
A, B, X, Y, Z, R¹ and n have the abovementioned meaning,
compounds of the formula (Ia) 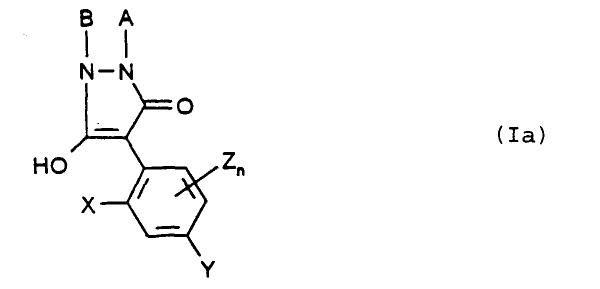 in which
A, B, X, Y, Z and n have the abovementioned meaning,
(α) are reacted with acid halides of the general formula (V) 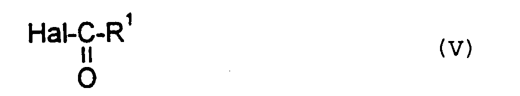 in which
R¹ has the abovementioned meaning
and
Hal represents halogen, in particular chlorine and bromine,
if appropriate in the presence of a diluent and if appropriate in the presence of an acid-binding agent,
or
(β) are reacted with carboxylic anhydrides of the general formula (VI)
R¹-CO-O-CO-R¹ (VI)
in which
R¹ has the abovementioned meaning,
if appropriate in the presence of a diluent and if appropriate in the presence of an acid-binding agent;
or
(C) to obtain compounds of the formula (Ic-1) 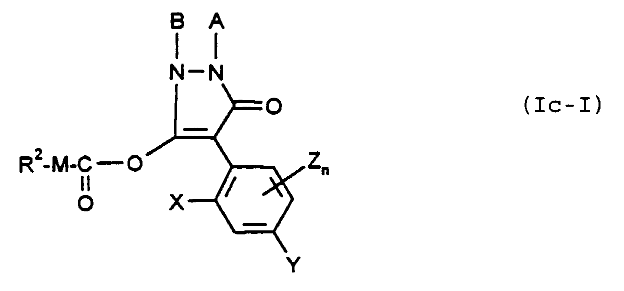 in which
A, B, X, Y, Z, R² and n have the abovementioned meaning,
and
M represents oxygen or sulphur,
compounds of the formula (Ia) 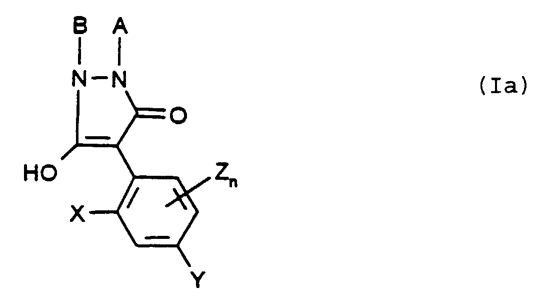 in which
A, B, X, Y, Z and n have the abovementioned meaning,
are reacted with chloroformic ester or chloroformic thioester of the general formula (VII)
R²-M-CO-Cl (VII)
in which
R² and M have the abovementioned meaning,
if appropriate in the presence of a diluent and if appropriate in the presence of an acid-binding agent;
or
(D) to obtain compounds of the formula (Ic-2) 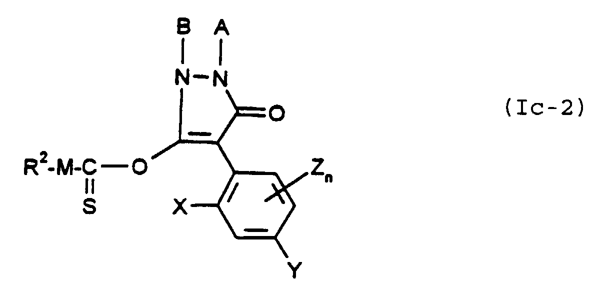 in which
A, B, R², X, Y, Z and n have the abovementioned meaning,
and
M represents oxygen or sulphur,
compounds of the formula (Ia) 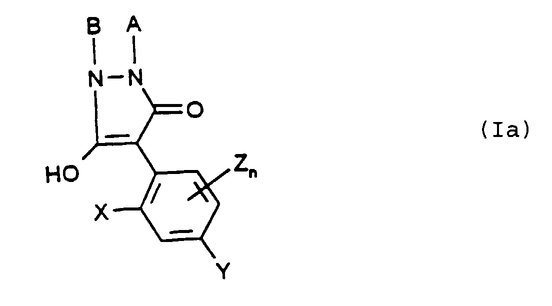 in which
A, B, X, Y, Z and n have the abovementioned meaning,
(α) are reacted with chloromonothioformic esters or chlorodithioformic esters of the general formula (VIII) 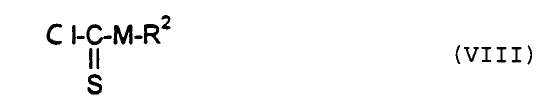 in which
M and R² have the abovementioned meaning,
if appropriate in the presence of a diluent and if appropriate in the presence of an acid-binding agent,
or
(β) are reacted with carbon disulphide and subsequently with alkyl halides of the general formula (IX)
R²-Hal (IX)
in which
R² has the abovementioned meaning
and
Hal represents chlorine, bromine or iodine;
or
(E) to obtain compounds of the formula (Id) 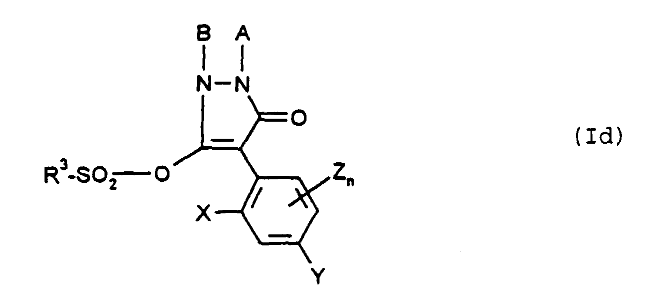 in which
A, B, X, Y, Z, R³ and n have the abovementioned meaning,
compounds of the formula (Ia) 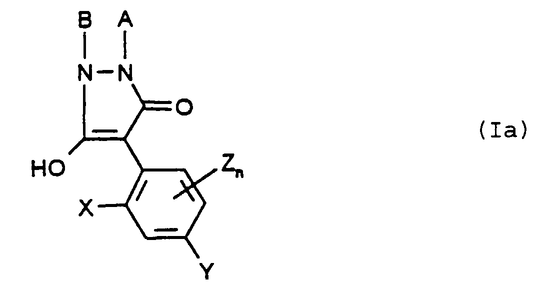 in which
A, B, X, Y, Z and n have the abovementioned meaning,
are reacted with sulphonyl chlorides of the general formula (X)
R³-SO₂-Cl (X)
in which
R³ has the abovementioned meaning,
if appropriate in the presence of a diluent and if appropriate in the presence of an acid-binding agent;
or
(F) to obtain compounds of the formula (Ie) 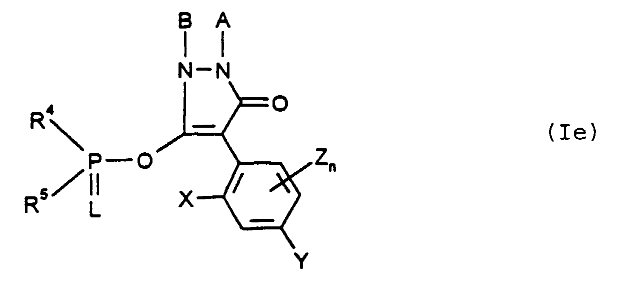 in which
A, B, L, X, Y, Z, R⁴, R⁵ and n have the above mentioned meaning,
3-hydroxy-4-aryl-5-oxo-pyrazolines of the formula (Ia) 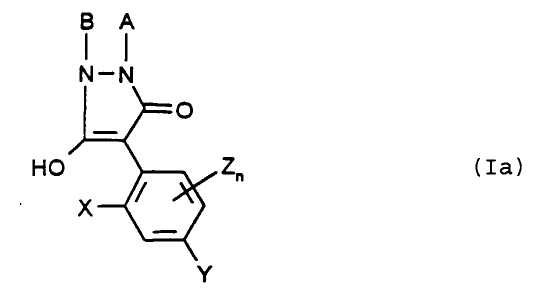 in which
A, B, X, Y, Z and n have the abovementioned meaning,
are reacted with phosphorus compounds of the general formula (XI) 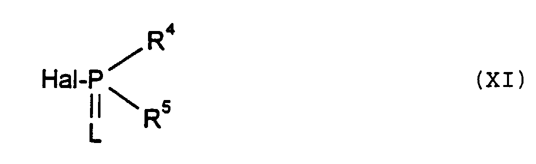 in which
L, R⁴ and R⁵ have the abovementioned meaning
and
Hal represents halogen, in particular chlorine and bromine,
if appropriate in the presence of a diluent and if appropriate in the presence of an acid-binding agent;
or
(G) to obtain compounds of the formula (If) 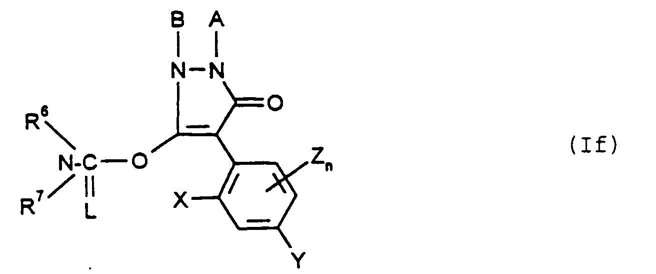 in which
A, B, L, X, Y, Z, R⁶, R⁷ and n have the above mentioned meaning,
compounds of the formula (Ia) 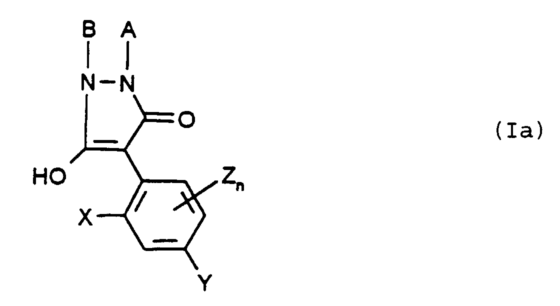 in which A, B, X, Y, Z and n have the abovementioned meaning,
(α) are reacted with isocyanates of the general formula (XII)
R⁶-N=C=O (XII)
in which
R⁶ has the abovementioned meaning,
if appropriate in the presence of a diluent and if appropriate in the presence of a catalyst,
or
(β) are reacted with carbamoyl chlorides or thiocarbamoyl chlorides of the general formula (XIII) 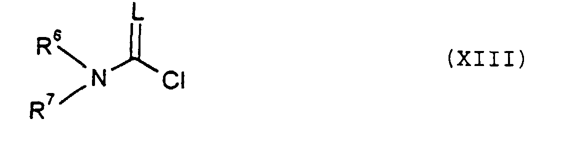 in which
L, R⁶ and R⁷ have the abovementioned meaning,
if appropriate in the presence of a diluent and if appropriate in the presence of an acid-binding agent,
or
(H) to obtain compounds of the formula (Ig) 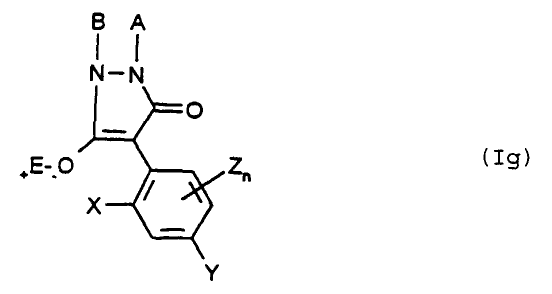 in which
A, B, X, Y, Z and n have the abovementioned meaning,
and
E represents a metal ion equivalent or an ammonium ion,
compounds of the formula (Ia) 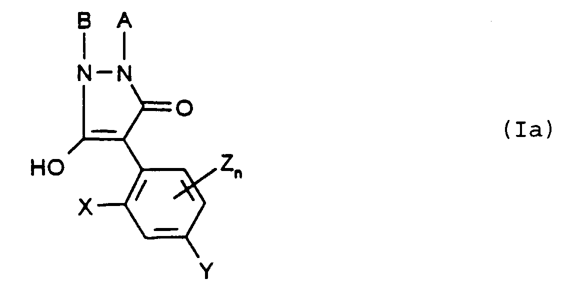 in which
A, B, X, Y, Z and n have the abovementioned meaning,
are reacted with metal hydroxides or amines of the general formulae (XIV) and (XV) 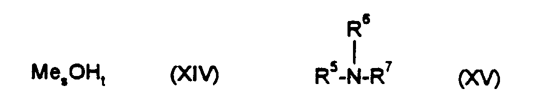 in which
Me represents monovalent or divalent metal ions,
s and t represent the number 1 and 2 and
R⁵, R⁶ and R⁷ independently of one another represent hydrogen and alkyl,
if appropriate in the presence of a diluent.

2. Pesticides, characterised in that they contain at least one 3-hydroxy-4-aryl-5-oxo-pyrazoline derivative of the formula (I).

3. Method of combating animal pests, characterised in that substituted 3-hydroxy-4-aryl-5-oxo-pyrazoline derivatives of the formula (I) are allowed to act on animal pests and/or their environment.

4. Use of substituted 3-hydroxy-4-aryl-5-oxo-pyrazoline derivatives of the formula (I) for combating animal pests.

5. Process for the preparation of pesticides, characterised in that substituted 3-hydroxy-4-aryl-5-oxopyrazoline derivatives of the formula (I) according to Claim 1 are mixed with extenders and/or surface-active agents.

6. Herbicidal agents, characterised in that they contain at least one 3-hydroxy-4-aryl-5-oxo-pyrazoline derivative of the formula (I) according to Claim 1.

7. Method of combating weeds, characterised in that 3-hydroxy-4-aryl-5-oxo-pyrazoline derivatives of the formula (I) according to Claim 1 are allowed to act on weeds and/or their environment.

8. Use of 3-hydroxy-4-aryl-5-oxo-pyrazoline derivatives of the formula (I) according to Claim 1 for combating weeds.

9. Process for the preparation of herbicidal agents, characterised in that 3-hydroxy-4-aryl-5-oxo-tpyrazoline derivatives of the formula (I) according to Claim 1 are mixed with extenders and/or surface-active agents.

10. Process for the preparation of halogenocarbonyl ketenes of the formula (IIa) 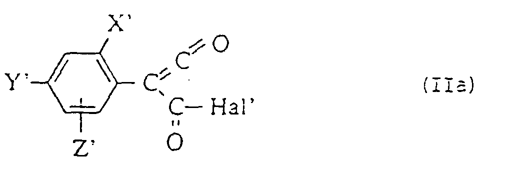 in which
Hal' represents halogen,
X' represents C₁-C₄-alkyl, halogen or trifluoromethyl,
Y' represents hydrogen, C₁-C₄-alkyl, halogen or trifluoromethyl
and
Z' represents hydrogen, C₁-C₄-alkyl, halogen or trifluoromethyl,
with the proviso that Z' does not represent hydrogen if Y' represents hydrogen,
characterized in that
arylmalonic acids of the formula (XVIa) 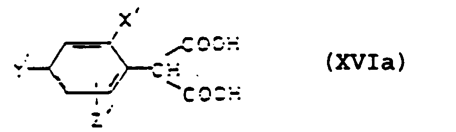 in which X', Y' and Z' have the abovementioned meaning, are reacted with acid halides, if appropriate in the presence of catalysts.

## Revendications (Revendications pour l'(les) Etat(s) contractant(s) suivant(s): BE, CH, DE, FR, GB, GR, IT, LI, NL)

1. Dérivés de 3-hydroxy-4-aryl-5-oxo-pyrazoline de formule (I) 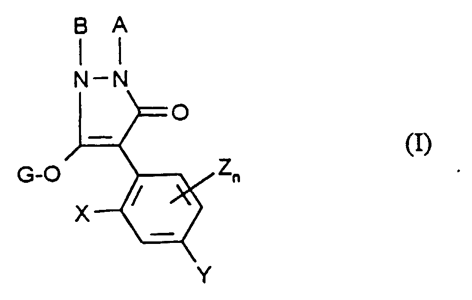 dans laquelle
A et B sont identiques ou différents et représentent chacun, indépendamment l'un de l'autre, un groupe alkyle en C₁ à C₄, alcényle en C₂ à C₄, alcynyle en C₂ à C₄, (alkoxy en C₁ à C₄)-(alkyle en C₁ à C₄), (alkylthio en C₁ à C₄)-(alkyle en C₁ à C₄), cycloalkyle ayant 3 à 7 atomes de carbone ou un groupe phényle portant éventuellement un à trois substituants identiques ou différents,
en considérant comme substituants du groupe phényle les substituants du groupe phényle indiqués pour R³, R⁴ et R⁵
A et B forment conjointement avec les deux atomes d'azote du noyau de pyrazoline, un groupement, portant éventuellement un à trois substituants identiques ou différents, des formules 1', 2', 3' ou 4' indiquées ci-après 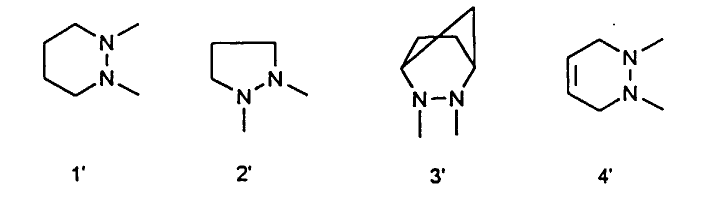 en considérant comme substituants un halogène, des groupes alkyle en C₁ à C₈, alcényle en C₂ à C₈ et (alkoxy en C₁ à C₄)-(alkyle en C₁ à C₄) éventuellement substitués par du fluor ou du chlore,
X représente les groupes méthyle, éthyle, propyle, isopropyle, le fluor, le chlore, le brome, les groupes méthoxy et éthoxy,
Y représente l'hydrogène, les groupes méthyle, éthyle, propyle, isopropyle, butyle, isobutyle, tertio-butyle, le fluor, le chlore, le brome, les groupes méthoxy, éthoxy et trifluorométhyle,
Z représente les groupes méthyle, éthyle, isopropyle, butyle, isobutyle, tertio-butyle, le fluor, le chlore, le brome, les groupes méthoxy et éthoxy,
n représente le nombre 0, 1, 2 ou 3,
G représente de l'hydrogène (a) ou les groupes 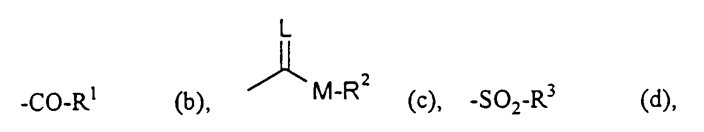 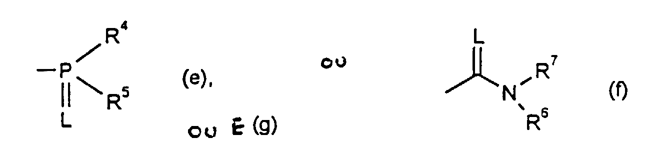 dans lesquels
E est un équivalent d'ion métallique ou un ion ammonium,
L et M représentent de l'oxygène et/ou du soufre,
R¹ est un groupe alkyle en C₁ à C₁₄, alcényle en C₂ à C₁₄, (alkoxy en C₁ à C₄)-(alkyle en C₂ à C₆), (alkylthio en C₁ à C₄)-(alkyle en C₂ à C₆), poly(alkoxy en C₁ à C₄)-(alkyle en C₂ à C₄) ou cycloalkyle à noyau de 3 à 6 atomes, qui peut être interrompu par 1 ou 2 atomes d'oxygène et/ou de soufre, chacun éventuellement substitué par du fluor ou du chlore, un groupe phényle facultativement substitué par du fluor, du chlore, du brome, les radicaux méthyle, éthyle, propyle, isopropyle, méthoxy, éthoxy, trifluorométhyle, trifluorométhoxy, nitro,
un groupe phényl-(alkyle en C₁ à C₃) éventuellement substitué par du fluor, du chlore, du brome, les radicaux méthyle, éthyle, propyle, isopropyle, méthoxy, éthoxy, trifluorométhyle, trifluorométhoxy,
les groupes pyridyle, pyrimidyle, thiazolyle et pyrazolyle éventuellement substitués par du fluor, du chlore, du brome, les radicaux méthyle, éthyle,
un groupe phénoxy-(alkyle en C₁ à C₄) éventuellement substitué par du fluor, du chlore, les radicaux méthyle, éthyle,
les groupes pyridyloxy-(alkyle en C₁ à C₄), pyrimidyloxy-(alkyle en C₁ à C₄) et thiazolyloxy-(alkyle en C₁ à C₄) éventuellement substitués par du fluor, du chlore, les radicaux amino, méthyle, éthyle,
R² représente des groupes alkyle en C₁ à C₁₄, alcényle en C₂ à C₁₄, (alkoxy en C₁ à à C₄)(alkyle en C₂ à C₆), poly(alkoxy en C₁ à C₄)(alkyle en C₂ à C₆) éventuellement substitués par du fluor ou du chlore,
ou un groupe phényle ou benzyle éventuellement substitué par du fluor, du chlore, les radicaux nitro, méthyle, éthyle, propyle, isopropyle, méthoxy, éthoxy, trifluorométhyle,
R³, R⁴ et R⁵ représentent, indépendamment les uns des autres, les groupes alkyle en C₁ à C₄, alkoxy en C₁ à C₄, alkylamino en C₁ à C₄, di(alkyle en C₁ à C₄)amino, alkylthio en C₁ à C₄ éventuellement substitués par du fluor ou du chlore, un groupe phényle, phénoxy ou phénylthio éventuellement substitué par du fluor, du chlore, du brome, les radicaux nitro, cyano, alkoxy en C₁ ou C₂, fluoralkoxy en C₁ à C₄, chloralkoxy en C₁ ou C₂, alkylthio en C₁ ou C₂, fluoralkylthio en C₁ ou C₂, chloralkylthio en C₁ ou C₂, alkyle en C₁ à C₃, halogénalkyle en C₁ à C₃,
R⁶ et R⁷ représentent, indépendamment l'un de l'autre, des groupes alkyle en C₁ à C₁₀, alkoxy en C₁ à C₁₀, (alkoxy en C₁ à C₁₀)(alkyle en C₁ à C₁₀) éventuellement substitués par du fluor, du chlore, du brome,
un groupe phényle éventuellement substitué par du fluor, du chlore, du brome, des radicaux halogénalkyle en C₁ à C₂₀, alkyle en C₁ à C₂₀ ou alkoxy en C₁ à C₄,
un groupe benzyle éventuellement substitué par du fluor, du chlore, du brome, des radicaux alkyle en C₁ à C₄, halogénalkyle en C₁ à C₄ ou alkoxy en C₁ à C₄,
sous réserve que
A et B, indépendamment l'un de l'autre, ne représentent pas un groupe alkyle en C₁ à C₄, alcényle en C₃ ou C₄ ou alcynyle en C₃ ou C₄ ou que
A et B ne forment pas ensemble un reste, éventuellement substitué jusqu'à trois fois par des radicaux alkyle en C₁ à C₄, de la série 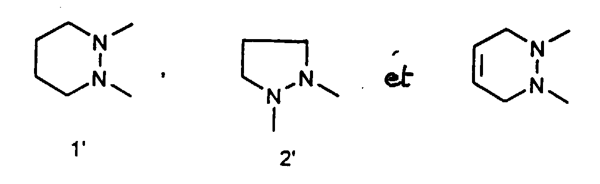 lorsque G représente de l'hydrogène.

2. Procédé de production de dérivés de 3-hydroxy-4-aryl-5-oxo-pyrazoline de formule (I) suivant la revendication 1, caractérisé en ce que
(A) pour obtenir des 3-hydroxy-4-aryl-5-oxo-pyrazolines de formule (Ia) 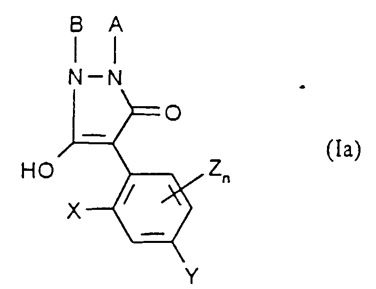 dans laquelle
A, B, X, Y, Z et n ont la définition indiquée ci-dessus,
on fait réagir
(α) des halogénocarbonylcétènes de formule (II) 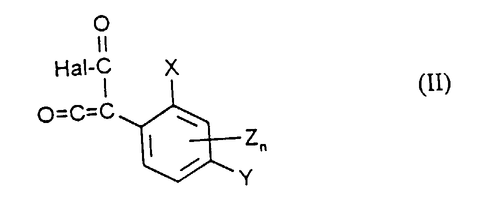 dans laquelle
X, Y, Z et n ont la définition indiquée ci-dessus
et
Hal représente un halogène, notamment du chlore ou du brome,
ou bien
(β) des dérivés d'acide malonique de formule (III) 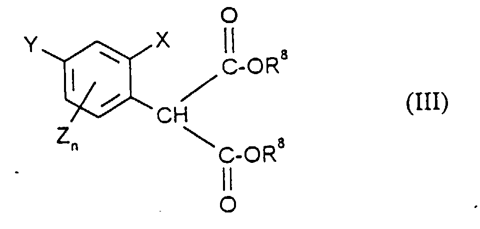 dans laquelle
X, Y, Z et n ont la définition indiquée ci-dessus
et
R⁸ est un groupe alkyle,
avec des hydrazines de formule (IV)
A-NH-NH-B (IV)
dans laquelle
A et B ont la définition indiquée ci-dessus,
le cas échéant en présence d'un diluant et en la présence éventuelle d'une base,
ou bien
(B) pour obtenir des composés de formule (Ib) 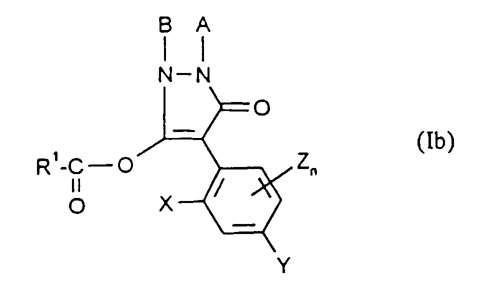 dans laquelle
A, B, X, Y, Z, R¹ et n ont la définition indiquée ci-dessus,
on fait réagir des composés de formule (Ia), 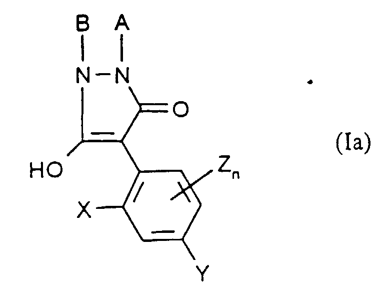 dans laquelle
A, B, X, Y, Z et n ont la définition indiquée ci-dessus,
(α) avec des halogénures d'acides de formule générale (V) 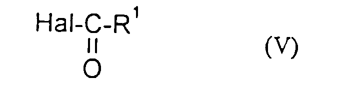 dans laquelle
R¹ a la définition indiquée ci-dessus,
et
Hal représente un halogène, en particulier le chlore et le brome,
le cas échéant en présence d'un diluant et en la présence éventuelle d'un accepteur d'acide,
ou bien
(β) avec des anhydrides d'acides carboxyliques de formule générale (VI)
R¹-CO-O-CO-R¹ (VI)
dans laquelle
R¹ a la définition indiquée ci-dessus,
le cas échéant en présence d'un diluant et en la présence éventuelle d'un accepteur d'acide,
ou bien
(C) pour obtenir des composés de formule (Ic-1) 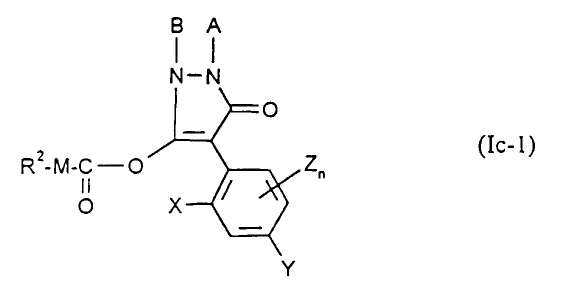 dans laquelle
A, B, X, Y, Z, R² et n ont la définition indiquée ci-dessus,
et
M est de l'oxygène ou du soufre,
on fait réagir des composés de formule (Ia) 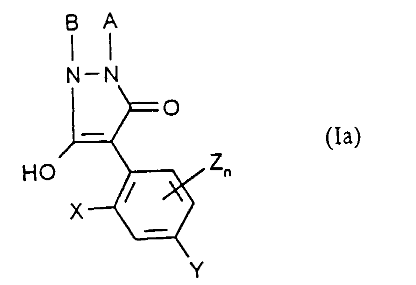 dans laquelle
A, B, X, Y, Z et n ont la définition indiquée ci-dessus, avec un ester d'acide chloroformique ou un thiolester d'acide chloroformique de formule générale (VII)
R²-M-CO-Cl (VII)
dans laquelle
R² et M ont la définition indiquée ci-dessus,
le cas échéant en présence d'un diluant et en la présence éventuelle d'un accepteur d'acide,
ou bien
(D) pour obtenir des composés de formule (Ic-2) 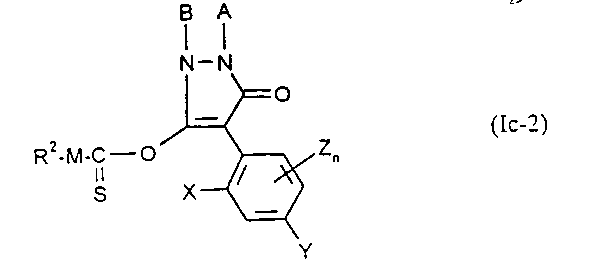 dans laquelle
A, B, R², X, Y, Z et n ont la définition indiquée ci-dessus,
et
M représente de l'oxygène ou du soufre,
on fait réagir des composés de formule (la) 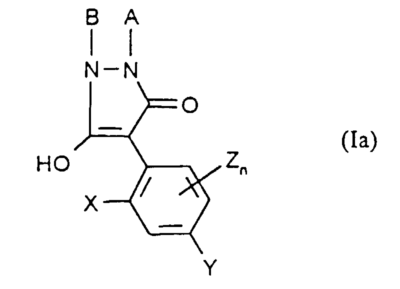 dans laquelle
A, B, X, Y, Z et n ont la définition indiquée ci-dessus,
(α) avec des esters d'acide chloromonothioformique ou des esters d'acide chlorodithioformique de formule générale (VIII) 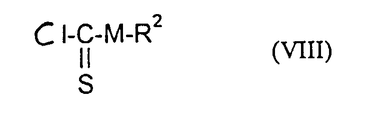 dans laquelle
M et R² ont la définition indiquée ci-dessus,
le cas échéant en présence d'un diluant et en la présence éventuelle d'un accepteur d'acide,
ou bien
(β) avec du sulfure de carbone, puis avec des halogénures d'alkyle de formule générale (IX)
R²-Hal (IX)
dans laquelle
R² a la définition ci-dessus,
et
Hal représente du chlore, du brome ou de l'iode,
ou bien
(E) pour obtenir des composés de formule (Id) 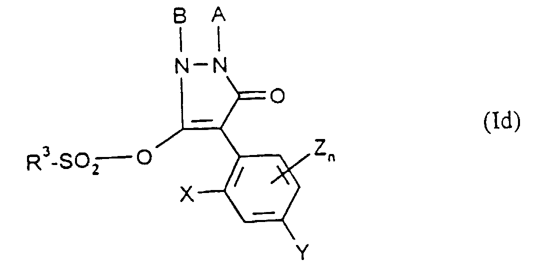 dans laquelle
A, B, X, Y, Z, R³ et n ont la définition indiquée ci-dessus,
on fait réagir des composés de formule (Ia) 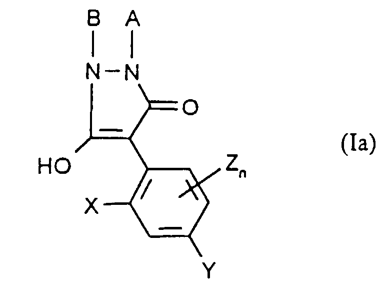 dans laquelle
A, B, X, Y, Z et n ont la définition indiquée ci-dessus,
avec des chlorures d'acides sulfoniques de formule générale (X)
R³-SO₂-Cl (X)
dans laquelle
R³ a la définition indiquée ci-dessus,
le cas échéant en présence d'un diluant et en la présence éventuelle d'un accepteur d'acide,
ou bien
(F) pour obtenir des composés de formule (Ie) 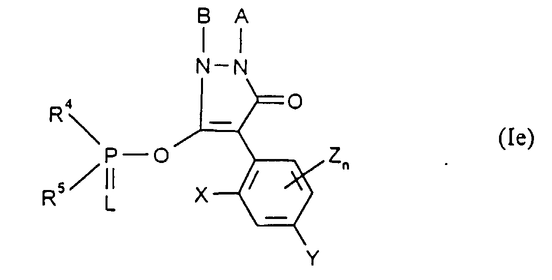 dans laquelle
A, B, L, X, Y, Z, R⁴, R⁵ et n ont la définition indiquée ci-dessus,
on fait réagir des 3-hydroxy-4-aryl-5-oxo-pyrazolines de formule générale (Ia) 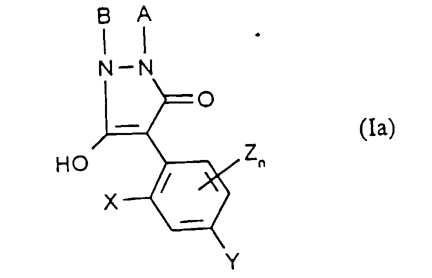 dans laquelle
A, B, X, Y, Z et n ont la définition indiquée ci-dessus, avec des composés phosphorés de formule générale (XI) 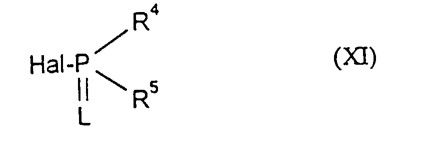 dans laquelle
L, R⁴ et R⁵ ont la définition indiquée ci-dessus,
et
Hal représente un halogène, en particulier le chlore ou le brome,
le cas échéant en présence d'un diluant et en la présence éventuelle d'un accepteur d'acide,
ou bien
(G) pour obtenir des composés de formule (If) 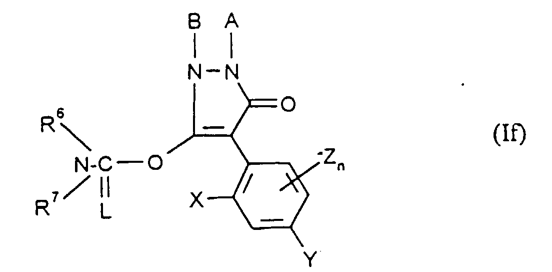 dans laquelle
A, B, L, X, Y, Z, R⁶, R⁷ et n ont la définition indiquée ci-dessus,
on fait réagir des composés de formule (Ia) 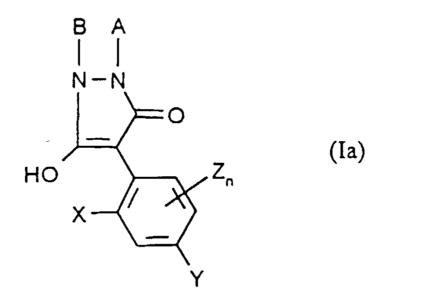 dans laquelle
A, B, X, Y, Z et n ont la définition indiquée ci-dessus,
(α) avec des isocyanates de formule générale (XII)
R⁶-N=C=O (XII)
dans laquelle
R⁶ a la définition indiquée ci-dessus,
le cas échéant en présence d'un diluant et en la présence éventuelle d'un catalyseur,
ou bien
(β) avec des chlorures d'acide carbamique ou des chlorures d'acide thiocarbamique de formule générale (XIII) 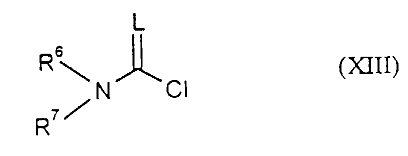 dans laquelle
L, R⁶ et R⁷ ont la définition indiquée ci-dessus,.
le cas échéant en présence d'un diluant et en la présence éventuelle d'un accepteur d'acide,
ou bien
(H) pour obtenir des composés de formule (Ig) 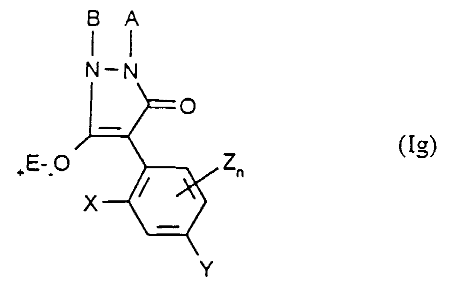 dans laquelle
A, B, X, Y, Z et n ont la définition indiquée ci-dessus,
et
E est un équivalent d'ion métallique ou un ion ammonium,
on fait réagir des composés de formule (Ia) 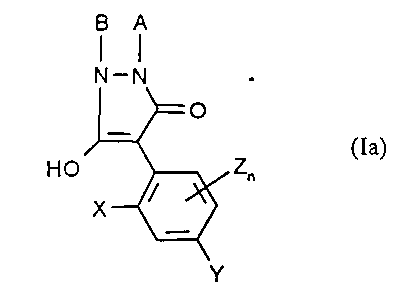 dans laquelle
A, B, X, Y, Z et n ont la définition indiquée ci-dessus, avec des hydroxydes métalliques ou des amines de formules générales (XIV) et (XV) 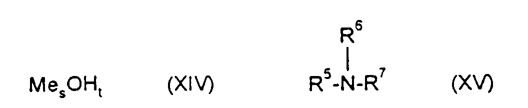 dans lesquelles
Me désigne des ions métalliques monovalents ou divalents,
s et t représentent les nombres 1 et 2 et
R⁵, R⁶ et R⁷ représentent, indépendamment les uns des autres, de l'hydrogène et des groupes alkyle,
le cas échéant en présence d'un diluant.

3. Compositions pesticides, caractérisées par une teneur en au moins un dérivé de 3-hydroxy-4-aryl-5-oxo-pyrazoline de formule (I) suivant la revendication 1.

4. Procédé pour combattre des parasites animaux, caractérisé en ce qu'on fait agir des dérivés substitués de 3-hydroxy-4-aryl-5-oxo-pyrazoline de formule (I) suivant la revendication 1 sur les parasites animaux et/ou sur leur habitat.

5. Utilisation de dérivés substitués de 3-hydroxy-4-aryl-5-oxo-pyrazoline de formule (I) suivant la revendication 1 pour combattre des parasites animaux.

6. Procédé de préparation de compositions pesticides, caractérisé en ce qu'on mélange des dérivés substitués de 3-hydroxy-4-aryl-5-oxo-pyrazoline de formule (I) suivant la revendication 1 avec des diluants et/ou des agents tensio-actifs.

7. Compositions herbicides, caractérisées par une teneur en au moins un dérivé de 3-hydroxy-4-aryl-5-oxo-pyrazoline de formule (I) suivant la revendication 1.

8. Procédé pour combattre des mauvaises herbes, caractérisé en ce qu'on fait agir des dérivés de 3-hydroxy-4-aryl-5-oxo-pyrazoline de formule (I) suivant la revendication 1 sur les mauvaises herbes et/ou sur leur milieu.

9. Utilisation de dérivés de 3-hydroxy-4-aryl-5-oxo-pyrazoline de formule (I) suivant la revendication 1 pour combattre des mauvaises herbes.

10. Procédé de préparation de compositions herbicides, caractérisé en ce qu'on mélange des dérivés de 3-hydroxy-4-aryl-5-oxo-pyrazoline de formule (I) suivant la revendication 1 avec des diluants et/ou des agents tensio-actifs.

11. Halogénocarbonylcétènes de formule (IIa) 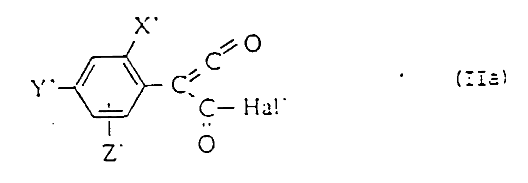 dans laquelle
Hal' représente un halogène,
X' est un groupe alkyle en C₁ à C₄, un halogène ou un groupe trifluorométhyle,
Y' est de l'hydrogène, un groupe alkyle en C₁ à C₄, un halogène ou un groupe trifluorométhyle,
et
Z' représente de l'hydrogène, un groupe alkyle en C₁ à C₄, un halogène ou un groupe trifluorométhyle,
sous réserve que Z' ne représente pas de l'hydrogène lorsque Y' est de l'hydrogène.

12. Halogénocarbonylcétènes de formule (IIa) suivant la revendication 11,
dans laquelle
Hal' est du brome ou du chlore,
X' est un groupe méthyle, du fluor, du chlore, du brome ou un groupe trifluorométhyle,
Y' est de l'hydrogène, un groupe méthyle, éthyle, n-propyle, isopropyle, n-butyle, isobutyle, tertio-butyle, du fluor, du chlore ou du brome ou un groupe trifluorométhyle
et
Z' est de l'hydrogène, un groupe méthyle, du fluor, du chlore, du brome ou un groupe trifluorométhyle,
sous réserve que Z' ne représente pas de l'hydrogène lorsque Y' est de l'hydrogène.

13. Halogénocarbonylcétènes de formule (IIa) suivant la revendication 15,
dans laquelle
X', Y' et Z' représentent chacun un groupe méthyle ou bien X' et Y' représentent du chlore en même temps que Z' est de l'hydrogène.

14. Procédé de production d'halogénocarbonylcétènes de formule (IIa) 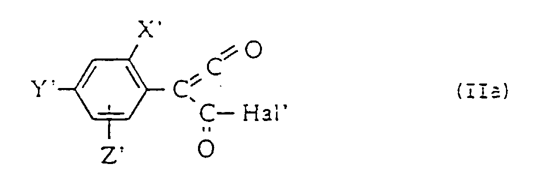 dans laquelle
Hal' représente un halogène,
X' est un groupe alkyle en C₁ à C₄, un halogène ou un groupe trifluorométhyle,
Y' est de l'hydrogène, un groupe alkyle en C₁ à C₄, un halogène ou un groupe trifluorométhyle, et
Z' représente de l'hydrogène, un groupe alkyle en C₁ à C₄, un halogène ou un groupe trifluorométhyle,
sous réserve que Z' ne représente pas de l'hydrogène lorsque Y' est de l'hydrogène,
caractérisé en ce qu'on fait réagir des acides arylmaloniques de formule (XVIa) 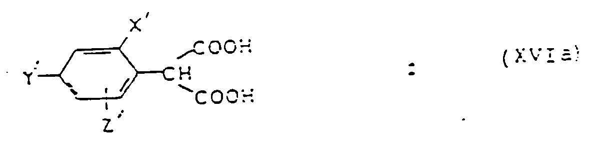 dans laquelle X', Y' et Z' ont la définition indiquée ci-dessus,
avec des halogénures d'acides, le cas échéant en présence de catalyseurs.

15. Acides arylmaloniques de formule (XVIa) 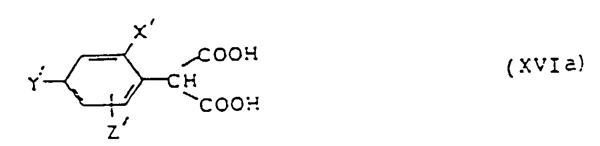 dans laquelle
X', Y' et Z' ont la définition indiquée dans la revendication 14.

## Revendications (Revendications pour l'(les) Etat(s) contractant(s) suivant(s): ES)

1. Procédé de production de dérivés de 3-hydroxy-4-aryl-5-oxo-pyrazoline de formule (I) 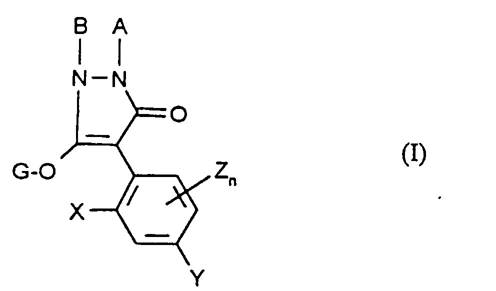 dans laquelle
A et B sont identiques ou différents et représentent chacun, indépendamment l'un de l'autre, un groupe alkyle en C₁ à C₄, alcényle en C₂ à C₄, alcynyle en C₂ à C₄, (alkoxy en C₁ à C₄)-(alkyle en C₁ à C₄), (alkylthio en C₁ à C₄)-(alkyle en C₁ à C₄), cycloalkyle ayant 3 à 7 atomes de carbone ou un groupe phényle portant éventuellement un à trois substituants identiques ou différents,
en considérant comme substituants du groupe phényle les substituants du groupe phényle indiqués pour R³, R⁴ et R⁵
A et B forment conjointement avec les deux atomes d'azote du noyau de pyrazoline, un groupement, portant éventuellement un à trois substituants identiques ou différents, des formules 1', 2', 3' ou 4' indiquées ci-après 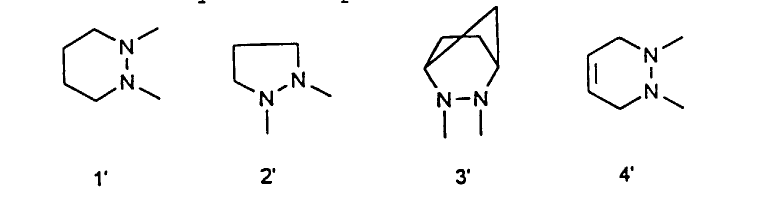 en considérant comme substituants un halogène, des groupes alkyle en C₁ à C₈, alcényle en C₂ à C₈ et (alkoxy en C₁ à C₄)-(alkyle en C₁ à C₄) éventuellement substitués par du fluor ou du chlore,
X représente les groupes méthyle, éthyle, propyle, isopropyle, le fluor, le chlore, le brome, les groupes méthoxy et éthoxy,
Y représente l'hydrogène, les groupes méthyle, éthyle, propyle, isopropyle, butyle, isobutyle, tertio-butyle, le fluor, le chlore, le brome, les groupes méthoxy, éthoxy et trifluorométhyle,
Z représente les groupes méthyle, éthyle, isopropyle, butyle, isobutyle, tertio-butyle, le fluor, le chlore, le brome, les groupes méthoxy et éthoxy,
n représente le nombre 0, 1, 2 ou 3,
G représente de l'hydrogène (a) ou les groupes 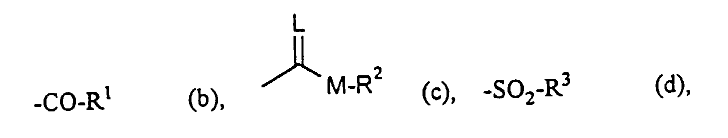 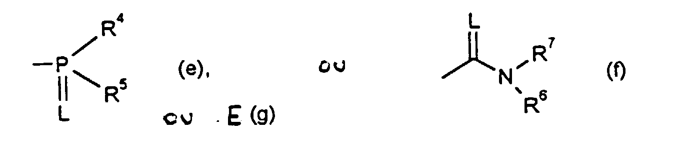 dans lesquels
E est un équivalent d'ion métallique ou un ion ammonium,
L et M représentent de l'oxygène et/ou du soufre,
R¹ est un groupe alkyle en C₁ à C₁₄, alcényle en C₂ à C₁₄, (alkoxy en C₁ à C₄)-(alkyle en C₂ à C₆), (alkylthio en C₁ à C₄)-(alkyle en C₂ à C₆), poly(alkoxy en C₁ à C₄)-(alkyle en C₂ à C₄) ou cycloalkyle à noyau de 3 à 6 atomes, qui peut être interrompu par 1 ou 2 atomes d'oxygène et/ou de soufre, chacun éventuellement substitué par du fluor ou du chlore, un groupe phényle facultativement substitué par du fluor, du chlore, du brome, les radicaux méthyle, éthyle, propyle, isopropyle, méthoxy, éthoxy, trifluorométhyle, trifluorométhoxy, nitro,
un groupe phényl-(alkyle en C₁ à C₃) éventuellement substitué par du fluor, du chlore, du brome, les radicaux méthyle, éthyle, propyle, isopropyle, méthoxy, éthoxy, trifluorométhyle, trifluorométhoxy,
les groupes pyridyle, pyrimidyle, thiazolyle et pyrazolyle éventuellement substitués par du fluor, du chlore, du brome, les radicaux méthyle, éthyle,
un groupe phénoxy-(alkyle en C₁ à C₄) éventuellement substitué par du fluor, du chlore, les radicaux méthyle, éthyle,
les groupes pyridyloxy-(alkyle en C₁ à C₄), pyrimidyloxy-(alkyle en C₁ à C₄) et thiazolyloxy-(alkyle en C₁ à C₄) éventuellement substitués par du fluor, du chlore, les radicaux amino, méthyle, éthyle,
R² représente des groupes alkyle en C₁ à C₁₄, alcényle en C₂ à C₁₄, (alkoxy en C₁ à C₄)-(alkyle en C₂ à C₆), poly(alkoxy en C₁ à C₄)-(alkyle en C₂ à C₆) éventuellement substitués par du fluor ou du chlore,
ou un groupe phényle ou benzyle éventuellement substitué par du fluor, du chlore, les radicaux nitro, méthyle, éthyle, propyle, isopropyle, méthoxy, éthoxy, trifluorométhyle,
R³, R⁴ et R⁵ représentent, indépendamment les uns des autres, les groupes alkyle en C₁ à C₄, alkoxy en C₁ à C₄, alkylamino en C₁ à C₄, di(alkyle en C₁ à C₄)amino, alkylthio en C₁ à C₄ éventuellement substitués par du fluor ou du chlore, un groupe phényle, phénoxy ou phénylthio éventuellement substitué par du fluor, du chlore, du brome, les radicaux nitro, cyano, alkoxy en C₁ ou C₂, fluoralkoxy en C₁ à C₄, chloralkoxy en C₁ ou C₂, alkylthio en C₁ ou C₂, fluoralkylthio en C₁ ou C₂, chloralkylthio en C₁ ou C₂, alkyle en C₁ à C₃, halogénalkyle en C₁ à C₃,
R⁶ et R⁷ représentent, indépendamment l'un de l'autre, des groupes alkyle en C₁ à C₁₀, alkoxy en C₁ à C₁₀, (alkoxy en C₁ à C₁₀)-(alkyle en C₁ à C₁₀) éventuellement substitués par du fluor, du chlore, du brome,
un groupe phényle éventuellement substitué par du fluor, du chlore, du brome, des radicaux halogénalkyle en C₁ à C₂₀, alkyle en C₁ à C₂₀ ou alkoxy en C₁ à C₄,
un groupe benzyle éventuellement substitué par du fluor, du chlore, du brome, des radicaux alkyle en C₁ à C₄, halogénalkyle en C₁ à C₄ ou alkoxy en C₁ à C₄,
sous réserve que
A et B, indépendamment l'un de l'autre, ne représentent pas un groupe alkyle en C₁ à C₄, alcényle en C₃ ou C₄ ou alcynyle en C₃ ou C₄ ou que
A et B ne forment pas ensemble un reste, éventuellement substitué jusqu'à trois fois par des radicaux alkyle en C₁ à C₄, de la série 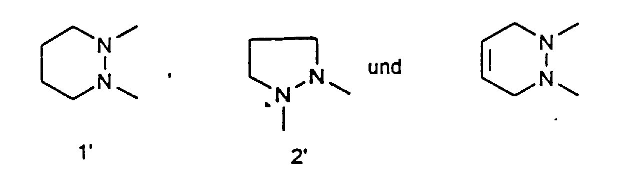
lorsque G représente de l'hydrogène,
caractérisé en ce que
(A) pour obtenir des 3-hydroxy-4-aryl-5-oxo-pyrazolines de formule (Ia) 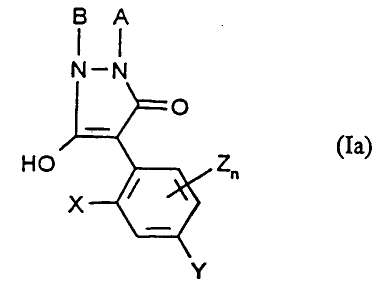 dans laquelle
A, B, X, Y, Z et n ont la définition indiquée ci-dessus,
on fait réagir
(α) des halogénocarbonylcétènes de formule (II) 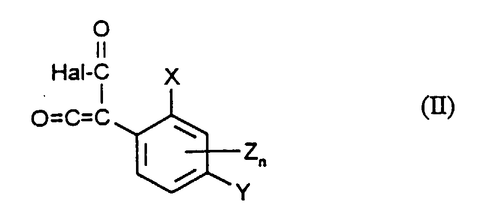 dans laquelle
X, Y, Z et n ont la définition indiquée ci-dessus
et
Hal représente un halogène, notamment du chlore ou du brome,
ou bien
(β) des dérivés d'acide malonique de formule (III) 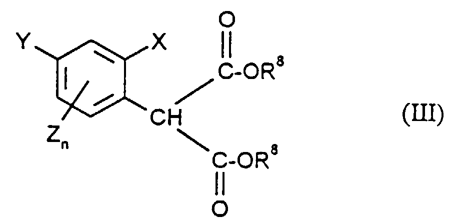 dans laquelle
X, Y, Z et n ont la définition indiquée ci-dessus
et
R⁸ est un groupe alkyle,
avec des hydrazines de formule (IV)
A-NH-NH-B (IV)
dans laquelle
A et B ont la définition indiquée ci-dessus,
le cas échéant en présence d'un diluant et en la présence éventuelle d'une base,
ou bien
(B) pour obtenir des composés de formule (Ib) 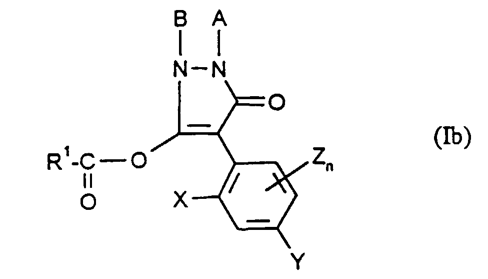 dans laquelle
A, B, X, Y, Z, R¹ et n ont la définition indiquée ci-dessus,
on fait réagir des composés de formule (Ia), 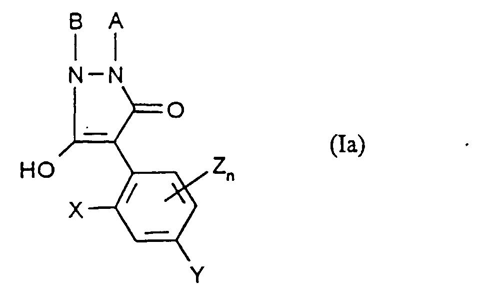 dans laquelle
A, B, X, Y, Z et n ont la définition indiquée ci-dessus,
(α) avec des halogénures d'acides de formule générale (V) 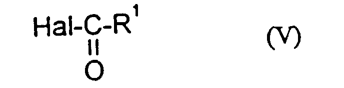 dans laquelle
R¹ a la définition indiquée ci-dessus,
et
Hal représente un halogène, en particulier le chlore et le brome,
le cas échéant en présence d'un diluant et en la présence éventuelle d'un accepteur d'acide,
ou bien
(β) avec des anhydrides d'acides carboxyliques de formule générale (VI)
R¹-CO-O-CO-R¹ (VI)
dans laquelle
R¹ a la définition indiquée ci-dessus,
le cas échéant en présence d'un diluant et en la présence éventuelle d'un accepteur d'acide,
ou bien
(C) pour obtenir des composés de formule (Ic-1) 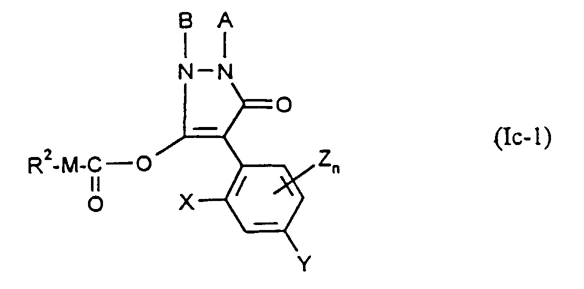 dans laquelle
A, B, X, Y, Z, R² et n ont la définition indiquée ci-dessus,
et
M est de l'oxygène ou du soufre,
on fait réagir des composés de formule (Ia) 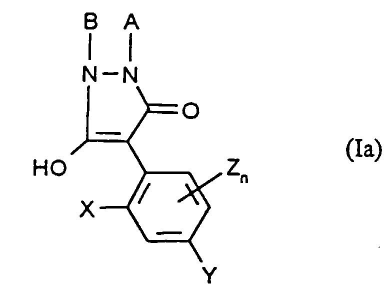 dans laquelle
A, B, X, Y, Z et n ont la définition indiquée ci-dessus,
avec un ester d'acide chloroformique ou un thiolester d'acide chloroformique de formule générale (VII)
R²-M-CO-Cl (VII)
dans laquelle
R² et M ont la définition indiquée ci-dessus,
le cas échéant en présence d'un diluant et en la présence éventuelle d'un accepteur d'acide,
ou bien
(D) pour obtenir des composés de formule (Ic-2) 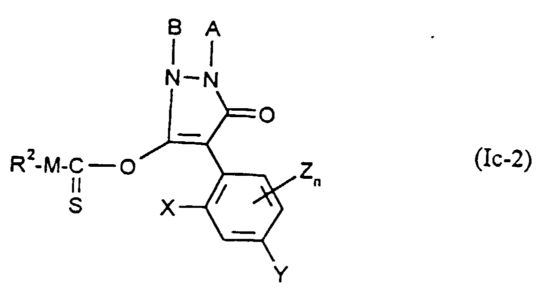 dans laquelle
A, B, R², X, Y, Z et n ont la définition indiquée ci-dessus,
et
M représente de l'oxygène ou du soufre,
on fait réagir des composés de formule (Ia) 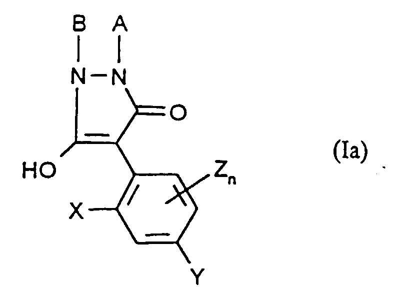 dans laquelle
A, B, X, Y, Z et n ont la définition indiquée ci-dessus,
(α) avec des esters d'acide chloromonothioformique ou des esters d'acide chlorodithioformique de formule générale (VIII) 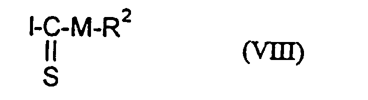 dans laquelle
M et R² ont la définition indiquée ci-dessus,
le cas échéant en présence d'un diluant et en la présence éventuelle d'un accepteur d'acide,
ou bien
(β) avec du sulfure de carbone, puis avec des halogénures d'alkyle de formule générale (IX)
R²-Hal (IX)
dans laquelle
R² a la définition ci-dessus,
et
Hal représente du chlore, du brome ou de l'iode,
ou bien
(E) pour obtenir des composés de formule (Id) 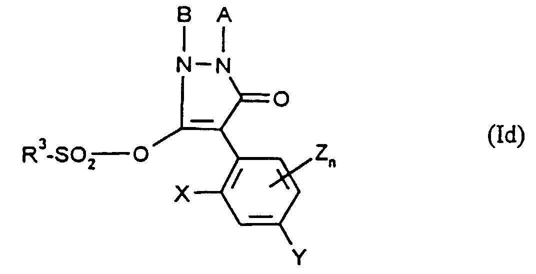 dans laquelle
A, B, X, Y, Z, R³ et n ont la définition indiquée ci-dessus,
on fait réagir des composés de formule (Ia) 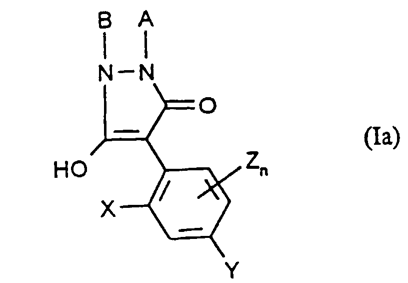 dans laquelle
A, B, X, Y, Z et n ont la définition indiquée ci-dessus,
avec des chlorures d'acides sulfoniques de formule générale (X)
R³-SO₂-Cl (X)
dans laquelle
R³ a la définition indiquée ci-dessus,
le cas échéant en présence d'un diluant et en la présence éventuelle d'un accepteur d'acide,
ou bien
(F) pour obtenir des composés de formule (Ie) 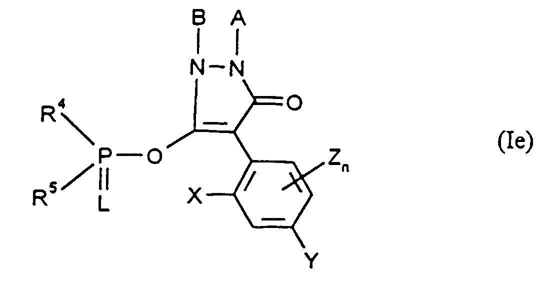 dans laquelle
A, B, L, X, Y, Z, R⁴, R⁵ et n ont la définition indiquée ci-dessus,
on fait réagir des 3-hydroxy-4-aryl-5-oxo-pyrazolines de formule générale (Ia) 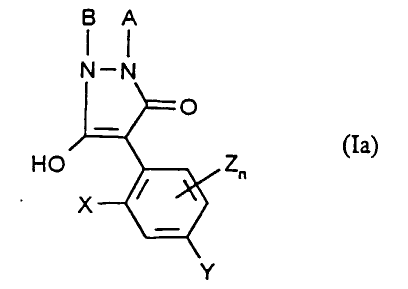 dans laquelle
A, B, X, Y, Z et n ont la définition indiquée ci-dessus, avec des composés phosphorés de formule générale (XI) 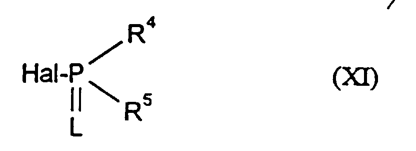 dans laquelle
L, R⁴ et R⁵ ont la définition indiquée ci-dessus,
et
Hal représente un halogène, en particulier le chlore ou le brome,
le cas échéant en présence d'un diluant et en la présence éventuelle d'un accepteur d'acide,
ou bien
(G) pour obtenir des composés de formule (If) 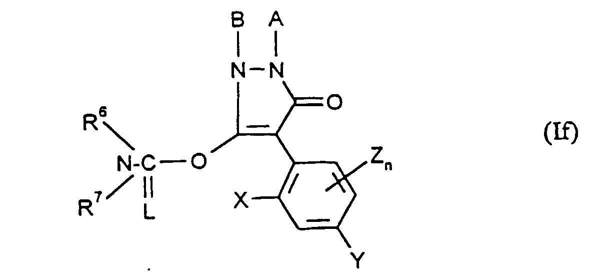 dans laquelle
A, B, L, X, Y, Z, R⁶, R⁷ et n ont la définition indiquée ci-dessus,
on fait réagir des composés de formule (Ia) 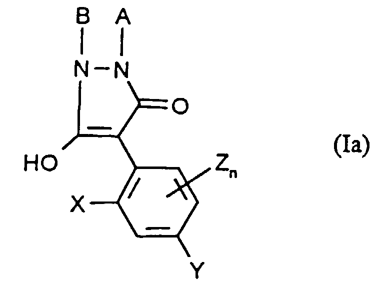 dans laquelle
A, B, X, Y, Z et n ont la définition indiquée ci-dessus,
(α) avec des isocyanates de formule générale (XII)
R⁶-N=C=O (XII)
dans laquelle
_{R}6 a la définition indiquée ci-dessus,
le cas échéant en présence d'un diluant et en la présence éventuelle d'un catalyseur,
ou bien
(β) avec des chlorures d'acide carbamique ou des chlorures d'acide thiocarbamique de formule générale (XIII) 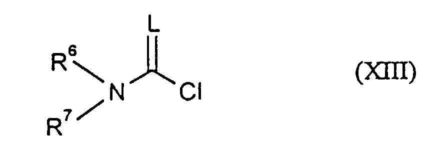 dans laquelle
L, R⁶ et R⁷ ont la définition indiquée ci-dessus,
le cas échéant en présence d'un diluant et en la présence éventuelle d'un accepteur d'acide,
ou bien
(H) pour obtenir des composés de formule (Ig) 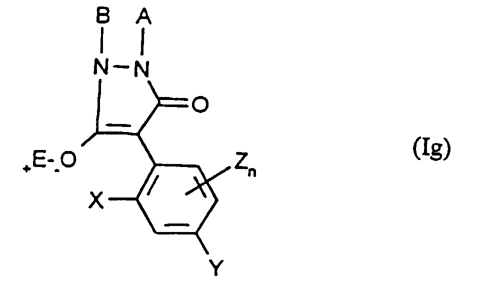 dans laquelle
A, B, X, Y, Z et n ont la définition indiquée ci-dessus,
et
E est un équivalent d'ion métallique ou un ion ammonium,
on fait réagir des composés de formule (Ia) 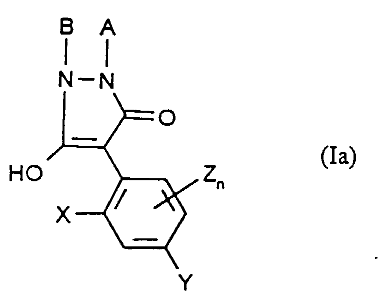 dans laquelle
A, B, X, Y, Z et n ont la définition indiquée ci-dessus, avec des hydroxydes métalliques ou des amines de formules générales (XIV) et (XV) 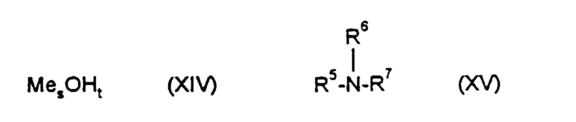 dans lesquelles
Me désigne des ions métalliques monovalents ou divalents,
s et t représentent les nombres 1 et 2 et
R⁵, R⁶ et R⁷ représentent, indépendamment les uns des autres, de l'hydrogène et des groupes alkyle,
le cas échéant en présence d'un diluant.

2. Compositions pesticides, caractérisées par une teneur en au moins un dérivé de 3-hydroxy-4-aryl-5-oxo-pyrazoline de formule (I).

3. Procédé pour combattre des parasites animaux, caractérisé en ce qu'on fait agir des dérivés de 3-hydroxy-4-aryl-5-oxo-pyrazoline de formule (I) sur des parasites animaux et/ou sur leur habitat.

4. Utilisation de dérivés de 3-hydroxy-4-aryl-5-oxo-pyrazoline de formule (I) pour combattre des parasites animaux.

5. Procédé de préparation de compositions pesticides, caractérisé en ce qu'on mélange des dérivés substitués de 3-hydroxy-4-aryl-5-oxo-pyrazoline de formule (I) suivant la revendication 1 avec des diluants et/ou des agents tensio-actifs.

6. Compositions herbicides, caractérisées par une teneur en au moins un dérivé de 3-hydroxy-4-aryl-5-oxo-pyrazoline de formule (I) suivant la revendication 1.

7. Procédé pour combattre des mauvaises herbes, caractérisé en ce qu'on fait agir des dérivés de 3-hydroxy-4-aryl-5-oxo-pyrazoline de formule (I) suivant la revendication 1 sur des mauvaises herbes et/ou sur leur milieu.

8. Utilisation de dérivés de 3-hydroxy-4-aryl-5-oxo-pyrazoline de formule (I) suivant la revendication 1 pour combattre des mauvaises herbes.

9. Procédé de préparation de compositions herbicides, caractérisé en ce qu'on mélange des dérivés de 3-hydroxy-4-aryl-5-oxo-pyrazoline de formule (I) suivant la revendication 1 avec des diluants et/ou des agents tensio-actifs.

10. Procédé de production d'halogénocarbonylcétènes de formule (IIa) 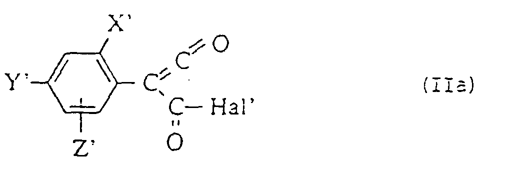 dans laquelle
Hal' représente un halogène,
X' est un groupe alkyle en C₁ à C₄, un halogène ou un groupe trifluorométhyle,
Y' est de l'hydrogène, un groupe alkyle en C₁ à C₄, un halogène ou un groupe trifluorométhyle, et
Z' représente de l'hydrogène, un groupe alkyle en C₁ à C₄, un halogène ou un groupe trifluorométhyle,
sous réserve que Z' ne représente pas de l'hydrogène lorsque Y' est de l'hydrogène,
caractérisé en ce qu'on fait réagir des acides arylmaloniques de formule (XVIa) 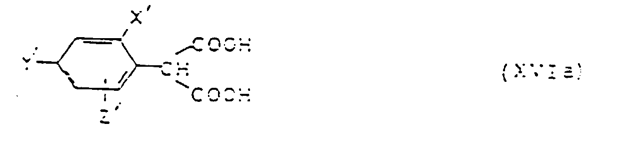 dasn laquelle X', Y' et Z' ont la définition indiquée ci-dessus,
avec des halogénures d'acides, le cas échéant en présence de catalyseurs.
